(19) Europäisches Patentamt
European Patent Office
Office européen des brevets

(11) Veröffentlichungsnummer: **0 324 988 B1**

(12) **EUROPÄISCHE PATENTSCHRIFT**

(45) Veröffentlichungstag der Patentschrift: **29.04.92**

(51) Int. Cl.⁵: **C07D 235/22**, C07D 235/28, C07D 235/26, A61K 31/415

(21) Anmeldenummer: **88121901.8**

(22) Anmeldetag: **30.12.88**

# (54) 4-Chlor-3-sulfamoyl-benzoesäure-hydrazide, Verfahren zu ihrer Herstellung, diese Verbindungen enthaltende Arzneimittelpräparate und Verwendung der neuen Verbindungen zur Herstellung von diuretischen und salzentziehend wirkenden Arzneimittelpräparaten.

(30) Priorität: **30.12.87 HU 613187**

(43) Veröffentlichungstag der Anmeldung:
**26.07.89 Patentblatt 89/30**

(45) Bekanntmachung des Hinweises auf die Patenterteilung:
**29.04.92 Patentblatt 92/18**

(84) Benannte Vertragsstaaten:
**AT BE CH DE ES FR GB GR IT LI LU NL SE**

(56) Entgegenhaltungen:
**DE-A- 1 122 541**
**DE-A- 1 158 927**
**DE-A- 2 247 828**
**DE-A- 2 300 521**
**US-A- 4 420 487**

(73) Patentinhaber: **CHINOIN Gyogyszer és Vegyészeti Termékek Gyára RT.**
**To utca 1-5**
**H-1045 Budapest IV(HU)**

(72) Erfinder: **Pálosi, Endre**
**Pasaréti ut 114/a**
**H-1026, Budapest(HU)**
Erfinder: **Korbonits, Dezs , Dr.**
**Vérhalom u.27/d**
**H-1025, Budapest(HU)**
Erfinder: **Molnár geb. Bak , Erzsébet, Dr.**
**J zsef A.u.5.**
**H-2133 Sz dliget(HU)**
Erfinder: **Szvoboda geb.Kanzel, Ida**
**Váczi M.u.21**
**H-2120 Dunakeszi(HU)**
Erfinder: **Hársing, Lászl , Dr.**
**Sallai u.41**
**H-1136 Budapest(HU)**
Erfinder: **Simon, György, Dr.**
**Dimitrov Tér 4.**
**H-1056 Budapest(HU)**

EP 0 324 988 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services
(3.08/2.18/2.0)

JOURNAL OF HETEROCYCLIC CHEMISTRY, Band 16, Nr. 5, Juli 1979, Seiten 1005-1008, Provo, Utah, US; R. CERRI et al.: "Oxidation and acid-catalyzed cyclization of aldehyde 2-aminophenylhydrazones. Alternative syntheses for 1,2,4-benzotriazines and benzimidazoles"

Erfinder: **Virág, Sándor, Dr.**
**Sallai u.29/a**
**H-1136, Budapest(HU)**
Erfinder: **Gergely, Vera, Dr.**
**Tin di u.9-11**
**H-1095, Budapest(HU)**
Erfinder: **Mármarosi geb. Kellner, Katalin**
**Ybl Mikl s s.19**
**2051, Biatorbágy(HU)**

(74) Vertreter: **Patentanwälte Beetz sen. - Beetz jun. Timpe - Siegfried - Schmitt-Fumian-Mayr**
**Steinsdorfstrasse 10**
**W-8000 München 22(DE)**

## Beschreibung

Die Erfindung betrifft 4-Chlor-3-sulfamoyl-benzoesäurehydrazide, ihre Salze, ein Verfahren zu ihrer Herstellung, die diese Verbindungen enthaltenden Arzneimittelpräparate sowie die Verwendung dieser Verbindungen zur Herstellung von diuretisch und salzentziehend wirkenden Arzneimittelpräparaten. Die Verbindungen entsprechen der allgemeinen Formel I

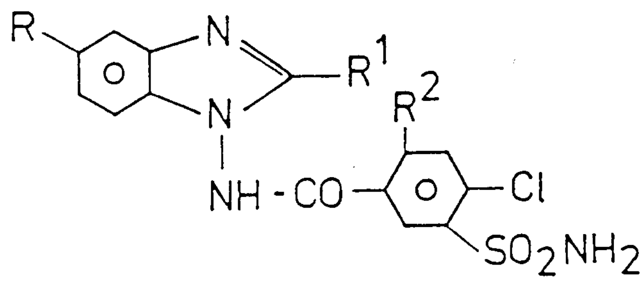

I

worin

R für Wasserstoff, Trifluormethyl-, Carboxylgruppe, Alkoxycarbonylgruppe mit 2-5 Kohlenstoffatomen, Cyano-, Benzoyl-, Sulfamoyl-gruppe oder Alkylsulfonylgruppe mit 1-4 Kohlenstoffatomen,

$R^1$ für Wasserstoff, gerade oder verzweigte Alkylgruppe mit 1-4 Kohlenstoffatomen, Alkylthio- oder Alkylsulfonylgruppe mit jeweils 1-4 Kohlenstoffatomen, Benzylthio-, Benzylsulfonyl-, Phenyl-, Hydroxyl- oder Thiolgruppe und

$R^2$ für Wasserstoff oder Chlor steht.

Diuretika vom Chlor-benzol-sulfonamid-Typ, die am Benzolring eine freie Carboxylgruppe ( DE-OS 1 122 541 und 2 247 828), eine Carbonsäureamidgruppe (DE-OS 1 158 927) oder eine Carbonsäurehydrazidgruppe (ungarische Patentschriften Nr. 150 352 und 152 300) enthalten, sind bereits beschrieben werden. Zum Beispiel ist aus der ersten Gruppe das Furosemid (DE-PS 1 122 541 bzw. K. Sturm, W. Siedel, R. Weyer, H. Ruschig: Chem. Ber. 99, 328 (1966)), aus der zweiten Gruppe das Diapamid (L.T. Blouin, D.H. Kaump, R.L. Fransway, D. Williams: J. New Drugs 3, 302 /1963/), aus der dritten Gruppe das Clopamid (E. Jucker, A. Lindenmann, E. Schenker, E. Fluckinger, M. Taeschler: Arzneim.-Forschung 13, 269 /1963/) bekanntgeworden.

Die chemische Struktur der erfindungsgemäßen Verbindungen weicht von der der genannten, diuretisch wirksamen Verbindungen in bedeutendem Maße ab.

Aus der USP 4 420 487 sind diuretisch wirkende Benzimidazolderivate bekannt, jedoch enthalten diese Verbindungen keine Benzoesäurehydrazid-Gruppierung als Strukturelement, und dadurch unterscheidet sich ihre Struktur bedeutend von der der erfindungsgemäßen Verbindungen.

In an Ratten vorgenommenen Screen-Untersuchungen (5 mg/kg oral) wurden die erfindungsgemäßen Verbindungen mit Dihydrochlorthiazid und Furosemid verglichen. Die gemäß Beispiel 1 hergestellte Verbindung erwies sich hinsichtlich der Harnmenge und der Ionenausscheidung als besonders vorteilhaft und weist ein besonders günstiges Na/K-Verhältnis auf.

Es ist besonders vorteilhaft, daß die erfindungsgemäßen Verbindungen neben ihrer ausgezeichneten Wirkung auch eine größere therapeutische Zuverlässigkeit aufweisen als die "high-ceiling"-Verbindungen, weil im Falle der ersteren Eintreten und Verlauf der Diurese und Salurese nicht so schnell und heftig sind. Nach der Verabreichung hält die Wirkung 24 Stunden lang an.

Ein weiterer Vorteil der Verbindung gemäß Beispiel 1 im Vergleich zu den Kontrollverbindungen besteht darin, daß sie auch in hoher Dosis (30 mg/kg) die Glucosetoleranz nicht verschlechtert und weder auf die Harnsäurekonzentration des Serums noch auf den Cholesterinspiegel des Serums einen signifikanten Einfluß hat.

Die akute Toxizität der erfindungsgemäßen Verbindungen ist wesentlich geringer, infolgedessen ihr therapeutischer Index wesentlich besser als der der zum Vergleich herangezogenen Verbindungen.

Die blutdrucksenkende Wirkung der Verbindungen wurde in einer Dosis von 5 mg/kg an Spontan hypertensiven Ratten untersucht. Die gemäß Beispiel 1 hergestellte Verbindung bewirkte 12 Stunden nach der Applikation eine Blutdrucksenkung von 21,1 %. Das als Vergleichssubstanz verwendete Furosemid zeigte eine ähnliche blutdrucksenkende Wirkung in einer Dosis von 100 mg/kg.

Für die orale Verabreichung in der Humantherapie kommen die die üblichen Hilfsstoffe enthaltenden Tabletten, Dragees oder Kapseln (mit 1-200 mg Wirkstoff) in Frage, während für die intravenöse Anwendung vorzugsweise wäßrige Injektionslösungen hergestellt werden, die den Wirkstoff in Form eines wasserlöslichen Salzes enthalten. Derartige Salze sind zum Beispiel die Alkalisalze, wie das Natriumsalz.

Im erfindungsgemäßen Verfahren zur Herstellung der Verbindungen der allgemeinen Formel I und ihrer physiologisch unbedenklichen Salze (wobei die Bedeutung von R, $R^1$ und $R^2$ die gleiche wie oben ist), werden

a) 1-Aminobenzimidazole der allgemeinen Formel II

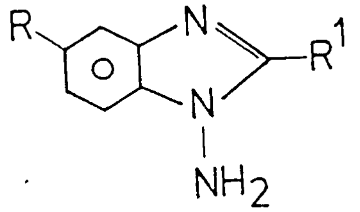

II

(worin die Bedeutung von R und $R^1$ die gleiche wie oben ist) mit Carbonsäurederivaten der allgemeinen Formel III

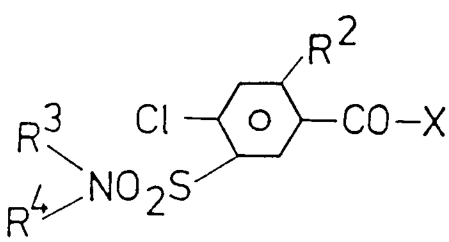

III

worin

X für Chlor, -OH, $-OCH_2CN$, $-OCH_3$, $-OC_2H_5$, $-OCOCCH_3$ oder $-OCOOC_2H_5$ steht, die Bedeutung von

$R^2$ die gleiche wie oben ist und

$R^3$ und $R^4$ für Wasserstoffatom oder zusammen für die Gruppierung $=CHN(CH_3)_2$ stehen,

umgesetzt und im Falle von Verbindungen der allgemeinen Formel Ia

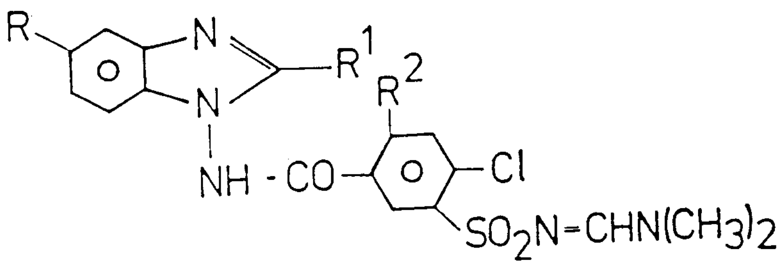

Ia

(worin die Bedeutung von R, $R^1$ und $R^2$ die gleiche wie oben ist) wird die Schutzgruppe in alkalischem Medium abgespalten, oder

b) zur Herstellung der eine engere Gruppe der Verbindungen der allgemeinen Formel I bildenden Verbindungen der allgemeinen Formel Ie

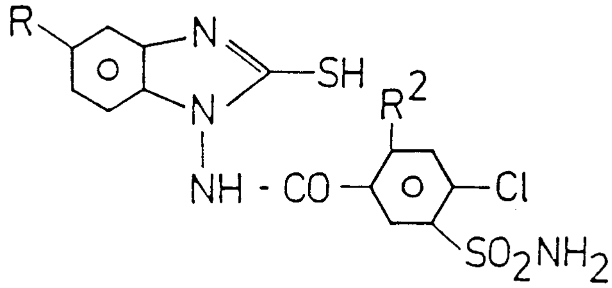

Ie

(worin die Bedeutung von R und $R^2$ die gleiche wie oben ist) werden 2-Aminophenylhydrazin-Derivate der allgemeinen Formel IVb

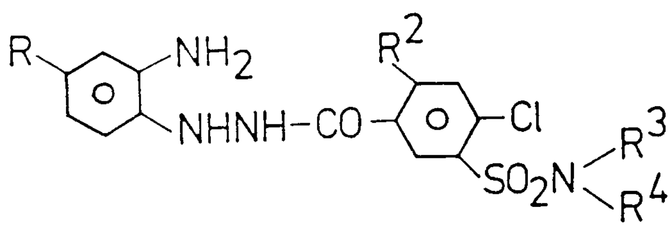

IVb

(worin die Bedeutung von R, $R^2$, $R^3$ und $R^4$ die gleiche wie oben ist) mit Schwefelkohlenstoff, Kaliumäthylxanthat oder Thiophosgen umgesetzt und im Falle von Verbindungen der allgemeinen Formel Ib

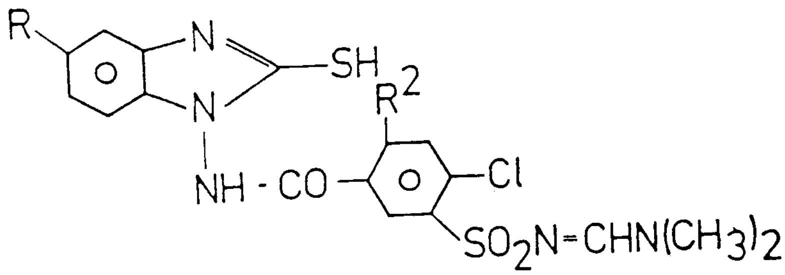

Ib

wird die Schutzgruppe in alkalischem Medium abgespalten, oder

c) zur Herstellung von Verbindungen der allgemeinen Formel I, in denen die Bedeutung von R und $R^2$ die gleiche wie oben ist und

$R^1$ für Wasserstoff, Methyl-, Äthyl-, Propyl-, Isopropyl-, Butyl-, Isobutyl-, tert.-Butyl- oder Phenylgruppe steht,

werden 2-Aminophenylhydrazin-Derivate der allgemeinen Formel IVa

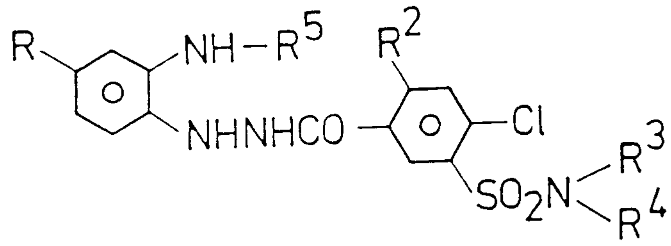

IVa

(worin die Bedeutung von R, $R^2$, $R^3$ und $R^4$ die gleiche wie oben ist und
$R^5$ für Wasserstoff, Acetyl-, Propionyl-, Butyryl-, Isobutyryl-, Pentanoyl-, 2-Methylbutyryl-, Trimethylacetyl- oder Benzoylgruppe steht,
mit Ameisensäure oder Essigsäure umgesetzt, und die erhaltene Verbindung wird gewünschtenfalls in alkalischem Medium hydrolysiert, oder
d) zur Herstellung der eine engere Gruppe der Verbindungen der allgemeinen Formel I bildenden Verbindungen der allgemeinen Formel Id

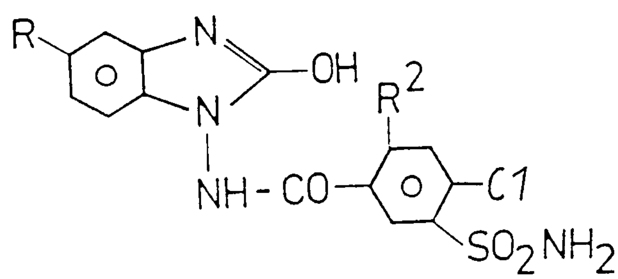

Id

(worin die Bedeutung von R und $R^2$ die gleiche wie oben ist) werden 2-Aminophenylhydrazin-Derivate der allgemeinen Formel IVb (s. oben, worin die Bedeutung von R, $R^2$, $R^3$ und $R^4$ die gleiche wie oben ist) mit N,N$^1$-Dialkoxycarbonyl-S-methylisothioharnstoffen der allgemeinen Formel VII

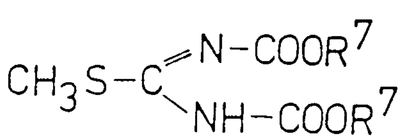

VII

worin $R^7$ für Alkylgruppe mit 1-4 Kohlenstoffatomen steht, umgesetzt und im Fall der Verbindungen der allgemeinen Formel If

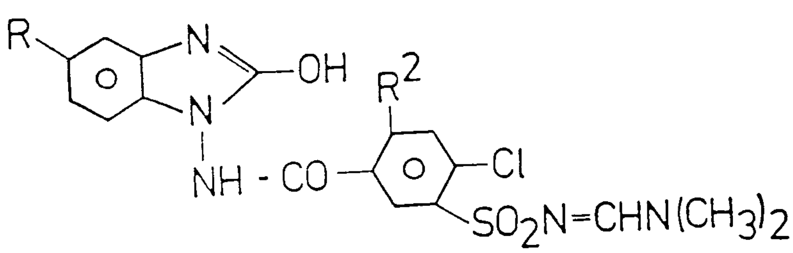

If

(worin die Bedeutung von R und $R^2$ die gleiche wie oben ist) wird die Schutzgruppe in alkalischem Medium abgespalten, oder

e) zur Herstellung der eine engere Gruppe der Verbindungen der allgemeinen Formel I bildenden Verbindungen der allgemeinen Formel Ic

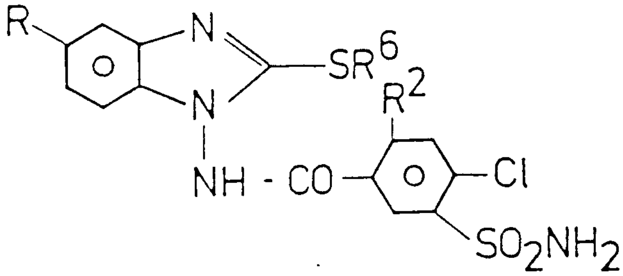

Ic

(worin die Bedeutung von R und $R^2$ die gleiche wie oben ist und

$R^6$ für eine gerade oder verzweigte Alkylgruppe mit 1-4 Kohlenstoffatomen oder für Benzylgruppe steht), werden Verbindungen

der allgemeinen Formeln Ib oder Ie (worin die Bedeutung von R, $R^2$, $R^3$ und $R^4$ die gleiche wie oben ist) mit einem Alkylierungsmittel umgesetzt, und im Falle von Verbindungen der allgemeinen Formel Ih

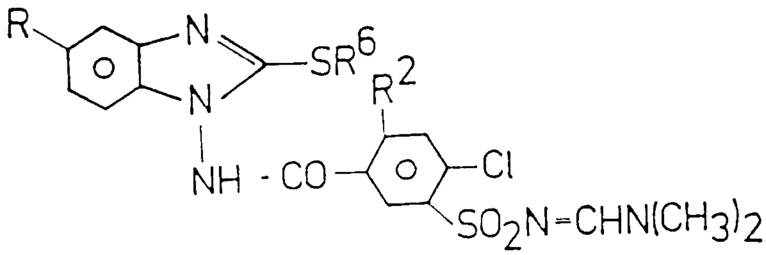

Ih

(worin die Bedeutung von R, $R^2$ und $R^6$ die gleiche wie oben ist) wird die Schutzgruppe in alkalischem Medium abgespalten, oder

f) zur Herstellung der eine engere Gruppe der Verbindungen der allgemeinen Formel I bildenden Verbindungen der allgemeinen Formel Ig

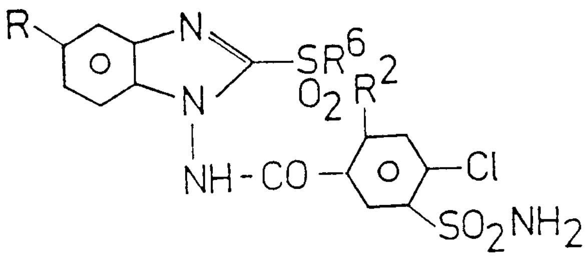

Ig

(worin die Bedeutung von R, $R^2$, $R^3$, $R^4$ und $R^6$ die gleiche wie oben ist) werden 2-Benzimidazolylthioäther der allgemeinen Formeln Ic oder Ih (worin die Bedeutung von R, $R^2$, $R^3$, $R^4$ und $R^6$ die gleiche wie oben its) mit Oxydationsmitteln umgesetzt und die entstandenen Verbindungen gewünschtenfalls in alkalischem Medium hydrolysiert, und die gemäß den Verfahren a) - f) erhaltenen Verbindungen werden gewünschtenfalls zu ihren physiologisch verträglichen Salzen umgesetzt.

Die eine Untergruppe der Verbindungen der allgemeinen Formel I bildenden Verbindungen der allgemeinen Formeln Id und Ie können in den tautomeren Formen gemäß den Abbildungen H beziehungsweise K vorliegen.

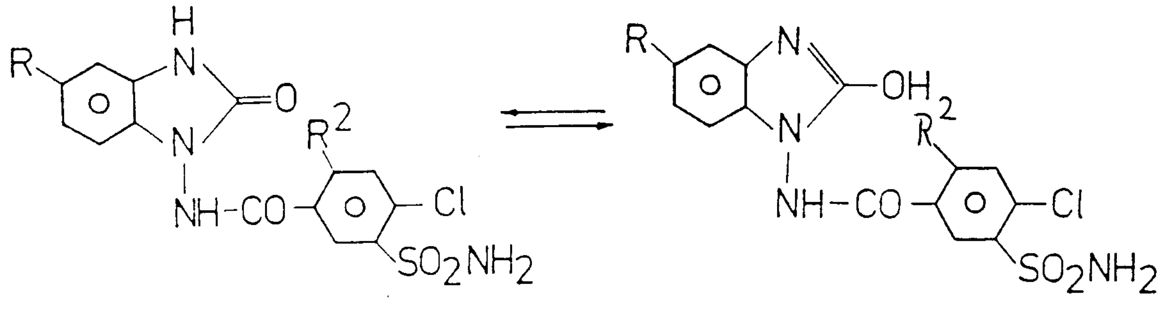

H.

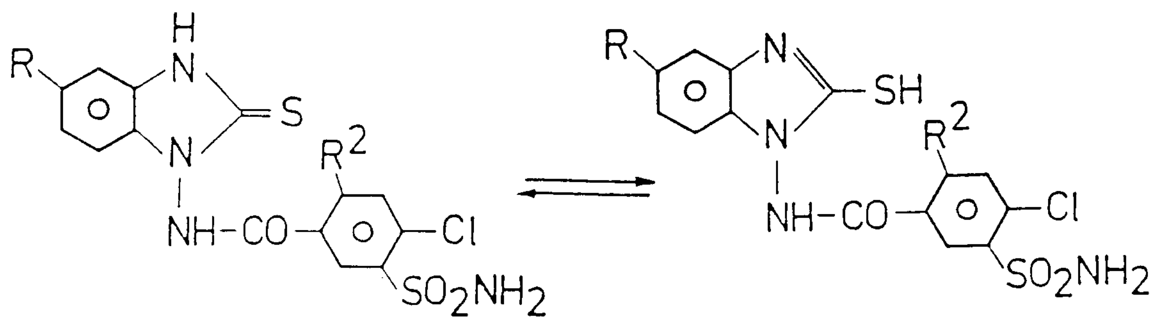

K

Im Verfahren a) werden 1-Aminobenzimidazol-Derivate der allgemeinen Formel II und Carbonsäurederivate der allgemeinen Formel III gemäß dem Reaktionsschema A zu Verbindungen der allgemeinen Formel I umgesetzt. (Die Reaktionsschemata befinden sich am Ende der Beschreibung.)

In dieser Reaktion werden zum Acylieren die freie Carbonsäure oder ihre reaktionsfähigen Derivate, wie das Säurehalogenid, die mit niederen Alkanolen gebildeten Ester, die aktiven Ester oder gemischte Anhydride verwendet. Als Alkylester kommen in erster Linie der Methyl- und der Äthylester, als aktiver Ester der Cyanomethylester in Frage. Die Reaktanten werden in äquimolaren Mengen eingesetzt, dem Reaktionsgemisch wird Triäthylamin oder Natriumamid zugesetzt.

In manchen Fällen kann es vorteilhaft sein, die Sulfonamidgruppe zu substituieren. Für diesen Zweck hat sich die Kondensation mit Formamidacetalen bewährt, bei der Aminomethylidensulfamide entstehen (Reaktionsschema B). Diese Reaktion ist insbesondere dann vorteilhaft, wenn zum Acylieren gemäß Reaktionsschema A das Säurechlorid verwendet wird. Diese "geschützten" Säurechloride sind nämlich viel stabildere Verbindungen als ihre die freie Sulfonamidgruppe enthaltenden Analogen. Die Umsetzung kann in Dimethylformamid mit Dimethylformamid-dimethylacetal bei 40-80 °C vorgenommen werden. Besonders vorteilhaft wird die Verbindung VI (s. Reaktionsschema B) hergestellt, indem das Dimethylformamiddimethylacetal im Reaktionsgemisch in situ erzeugt wird, das sofort mit der Sulfonamidgruppe reagiert, wobei die "geschützte" Säure VI entsteht, die dann durch Umsetzen mit Thionylchlorid in sehr guter Ausbeute zu dem Säurechlorid der allgemeinen Formel IIIa umgewandelt werden kann.

Das Acylieren mit Säurechloriden oder gemischten Anhydriden wird in polaren Lösungsmitteln, zum Beispiel Tetrahydrofuran, Dioxan, Pyridin, Dimethylformamid, Dimethylacetamid, Dimethylcarbamid vorgenommen. Die Reaktionstemperatur kann zwischen -20 °C und dem Siedepunkt des verwendeten Lösungsmittels liegen. Wenn das Lösungsmittel an sich nicht basisch ist, so werden dem Reaktionsgemisch als Säurebindemittel organische Basen wie Triäthylamin oder Dimethylanilin zugesetzt.

Beim Acylieren mit Säurechloriden der allgemeinen Formel IIIa kann man die Acylierungsreaktion auch in einem Gemisch aus Wasser und einem wassermischbaren organischen Lösungsmittel, in Gegenwart von Alkali- oder Erdalkalicarbonat oder -hydrogencarbonat als Säurebindemittel ablaufen lassen. Die mit Wasser

mischbaren organischen Lösungsmittel können protisch oder aprotisch sein. Von den aprotischen Lösungsmitteln kommen Äther (Dioxan, Tetrahydrofuran), Ketone (Aceton) und Säureamide (Dimethylformamid, Dimethylacetamid), von den protischen Lösungsmitteln mit Wasser unbegrenzt mischbare niedere aliphatische Alkohole (Methyl-, Äthyl-, Propylalkohol) in Frage.

Als Alkalicarbonat können Natrium- und Kaliumcarbonat, als Erdalkalicarbonate Magnesium- und Calciumcarbonat, als Alkalihydrogencarbonat Natrium- und Kaliumhydrogencarbonat verwendet werden.

Die Umsetzung erfolgt bevorzugt bei 0-100 °C, insbesondere bei 10-30 °C.

Zur Herstellung gemischter Anhydride werden die Säuren der allgemeinen Formel VI mit Chlorkohlensäurealkylestern umgesetzt. In erster Linie kommen der Chlorkohlensäuremethyl- und -äthylester in Frage. Das gemischte Anhydrid kann abgetrennt werden, vorteilhafter ist es jedoch, ihn im Reaktionsgemisch herzustellen und ohne zwischenzeitliche Abtrennung mit der Aminoverbindung der allgemeinen Formel II umzusetzen.

Die Schutzgruppe wird durch alkalische Hydrolyse abgetrennt. Die Reaktion wird in wässrigem Medium mit starken anorganischen Basen, wie vorzugsweise Natrium- oder Kaliumhydroxyd ausgeführt. Die Temperaturen liegen dabei zwischen 20 und 80 °C, vorzugsweise zwischen 50 und 60 °C. Auf 1 Mol zu hydrolysierende Verbindung rechnet man 2-6 Mol, vorzugsweise 3-4 Mol anorganische Base.

Wird als Acylierungsmittel eine Carbonsäure (X = OH) verwendet, so empfiehlt sich der Zusatz eines Kondensationsmittels. Ausgezeichnet geeignet sind zum Beispiel das Dicyclohexylcarbodiimid sowie das Tetrachlorsilan. Die Umsetzung wird bevorzugt in Pyridin vorgenommen.

Im Verfahren b) werden 2-Aminophenylhydrazine der allgemeinen Formel IVb mit Schwefelkohlenstoff, Kaliumäthylxanthat oder Thiophosgen zu Benzimidazolderivaten der allgemeinen Formel Ie umgesetzt (Reaktionsschema C). Gemäß einer Variante dieser Reaktion wird die Verbindung der allgemeinen Formel IVb mit Schwefelkohlenstoff umgesetzt. Die Temperaturen liegen dabei zwischen 10 und 150 °C, vorzugsweise zwischen 30 und 100 °C. Die Umsetzung wird in Wasser oder einem organischen Lösungsmittel, in Gegenwart einer Base ausgeführt.

Als organische Lösungsmittel kommen Alkohole, zum Beispiel Methanol, Äthanol oder die Propylalkohole in Frage. Als Base können tertiäre Amine, wie Triäthylamin oder Pyridin, oder vorzugsweise Alkalihydroxyde, insbesondere Kaliumhydroxyd, verwendet werden.

Gemäß einer weiteren Variante wird die Verbindung der allgemeinen Formel IVb mit einem Alkaliäthylxanthat, vorzugsweise mit Kaliumäthylxanthat, umgesetzt. Die Umsetzung wird bei 20-150 °C, vorzugsweise 50-100 °C in Wasser oder einem organischen Lösungsmittel vorgenommen. Als organische Lösungsmittel kommen Alkohole, wie zum Beispiel Methanol, Äthanol oder die Propylalkohole, sowie tertiäre Amine, zum Beispiel Triäthylamin und Pyridin, in Frage.

Gemäß einer dritten Möglichkeit wird die Verbindung der allgemeinen Formel IVb in Wasser oder einem organischen Lösungsmittel, in Gegenwart einer Base mit Thiophosgen umgesetzt. Die Reaktion wird bei 0-120 °C, vorzugsweise 20-100 °C, vorgenommen. Als organische Lösungsmittel kommen gegenüber den Reaktionspartnern inerte Lösungsmittel, zum Beispiel Äther wie Dioxan oder Tetrahydrofuran, Als Base können tertiäre Amine, zum Beispiel Triäthylamin oder Pyridin, oder bevorzugt Alkalihydroxyde wie Natrium- oder Kaliumhydroxyd verwendet werden. Von Verbindungen der allgemeinen Formel Ib kann die Schutzgruppe durch alkalische Hydrolyse auf die bei der Reaktion a) beschriebene Weise abgetrennt werden.

Beim Verfahren c) werden 2-Aminophenylhydrazine der allgemeinen Formel IVa durch Erwärmen mit Ameisensäure oder Essigsäure gemäß dem Reaktionsschema D zu Verbindungen der allgemeinen Formel I umgesetzt. Die Ameisensäure beziehungsweise Essigsäure dient gleichzeitig als Lösungsmittel. Die Reaktionstemperatur liegt zwischen 50 °C und dem Siedepunkt des Reaktionsgemisches und beträgt bevorzugt 80-100 °C. Wenn $R^3$ und $R^4$ zusammen für die Gruppe $=CHN(CH_3)_2$ stehen, so kann die Schutzgruppe durch alkalische Hydrolyse auf die bereits beschriebene Weise entfernt werden.

Im Verfahren d) werden 2-Aminophenylhydrazinderivate der allgemeinen Formel IVb mit der äquimolaren Menge $N,N^1$-Dialkoxycarbonyl--S-methylisothioharnstoff zu Benzimidazolderivaten der allgemeinen Formel Id umgesetzt (Reaktionsschema E). Die Umsetzung erfolgt in einem polaren organischen Lösungsmittel bei Temperaturen zwischen 50 °C und dem Siedepunkt des verwendeten Lösungsmittels. Als Lösungsmittel kommen Pyridin, Äthylenglycol, Dimethylsulfoxyd, vorzugsweise jedoch Dimethylformamid oder Dimethylacetamid in Frage. Von erhaltenen Verbindungen der allgemeinen Formel If wird die Schutzgruppe durch alkalische Hydrolyse auf die im Zusammenhang mit dem Verfahren a) beschriebene Weise entfernt.

Im Verfahren e) werden Verbindungen der allgemeinen Formel Ib oder Ie in an sich bekannter Weise durch Umsetzen mit einem Alkylierungsmittel zu Verbindungen der allgemeinen Formel Ic umgesetzt (Reaktionsschema F).

Als Alkylierungsmittel können Dialkylsulfate, Alkylhalogenide oder p-Toluolsulfonsäurealkylester in Wasser oder einem organischen Lösungsmittel verwendet werden. Von den Dialkylsulfaten sind Dimethyl- und Diäthylsulfat, von den Alkylhalogeniden Methyl-, Äthyl-, Propyl-, Butyl- und Benzylchlorid, -bromid oder -jodid, von den p-Toluolsulfonsäureestern der Methyl-, Äthyl-, Propyl-und Benzylester bevorzugt. Als organisches Lösungsmittel kommen niedere Alkohole, zum Beispiel Methanol oder Äthanol, in Frage. Die Reaktion wird in Gegenwart einer Base vorgenommen. Als Base können in erster Linie die Alkalihydroxyde, vorzugsweise Natrium-oder Kaliumhydroxyd, oder die Alkalialkoholate, vorzugsweise Natriummethylat oder Natriumäthylat, verwendet werden.

Gemäß einer bevorzugten Ausführungsform wird die Verbindung der allgemeinen Formel Ib in der äquivalenten Menge wässriger Natriumhydroxydlösung oder der äquivalenten Menge einer alkoholischen Lösung von Natriumäthylat gelöst und das Alkylierungsmittel zu der Lösung gegeben.

Die Alkylierung wird bei Temperaturen zwischen 20 °C und dem Siedepunkt des verwendeten Lösungsmittels ausgeführt. Von gebildeten Verbindungen der allgemeinen Formel Ih wird die Schutzgruppe durch alkalische Hydrolyse auf die bereits beschriebene Weise entfernt.

Gemäß dem Verfahren f) werden 2-Benzimidazolilthioäther der allgemeinen Formeln Ic oder Ih in an sich bekannter Weise mit Oxydationsmitteln umgesetzt, wobei Verbindungen der allgemeinen Formel Ig entstehen (Reaktionsschema G).

Als Oxydationsmittel werden Wasserstoffperoxyd oder Peressigsäure verwendet. Die Reaktion kann in Wasser, organischen Lösungsmitteln oder Gemischen aus Wasser und organischen Lösungsmitteln vorgenommen werden. Als organisches Lösungsmittel sind Ameisensäure oder Essigsäure geeignet. Die Reaktionstemperatur liegt zwischen 20 und 120 °C, vorzugsweise zwischen 60 und 100 °C. Von entstandenen Verbindungen der allgemeinen Formel Ij wird die Schutzgruppe durch alkalische Hydrolyse auf die bereits beschriebene Weise entfernt.

Für therapeutische Zwecke sind insbesondere Verbindungen der allgemeinen Formel I geeignet, die als Substituenten R eine Carboxylgruppe tragen. Diese Verbindungen bilden stabile, in Wasser gut lösliche, neutral reagierende Lösungen ergebende Salze. In erster Linie sind die Natrium- und Kaliumsalze von Bedeutung, für spezielle Zwecke können jedoch auch die Calcium- und Magnesiumsalze in Frage kommen.

Auf Grund von an Ratten und Hunden vorgenommenen Versuchen kann festgestellt werden, daß die hergestellten Verbindungen eine ausgezeichnete saldiuretische Wirkung aufweisen. Die Wirkung tritt 1-2 Stunden nach der Verabreichung ein, erreicht ihr Maximum zwischen der 3. und 5. Stunde und hält 24 Stunden lang an. Dadurch wird eine schonende und langanhaltende Diurese gewährleistet. Ein besonderer Vorteil der neuen Verbindungen ist ihr günstiges Natrium-Kalium-Verhältnis sowie ihre sehr geringe Wirkung auf den Cholesterinspiegel des Serums. Die Verbindungen sind insbesondere zur intravenösen Verabreichung geeignet.

In der Humanmedizin können die Verbindungen in Form wässriger Injektionslösungen verwendet werden, die 0,1-100 mg Wirkstoff in Form eines Alkalisalzes enthalten. Zur oralen Applikation sind Tabletten, Dragees oder Kapseln geeignet, die 1-300 mg Wirkstoff und die üblichen Träger- und Füllstoffe enthalten.

Die Erfindung wird im folgenden an Hand von Ausführungsbeispielen näher erläutert.

## Beispiel 1

100 g 2-Amino-4-methoxycarbonyl-N-(4′-chlor-3′-sulfamoyl-benzoyl)-phenylhydrazin werden in 400 ml Methanol suspendiert und zu der Suspension 105 ml Schwefelkohlenstoff gegeben. Das Reaktionsgemisch wird unter Kühlen mit kaltem Wasser und unter Rühren tropfenweise mit der Lösung von 37,8 g Kaliumhydroxyd in 190 ml abs. Alkohol versetzt, wobei die Temperatur unter 20 °C gehalten wird. Das Reaktionsgemisch wird unter ständigem Rühren 2 Stunden lang gekocht. Die entstandene klare braune Lösung wird über Nacht stehengelassen und anderentags mit 145 ml Essigsäure versetzt. Die Lösung wird eine Stunde lang gerührt, dann auf die Hälfte eingedampft und der Rückstand unter Rühren in 1500 ml Wasser gegossen. Der ausfallende sandfarbene Niederschlag wird abgesaugt, mit Wasser gewaschen und dann getrocknet. Das Rohprodukt wird in einem aus gleichen Teilen Dimethylformamid und Wasser bestehenden Gemisch kochend gelöst. Die heiße Lösung wird mit Aktivkohle geklärt und heiß filtriert. Nach dem Abkühlen werden die ausgefallenen sandfarbenen Kristalle abgesaugt, mit Wasser gewaschen und im Vakuum bei 100 °C getrocknet. Man erhält 75 g (68 %) 1-[(4′-Chlor-3′-sulfamoyl-benzoyl)-amino]-5-methoxycarbonyl-benzimidazol-2-thion, das sich bei 242-245 °C zersetzt. Nach Umkristallisieren aus einem im Verhältnis 1:2 bereiteten Gemisch von Dimethylformamid und Wasser schmilzt die Verbindung bei 258-261 °C unter Zersetzung.

| Analyse für $C_{16}H_{13}ClN_4O_5S_2$ | | | | | | | | | |
|---|---|---|---|---|---|---|---|---|---|
| berechnet, %: | C | 43,59 | H | 2,97 | N | 12,71 | Cl | 8,04 | S | 14,55 |
| gefunden, %: | C | 43,80 | H | 2,78 | N | 12,57 | Cl | 8,62 | S | 14,60. |

48,4 g des auf die beschriebene Weise erhaltenen Esters werden in 330 ml 2n Natronlauge bei 50 °C 4 Stunden lang gerührt. Die entstandene klare gelbe Lösung wird nach dem Abkühlen mit 2n Salzsäure schwach angesäuert ( pH 3-4). Der ausgefallene Niederschlag wird abgesaugt, mit Wasser gewaschen und bei 80 °C getrocknet. Das erhaltene Rohprodukt wird aus einem 1:1-Gemisch von Dimethylformamid und Wasser nach Klären mit Aktivkohle umkristallisiert. Das 1 Molekül Dimethylformamid enthaltende 1-[(4′-Chlor-3′-sulfamoyl-benzoyl)-amino]-5-carboxy-bezimidazol-2-thion fällt in Form schneeweißer, winziger Kristalle an.

Das erhaltene, dimethylformamid-haltige Produkt wird in 430 ml destilliertem Wasser 30 Minuten lang gekocht. Nach dem Abkühlen werden die weißen Kristalle abfiltriert, mit Wasser gewaschen und im Vakuum bei 80 °C getrocknet. Man erhält 37,4 g (76,5 %) 1-[(4′-Chlor-3′-sulfamoyl-benzoyl)-amino]-5-carboxy-benzimidazol-2-thion-monohydrat, das bei 320-322 °C schmilzt.

| Analyse für $C_{15}H_{11}ClN_4O_5S_2.H_2O$ | | | | | | | | | | |
|---|---|---|---|---|---|---|---|---|---|---|
| berechnet, % | C | 40,50 | H | 2,94 | N | 12,59 | Cl | 7,97 | S | 14,41 |
| gefunden, % | C | 40,22 | H | 2,80 | N | 12,20 | Cl | 7,70 | S | 14,65. |

Herstellung der Ausgangssubstanz

A) Zu der Suspension von 172,5 g 3-Nitro-4-chlor-benzoesäuremethylester in 1600 ml abs. Alkohol werden unter Rühren 80 ml Hydrazinhydrat gegeben. Das Reaktionsgemisch wird unter Rühren 45 Minute lang gekocht. Innerhalb dieser Zeit geht der Ausgangsstoff in Lösung, und dann scheidet sich das Produkt aus. Es wird nach dem Abkühlen abgesaugt, zuerst mit abs. Alkohol und dann mit Wasser chloridionenfrei gewaschen. Man erhält 143,6 g (85 %) 2-Nitro-4-methoxycarbonyl-phenylhydrazin in Form gelber Kristalle. Schmelzpunkt: 169-171 °C.

| Analyse für $C_8H_9N_3O_4$ | | | | | | |
|---|---|---|---|---|---|---|
| berechnet, %: | C | 45,49 | H | 4,29 | N | 19,89 |
| gefunden, %: | C | 45,58 | H | 4,31 | N | 20,33. |

B) 105 g des auf die beschriebene Weise hergestellten Phenylhydrazinderivates werden in 1 Liter Dioxan suspendiert und zu der Suspension unter Rühren zuerst die mit 500 ml Dioxan bereitete Lösung von 127 g 4-Chlor-3-sulfamoyl-benzoylchlorid (Herstellung: J. Med. Chem. 11, 970/1968/) und dann 26,5 g wasserfreies Natriumcarbonat gegeben. Das Reaktionsgemisch wird auf dem kochenden Wasserbad unter einem Rückflußkühler 5 Stunden lang gerührt. Nach dem Abkühlen wird filtriert und das Filtrat im Vakuum eingedampft. Der harzartige Rückstand wird in 1,5 Liter Wasser so lange gerührt, bis er zu Staub zerfällt. Das Produkt wird abgesaugt, mit Wasser gewaschen und bei 80 °C getrocknet. Man erhält 203 g (94,7 %) 2-Nitro-4-methoxycarbonyl-N-(4′-chlor-3′-sulfamoyl-benzoyl)-phenylhydrazin in Form eines gelben Pulvers, das bei 144-147 °C schmilzt.

| Analyse für $C_{15}H_{13}ClN_4O_7S$ | | | | | | | | | | |
|---|---|---|---|---|---|---|---|---|---|---|
| berechnet, %: | C | 42,01 | H | 3,05 | N | 13,07 | Cl | 8,27 | S | 7,48 |
| gefunden, %: | C | 41,71 | H | 3,38 | N | 13,06 | Cl | 8,01 | S | 7,86. |

C) Zu der auf etwa 70 °C erwärmten Lösung von 60 g der auf die beschriebene Weise hergestellten Nitroverbindung in 500 ml Methylcellosolv werden 10 g Raney-Nickel gegeben. Das Gemisch wird bei 70 °C unter 10 atm Druck auf dem Schüttelapparat hydriert. Nach Beendigung der Wasserstoffaufnahme wird das Gemisch abgekühlt und der Katalysator durch Filtrieren entfernt. Das Filtrat wird im Vakuum

eingedampft. Der harzartige Rückstand wird in 500 ml Wasser so lange gerührt, bis er zu einem filtrierbaren Staub zerfällt. Das Produkt wird abfiltriert, mit Wasser gewaschen und bei 80 °C getrocknet. Man erhält 51 g (91 %) 2-Amino-4-methoxycarbonyl-N-(4′-chlor-3′-sulfamoyl-benzoyl)-phenylhydrazin, das sich bei 214-215 °C zersetzt.

| Analyse für $C_{15}H_{15}ClN_4O_5S$ | | | | | | | | | | |
|---|---|---|---|---|---|---|---|---|---|---|
| berechnet, %: | C | 45,17 | H | 3,79 | N | 14,05 | Cl | 8,89 | S | 8,04 |
| gefunden, %: | C | 45,72 | H | 3,71 | N | 13,75 | Cl | 9,29 | S | 7,94. |

Beispiel 2

Zu der Suspension von 38,5 g 2-Amino-4-carboxy-N-(4′-Chlor-3′-sulfamoyl-benzoyl)-phenylhydrazin in 160 ml Methanol werden 42 ml Schwefelkohlenstoff gegeben. Dann wird dem Reaktionsgemisch unter Rühren tropfenweise die Lösung von 20,72 g Kaliumhydroxyd in 105 ml abs. Alkohol zugesetzt. Die erhaltene Lösung wird eine Stunde lang gerührt, dann abgekühlt und mit 2n Salzsäure auf pH 4-5 angesäuert. Der ausgeschiedene Niederschlag wird abfiltriert, mit Wasser gewaschen und bei 80 °C getrocknet. Man erhält 31,8 g (75 %) 1-[(4′-Chlor-3′-sulfamoyl-benzoyl)-amino]-5-carboxy-benzimidazol-2-thion in Form eines sandfarbenen Pulvers, das sich bei 290-295 °C zersetzt. Es wird auf die im Beispiel 1 angegebene Weise gereinigt; die beiden Substanzen sind in jeder Hinsicht miteinander identisch.

Herstellung der Ausgangssubstanz

A) 25,7 g 2-Nitro-4-methoxycarbonyl-N-(4′-chlor-3′-sulfamoyl-benzoyl)-phenylhydrazin werden mit 120 ml 2n Natronlauge bei 50 °C 4 Stunden lang gerührt. Nach dem Abkühlen wird der pH-Wert der entstandenen dunkellila Lösung mit 2n Salzsäure auf 5 eingestellt. Der ausgefallene gelbe Niederschlag wird abgesaugt, mit Wasser gewaschen und bei 80 °C getrocknet. Man erhält 23 g (92,5 %) 2-Nitro-4-carboxy-N-(4′-chlor-3′-sulfamoyl-benzoyl)-phenylhydrazin in Form eines gelben Pulvers, das sich bei 273-275 °C zersetzt.

| Analyse für $C_{14}H_{11}ClN_4O_7S$ | | | | | | | | | | |
|---|---|---|---|---|---|---|---|---|---|---|
| berechnet, %: | C | 40,53 | H | 2,67 | N | 13,50 | Cl | 8,50 | S | 7,70 |
| gefunden, %: | C | 40,23 | H | 2,88 | N | 13,76 | Cl | 8,54 | S | 7,66. |

B) Zu einer Suspension von 41,4 g der auf die beschriebene Weise hergestellten Nitroverbindung in 500 ml 96 %igem Alkohol werden 4 g 10 %ige Palladiumaktovkohle gegeben. Das Reaktionsgemisch wird unter Rühren auf 60-70 °C erwärmt und bei dieser Temperatur unter Rühren mit 150 ml 30 %iger wässriger Natriumhypophosphitlösung tropfenweise versetzt. Die Dosiergeschwindigkeit wird so gewählt, daß das Gemisch nicht schäumt. Nach dem Abkühlen wird abgesaugt und der Nutschkuchen mit 70 ml 2n Natronlauge verrührt. Aus der Lösung wird der Katalysator abfiltriert, und das Filtrat wird mit 2n Salzsäure auf pH 5 angesäuert. Der ausfallende Niederschlag wird abfiltriert, mit Wasser gewaschen und bei 80 °C getrocknet. Man erhält 32 g (83 %) 2-Amino-4-carboxy-N-(4′-chlor-3′-sulfamoyl-benzoyl)-phenylhydrazin in Form eines sandfarbenen Pulvers, das sich bei 240-244 °C zersetzt. Nach Umkristallisieren aus einem im Verhältnis 1:2 bereiteten Gemisch von Dimethylformamid und Wasser schmilzt die Substanz unter Zersetzung bei 245-246 °C.

| Analyse für $C_{14}H_{13}ClN_4O_5S$ | | | | | | | | | | |
|---|---|---|---|---|---|---|---|---|---|---|
| berechnet, %: | C | 43,70 | H | 3,40 | N | 14,56 | Cl | 9,21 | S | 8,33 |
| gefunden, %: | C | 43,83 | H | 3,40 | N | 14,21 | Cl | 8,94 | S | 8,10 |

Beispiel 3

Ein Gemisch aus 8 g 2-Amino-4-methoxycarbonyl-N-(4′-chlor-3′-sulfamoyl-benzoyl)-phenylhydrazin, 30 ml Pyridin und 3,2 g Kaliumäthylxanthat wird unter dem Rückflußkühler 30 Minuten lang gekocht. Das Pyridin wird im Vakuum abdestilliert und der Rückstand in 40 ml Eisessig gelöst. Die Lösung wird in 160 ml Wasser gegossen. Der ausgefallene Niederschlag wird abfiltriert, mit Wasser gewaschen und bei 80 °C getrocknet. Man erhält 8,2 g (93 %) 1-[(4′-Chlor-3′-sulfamoyl-benzoyl)-amino]-5-methoxycarbonyl-benzimidazol-2-thion in Form eines rosenfarbenen Pulvers. Die Verbindung schmilzt nach Umkristallisieren aus einem im Verhältnis 1:2 bereiteten Gemisch von Dimethylformamid und Wasser unter Zersetzung bei 258-261 °C.

Beispiel 4

Zu der Lösung von 4 g 2-Amino-4-methoxycarbonyl-N-(4′-chlor-3′-sulfamoyl-benzoyl)-phenylhydrazin in 22 ml n Natronlauge werden unter Kühlen mit kaltem Wasser und unter Rühren 0,85 ml Thiophosgen tropfenweise zugegeben. Das Reaktionsgemisch wird zunächst bei Raumtemperatur 4 Stunden lang gerührt und dann noch 30 Minuten lang gekocht. Nach dem Abkühlen wird der pH-Wert des Reaktionsgemisches mit n Natriumhydrogencarbonat auf 7 eingestellt. Das Gemisch wird 30 Minuten lang gerührt und dann mit Essigsäure auf pH 5 angesäuert. Der Niederschlag wird abgesaugt, mit Wasser gewaschen und bei 80 °C getrocknet. Man erhält 4,35 g (98,5 %) 1-[(4′-Chlor-3′-sulfamoyl-benzoyl)-amino]-5-methoxycarbonyl-benzimidazol-2-thion in Form eines sandfarbenen Pulvers. Die Verbindung schmilzt nach Umkristallisieren aus einem im Verhältnis 1:2 bereiteten Gemisch von Dimethylformamid und Wasser unter Zersetzung bei 258-261 °C.

Beispiel 5

Zu der Lösung von 2,2 g 1-Amino-5-methoxycarbonyl-benzimidazol-2-thion in 8 ml Dimethylformamid werden 1,4 ml Triäthylamin gegeben. Unter Rühren werden in kleinen Portionen 2,54 g 4-Chlor-3-sulfamoyl-benzoylchlorid eingetragen. Das Reaktionsgemisch wird über Nacht stehengelassen, anderentags mit Essigsäure auf pH 5 angesäuert und mit 100 ml Wasser verdünnt. Die sich ausscheidende harzartige Masse zerfällt langsam zu einem Pulver. Das Produkt wird abgesaugt, mit Wasser gewaschen und bei 80 °C getrocknet. Das Rohprodukt wird in 10 ml Eisessig 30 Minuten lang gekocht und die Lösung dann in 50 ml Wasser eingegossen. Das sich ausscheidende Harz zerfällt bei Rühren in ein Pulver, es wird abgesaugt, mit Wasser gewaschen und bei 80 °C getrocknet. Man erhält 2,4 g (54,5 %) 1-[(4′-Chlor-3′-sulfamoyl-benzoyl)-amino]-5-methoxycarbonyl-benzimidazol-2-thion in Form eines sandfarbenen Pulvers, das nach Umkristallisieren aus einem im Verhältnis 1:2 bereiteten Gemisch von Dimethylformamid und Wasser unter Zersetzung bei 258-261 °C schmilzt.

Herstellung der Ausgangssubstanz

A) 105,6 g 2-Nitro-methoxycarbonyl-phenylhydrazin werden in einem Liter Eisessig eine Stunde lang gekocht. Die klare orangenfarbene Lösung wird im Vakuum eingedampft. Der Rückstand wird mit 1,5 Liter Wasser verrührt. Die ausfallenden Kristalle werden abgesaugt, mit Wasser gewaschen und bei 80 °C getrocknet. Man erhält 123 g (97 %) 2-Nitro-4-methoxycarbonyl-N-acetyl-phenylhydrazin in Form gelber, pulverförmiger Kristalle, die bei 182-185 °C schmelzen. Nach Umkristallisieren aus 50 %igem wässrigen Alkohol liegt der Schmelzpunkt bei 190-192 °C.

| Analyse für $C_{10}H_{11}N_3O_5$ | | | | | | |
|---|---|---|---|---|---|---|
| berechnet, %: | C | 47,43 | H | 4,38 | N | 16,59 |
| gefunden, %: | C | 47,55 | H | 4,73 | N | 16,58. |

B) 107,7 g des auf die beschriebene Weise hergestellten Benzoesäurederivates werden in 425 ml n Natronlauge 10 Minuten lang gekocht. Die erhaltene Lösung wird klarfiltriert, dann wird die dunkelrote Lösung mit Essigsäure auf pH 3-4 angesäuert. Die ausgeschiedenen Kristalle werden abfiltriert, mit Wasser gewaschen und bei 80 °C getrocknet. Man erhält 98 g (96,4 %) 2-Nitro-4-carboxy-N-acetyl-phenylhydrazin in Form gelber Kristalle, die sich bei 262-263 °C zersetzen. Nach Umkristallisieren aus 50 %igem wässrigen Alkohol schmilzt die Verbindung unter Zersetzung bei 272-274 °C.

C) 116,3 g der auf die beschriebene Weise hergestellten Nitroverbindung werden in 490 ml n Natriumhydrogencarbonatlösung gelöst. Die Lösung wird klarfiltriert, mit 10 g 10 %iger Palladiumaktivkohle versetzt und bis zur Beendigung der Wasserstoffaufnahme hydriert. Nach dem Abfiltrieren des Katalysators wird das Filtrat mit 5n Salzsäure auf pH 4 angesäuert. Die ausgefallenen Kristalle werden abfiltriert, mit Wasser gewaschen und bei 80 °C getrocknet. Man erhält 80,9 g (79,6 %) 2-Amino-4-carboxy-N-acetyl-phenylhydrazin in Form sandfarbener Kristalle. Das Produkt zersetzt sich bei 145-150 °C und schmilzt nach Umkristallisieren aus abs. Alkohol unter Zersetzung bei 156-158 °C.

| Analyse für $C_9H_{11}N_3O_3$ | | | | | | |
|---|---|---|---|---|---|---|
| berechnet, %: | C | 51,67 | H | 5,30 | N | 20,09 |
| gefunden, %: | C | 51,31 | H | 5,49 | N | 19,79. |

D) In der mit 450 ml abs. Alkohol bereiteten Lösung von 36 g Kaliumhydroxyd werden 62,75 g des auf die beschriebene Weise hergestellten Amins aufgelöst. Die Lösung wird mit 20,5 ml Schwefelkohlenstoff versetzt und unter Rühren 5 Stunden lang gekocht. Dabei scheidet sich eine gelbe Kristallmasse ab. Zu dem Gemisch werden 250 ml kochendes Wasser gegeben, wobei eine Lösung entsteht. Diese wird mit Aktivkohle geklärt, dann filtriert,und aus dem Filtrat werden im Vakuum etwa 200 ml Alkohol abdestilliert. Zu der verbliebenen Lösung werden 65 ml Essigsäure gegeben. Die ausfallenden gelben Kristalle werden abgesaugt, mit Wasser gewaschen und bei 80 °C getrocknet. Man erhält 46 g (61 %) 1-Acetamino-5-carboxy-benzimidazol-2-thion, das aus 50 %igem wässrigem Alkohol umkristallisiert wird. Die weißen Kristalle schmelzen bei 334-338 °C.

| Analyse für $C_{10}H_9N_3O_3S$ | | | | | | | | |
|---|---|---|---|---|---|---|---|---|
| berechnet, %: | C | 48,00 | H | 3,61 | N | 16,72 | S | 12,76 |
| gefunden, %: | C | 48,70 | H | 3,50 | N | 16,70 | S | 12,74 |

E) 25,1 g des auf die beschriebene Weise hergestellten Benzimidazols werden unter Rühren in 100 ml 2n Salzsäure 6 Stunden lang gekocht. Nach dem Abkühlen werden die Kristalle abgesaugt, mit Wasser gewaschen und dann bei 80 °C getrocknet. Man erhält 20,5 g (98 %) 1-Amino-5-carboxy-benzimidazol-2-thion in Form sandfarbener Kristalle. Zersetzung bei 301-304 °C.

| Analyse für $C_8H_7NO_2S$ | | | | | | | | |
|---|---|---|---|---|---|---|---|---|
| berechnet, % | C | 45,92 | H | 3,37 | N | 20,08 | S | 15,30 |
| gefunden, %: | C | 45,40 | H | 3,34 | N | 19,92 | S | 15,70. |

F) In die Suspension von 20,5 g der auf die beschriebene Weise hergestellten Carbonsäure in 250 ml Methanol wird unter Rühren Salzsäuregas bis zur Sättigung eingeleitet. Dann wird das Gemisch unter Rühren eine halbe Stunde lang gekocht, dabei wird langsam weiteres Salzsäuregas eingeleitet. Nach dem Abkühlen werden die Kristalle abgesaugt und unter kräftigem Rühren noch feucht in 80 ml n Natriumhydrogencarbonatlösung eingestreut. Nach 30 minütigem Rühren werden die Kristalle abgesaugt, mit Wasser gewaschen und bei 80 °C getrocknet. Man erhält 19,9 g (91 %) 1-Amino-5-methoxycarbonyl-benzimidazol-2-thion in Form beinfarbener Kristalle. Nach Umkristallisieren aus einem im Verhältnis 2:1 bereiteten Gemisch aus Dimethylformamid und Wasser schmilzt die Verbindung unter Zersetzung bei 239 °C.

| Analyse für $C_9H_9N_3O_2S$ | | | | | | | | |
|---|---|---|---|---|---|---|---|---|
| berechnet, %: | C | 48,42 | H | 4,06 | N | 18,82 | S | 14,36 |
| gefunden, %: | C | 48,16 | H | 3,94 | N | 18,50 | S | 14,61. |

Beispiel 6

Zu der Suspension von 44,6 g 1-Amino-5-methoxycarbonyl-benzimidazol-2-thion in 400 ml Dioxan wird die Lösung von 12,7 g wasserfreiem Natriumcarbonat in 200 ml Wasser gegeben. Unter Kühlen mit kaltem Wasser und unter Rühren wird dem Reaktionsgemisch tropfenweise die Lösung von 68 g 4-Chlor-3-[(N-dimethylamino-methyliden)-sulfamoyl]-benzoylchlorid in 400 ml Dioxan zugesetzt, wobei die Temperatur zwischen 15 und 20 °C gehalten wird. Nach der Zugabe wird das Reaktionsgemisch bei 20 °C noch 2 Stunden lang gerührt und dann klarfiltriert. Das Filtrat wird mit 2 Liter Wasser verdünnt, die ausfallenden Kristalle werden ab getrennt, mit Wasser gewaschen und bei 80 °C getrocknet. Das Rohprodukt (84,5 g) wird in 355 ml Eisessig kochend gelöst, und die noch warme Lösung wird unter energischem Rühren mit einem Liter Wasser verdünnt. Die sich ausscheidenden Kristalle werden abgesaugt, mit Wasser gewaschen und bei 80 °C getrocknet. Man erhält in Form eines weißen Pulvers 74,4 g (75,5 %) 1-[(4′-Chlor-3′-/N-dimethylamino-methyliden/-sulfamoyl-benzoyl)-amino]-5-methoxycarbonyl-benzimidazol-2-thion, das sich bei 238-239 °C zersetzt.

| Analyse für $C_{19}H_{18}ClN_5O_5S_2$ | | | | | | | | | |
|---|---|---|---|---|---|---|---|---|---|
| berechnet, %: | C | 45,91 | H | 3,85 | N | 14,10 | Cl | 7,13 | S | 12,90 |
| gefunden, %: | C | 45,45 | H | 3,59 | N | 13,95 | Cl | 7,10 | S | 13,16 |

86,8 g der auf die beschriebene Weise hergestellten Verbindung werden in 500 ml 2n Natronlauge bei 50 °C 8 Stunden lang gerührt. Die Lösung wird mit Aktivkohle geklärt, filtriert und der pH-Wert des Filtrats mit 2n Salzsäure auf 6 gestellt, wobei gekühlt und gerührt wird. Nach Zusatz von 100 ml Äthanol wird der Niederschlag abgesaugt, mit Wasser gewaschen und bei 80 °C getrocknet. Man erhält 60,8 g (81,4 %) 1-[-(4′-Chlor-3′-sulfamoyl-benzoyl)-amino]-5-carboxy-benzimidazol-2-thion, das sich bei 285-290 °C zersetzt. Das Produkt wird auf die im Beispiel 1 beschriebene Weise gereinigt und stimmt dann in allen seinen Eigenschaften mit der gemäß Beispiel 1 erhaltenen Verbindung überein.

Beispiel 7

In die Lösung von 2,06 g 2-Amino-4-methoxycarbonyl-N-(4′-chlor-3′-sulfamoyl-benzoyl)-phenylhydrazin in 10 ml Dimethylformamid werden 3,66 g N,N′-Di-(methoxycarbonyl)-S-methyl-isothioharnstoff eingetragen, und die erhaltene Lösung wird 3 Stunden lang gekocht. Die Lösung wird im Vakuum eingedampft und der Rückstand in 10 ml Eisessig warm gelöst. Die Lösung wird mit Aktivkohle geklärt, filtriert und das Filtrat in 100 ml Wasser gegossen. Die ausfallenden Kristalle werden abfiltriert, mit Wasser gewaschen und bei 80 °C getrocknet. Man erhält 3,7 g (87 %) 1-[(4′-Chlor-3′-sulfamoyl-benzoyl)-amino]-5-methoxycarbonyl-benzimidazol-2-on, das sich bei 210-216 °C zersetzt. 3,7 g der Verbindung werden in 37 ml 2n Natronlauge bei 60 °C 5 Stunden lang gerührt. Die erhaltene Lösung wird mit Aktivkohle geklärt, filtriert und das Filtrat mit 2n Salzsäure angesäuert. Die ausgefallenen Kristalle werden abgesaugt, mit Wasser gewaschen und bei 80 °C getrocknet. In Form sandfarbener Kristalle erhält man 2,6 g (72 %) 1-[(4′-Chlor-3′-sulfamoyl-benzoyl)-amino]-5-carboxy-benzimidazol-2-on. Nach Umkristallisieren aus 50 %igem wässrigem Alkohol schmilzt die Verbindung unter Zersetzung bei 339 °C.

| Analyse für $C_{15}H_{11}ClN_4O_6S$ | | | | | | | | |
|---|---|---|---|---|---|---|---|---|
| berechnet, %: | C | 43,85 | H | 2,70 | N | 13,64 | S | 7,80 |
| gefunden, %: | C | 43,57 | H | 2,86 | N | 13,58 | S | 7,42. |

Beispiel 8

In einer aus 0,58 g Natrium und 100 ml Methanol bereiteten Natriummethylatlösung werden 11 g 1-[-(4′Chlor-3′-sulfamoyl-benzoyl)-amino]-5-methoxycarbonyl-benzimidazol-2-tion gelöst, zu der Lösung 1,56 ml Methyljodid gegeben und die erhaltene Lösung 3 Stunden lang gekocht. Dann wird das Methanol abdestilliert und der Rückstand mit Wasser verrieben. Die pulverartigen Kristalle werden abgesaugt, mit Wasser gewaschen und bei 80 °C getrocknet. Man erhält 10,8 g (95 %) 1-[(4′-Chlor-3′-sulfamoyl-benzoyl)-amino]-5-methoxycarbonyl-2-methylthio-benzimidazol. Zersetzungspunkt: 186-188 °C.

| Analyse für $C_{17}H_{15}ClN_4O_5S_2$ | | | | | | | | | | | | |
|---|---|---|---|---|---|---|---|---|---|---|---|---|
| berechnet, %: | C | 44,88 | H | 3,32 | N | 12,32 | Cl | 7,79 | S | 14,10 |
| gefunden, %: | C | 44,75 | H | 3,68 | N | 12,50 | Cl | 7,60 | S | 13,91 |

Beispiel 9

10 g des gemäß Beispiel 8 hergestellten Esters werden in 50 ml 2n Natriumhydroxyd auf die im Beispiel 1 beschriebene Weise hydrolysiert. Man erhält 9 g (94 %) 1-[(4′-Chlor-3′-sulfamoyl-benzoyl)-amino]-5-carboxy-2-methylthio-benzimidazol, das aus 50 %igem wässrigem Alkohol umkristallisiert wird. Die erhaltene Substanz enthält 1,5 Moleküle Kristallwasser und zersetzt sich bei 214-222 °C.

| Analyse für $C_{16}H_{13}ClN_4O_5S_2$.1,5 $H_2O$ | | | | | | | | | | |
|---|---|---|---|---|---|---|---|---|---|---|
| berechnet, %: | C | 41,06 | H | 3,44 | N | 11,97 | Cl | 7,57 | S | 13.07 |
| gefunden, %: | C | 41,00 | H | 3,35 | N | 11,71 | Cl | 7,11 | S | 13,15. |

Beispiel 10

In einer aus 0,35 g Natrium und 60 ml Methanol bereiteten Natriummethanolatlösung werden 6,6 g 1-[-(4′-Chlor-3′-sulfamoyl-benzoyl)-amino]-5-methoxycarbonyl-benzimidazol-2-thion gelöst. Das Gemisch wird mit 2 ml Benzylchlorid versetzt und unter Rühren 16 Stunden lang gekocht. Dann wird das Gemisch im Vakuum eingedampft, der Rückstand mit Wasser verrieben und die Festsubstanz abfiltriert. Nach dem Trocknen wird das Produkt an einer Silikagelsäule mit Benzol und Aceton im Verhältnis 2:1 chromatographiert. Man erhält in Form eines weißen Pulvers 5,37 g (67,4 %) 1-[(4′-Chlor-3′-sulfamoyl-benzoyl)-amino]-2-benzylthio-5-methoxycarbonyl-benzimidazol. Zersetzungspunkt: 111-118 °C.
DC (Chloroform/Essigsäure/Methanol): $R_f$ 0,90.

| Analyse für $C_{23}H_{19}ClN_4O_5S_2$ | | | | | | | | | | |
|---|---|---|---|---|---|---|---|---|---|---|
| berechnet, %: | C | 52,02 | H | 3,61 | N | 10,55 | Cl | 6,68 | S | 12,08 |
| gefunden, %: | C | 52,32 | H | 4,00 | N | 9,90 | Cl | 6,00 | S | 12,00 |

Beispiel 11

Eine Suspension von 5,3 g 1-[(4′-Chlor-3′-sulfamoyl-benzoyl)-amino]-2-benzylthio-5-methoxycarbonyl-benzimidazol in 30 ml 2n Natronlauge wird bei 50 °C 4 Stunden lang gerührt. Dabei geht ein großer Teil des Ausgangsstoffes in Lösung. Dann wird vom Umgelösten abfiltriert und das Filtrat mit 30 ml 2n Salzsäure neutralisiert. Der entstehende weiße Niederschlag wird abgesaugt, mit Wasser gewaschen und dann getrocknet. Man erhält in Form eines weißen Pulvers 4,9 g (94,8 %) 1-[(4′-Chlor-3′-sulfamoyl-benzoyl)-amino]-2-benzylthio-5-carboxy-benzimidazol, das sich bei 190-196 °C zersetzt.

| Analyse für $C_{23}H_{19}ClN_4O_5S_2$ | | | | | | | | | | |
|---|---|---|---|---|---|---|---|---|---|---|
| berechnet, %: | C | 51,10 | H | 3,31 | N | 10,84 | Cl | 6,86 | S | 12,40 |
| gefunden, %: | C | 49,23 | H | 3,77 | N | 11,00 | Cl | 6,84 | S | 11,40 |

Beispiel 12

Zu der Suspension von 9 g 1-[(4′-Chlor-3′-sulfamoyl-benzoyl)-amino]-2-methylthio-5-methoxycarbonyl-benzimidazol in 20 ml Eisessig werden bei 20 °C 3,85 ml 35 %ige Wasserstoffperoxydlösung tropfenweise zugegeben. Das Gemisch wird auf dem kochenden Wasserbad 1,5 Stunden lang gerührt. Nach dem

Abkühlen wird die Festsubstanz abgesaugt, mit Wasser gewaschen und dann getrocknet. Man erhält in Form eines weißen Pulvers 6,4 g (65,7 %) 1-[(4′-Chlor-3′-sulfamoyl-benzoyl)-amino]-2-methylsulfonyl-5-methoxycarbonyl-benzimidazol, das nach Umkristallisieren aus einem 1:1-Gemisch von Dimethylformamid und Wasser bei 254-255 °C unter Zersetzung schmilzt.

| Analyse für $C_{17}H_{15}ClN_4O_4S_2$ | | | | | | | | | | |
|---|---|---|---|---|---|---|---|---|---|---|
| berechnet, %: | C | 41,93 | H | 3,10 | N | 11,50 | Cl | 7,28 | S | 13,17 |
| gefunden, %: | C | 42,01 | H | 3,15 | N | 11,34 | Cl | 7,35 | S | 13,01 |

Beispiel 13

Die Suspension von 5,4 g 1-[(4′-Chlor-3′-sulfamoyl-benzoyl)-amino]-2-methyl-sulfonyl-5-methoxycarbonyl-benzimidazol in 30 ml 2n Natronlauge wird bei 50 °C 4 Stunden lang gerührt. Es bildet sich eine klare lila Lösung. Diese wird mit Aktivkohle geklärt, filtriert und mit 2n Salzsäure auf pH 2 gestellt. Der weiße Niederschlag wird abfiltriert, mit Wasser gewaschen und dann getrocknet. In Form eines weißen Pulvers erhält man 4,43 g (85,7 %) 1-[(4′-Chlor-3′-sulfamoyl-benzoyl)-amino]-2-methylsulfonyl-5-carboxy-benzimidazol, das sich bei 222-225 °C zersetzt. Durch Umkristallisieren aus Dimethylformamid und Wasser im Verhältnis 3:2 ändert sich der Zersetzungspunkt nicht.

| Analyse für $C_{16}H_{23}ClN_4O_7S_2$ | | | | | | | | | | |
|---|---|---|---|---|---|---|---|---|---|---|
| berechnet, %: | C | 40,64 | H | 2,77 | N | 11,85 | Cl | 7,50 | S | 13,56 |
| gefunden, %: | C | 40,07 | H | 3,01 | N | 12,02 | Cl | 7,65 | S | 13,29. |

Beispiel 14

Zu der Suspension von 11,45 g 1-Amino-2-methyl-5-methoxycarbonyl-benzimidazol in 25 ml Pyridin werden 17,25 g 4-Chlor-3-(N-dimethylamino-methyliden)-sulfamoylbenzoyl-chlorid gegeben. Das Reaktionsgemisch wird auf etwa 60-70 °C erwärmt, wobei sich eine gelbe Lösung bildet. Diese wird über Nacht stehengelassen und anderntags mit 200 ml Wasser versetzt. Ein gelbes Harz scheidet sich ab, das nach ein paar Minuten Rühren zu einem sandfarbenen Pulver zerfällt. Das Produkt wird abgesaugt, mit Wasser gewaschen und dann getrocknet. In Form eines sandfarbenen Pulvers erhält man 23,9 g (90 %) 1-[(4′-Chlor-3′-N-dimethylamino-methyliden-sulfamoyl-benzoyl)-amino]-2-methyl-5-methoxycarbonyl-benzimidazol, das sich bei 240-245 °C zersetzt. Nach Umkristallisieren aus Nitromethan liegt der Zersetzungspunkt bei 260-263 °C.

| Analyse für $C_{20}H_{20}ClN_5O_5S$ | | | | | | | | | | |
|---|---|---|---|---|---|---|---|---|---|---|
| berechnet, %: | C | 50,26 | H | 4,22 | N | 14,65 | Cl | 7,42 | S | 6,71 |
| gefunden, %: | C | 49,85 | H | 4,31 | N | 15,12 | Cl | 7,44 | S | 6,52 |

Herstellung der Ausgangsstoffe

A) Zu der Suspension von 97,5 g 2-Amino-4-carboxy-N-acetyl-phenylhydrazinin 450 ml Dichlormethan werden 235 ml Acetanhydrid gegeben. Das Reaktionsgemisch wird bei Raumtemperatur 3 Stunden lang gerührt. Die Kristalle werden abgesaugt, mit Dichlormethan gewaschen und bei 60 °C getrocknet. In Form eines schneeweißen Kristallpulvers erhält man 101,2 g (86,5 %) 2-Acetamino-4-carboxy-N-acetyl-phenylhydrazin, das sich bei 238 °C zersetzt.

| Analyse für $C_{11}H_{13}N_3O_4$ | | | | | | |
|---|---|---|---|---|---|---|
| berechnet, %: | C | 52,58 | H | 5,21 | N | 16,93 |
| gefunden, %: | C | 52,82 | H | 5,12 | N | 17,20. |

B) Die auf die beschriebene Weise hergestellte Acetylverbindung (101,2 g) wird in 800 ml Essigsäure 13 Stunden lang gekocht. Dann wird die Lösung im Vakuum eingedampft und der Rückstand mit 800 ml Wasser verrührt. Die sich abscheidenden Kristalle werden abgesaugt, mit Wasser gewaschen und bei 80 °C getrocknet. In Form eines weißen Pulvers erhält man 73,1 g (78,5 %) 1-Acetamino-5-carboxy-2-methyl-benzimidazol, das sich bei 293-294 °C zersetzt.

| Analyse für $C_{11}H_{11}N_3O_3$ | | | | | | |
|---|---|---|---|---|---|---|
| berechnet, %: | C | 56,65 | H | 4,75 | N | 18,02 |
| gefunden, %: | C | 56,71 | H | 4,62 | N | 17,89 |

C) Das auf die beschriebene Weise hergestellte Benzimidazolderivat (73,1 g) wird in 310 ml 2n Salzsäure 5 Stunden lang gekocht. Die entstandene Lösung wird noch warm mit Aktivkohle geklärt und dann filtriert. Die beim Abkühlen ausfallenden Kristalle werden abgesaugt, mit wenig Wasser gewaschen und bei 80 °C getrocknet. In Form schneeweißer, glänzender Plättchen erhält man 68,9 g (96 %) 1-Amino-2-methyl-5-carboxy-benzimidazol-hydrochlorid, das sich bei 287-288 °C zersetzt.

| Analyse für $C_9H_{10}ClN_3O_2$ | | | | | | | | |
|---|---|---|---|---|---|---|---|---|
| berechnet, %: | C | 47,48 | H | 4,43 | N | 18,46 | Cl | 15,57 |
| gefunden, %: | C | 47,85 | H | 4,83 | N | 18,15 | Cl | 15,20. |

D) 68,1 des auf die beschriebene Weise hergestellten Hydrochlorids werden in einem Liter Methanol suspendiert. Im weiteren arbeitet man auf die im Beispiel 5/F beschriebene Weise. Man erhält 48 g (78 %) 1-Amino-2-methyl-5-methoxycarbonyl-benzimidazol, das bei 220-222 °C schmilzt.

| Analyse für $C_{10}H_{11}N_3O_2$ | | | | | | |
|---|---|---|---|---|---|---|
| berechnet, %: | C | 58,53 | H | 5,40 | N | 20,48 |
| gefunden, %: | C | 58,10 | H | 5,38 | N | 20,25. |

E) Zu der mit 50 ml Thionylchlorid bereiteten Suspension von 14,5 g 4-Chlor-3-(N-dimethylamino-methyliden -sulfamoyl)-benzoesäure (Herstellung: holländische Patentanmeldung 76 04356) werden 2 Tropfen Dimethylformamid gegeben. Das Reaktionsgemisch wird unter Rühren 2 Stunden lang gekocht, dann klarfiltriert und das Filtrat im Vakuum eingedampft. In Form einer schneeweißen, festen Substanz erhält man 12,7 g (82 %) 4-Chlor-3-(N-dimethylamino-methyliden,-sulfamoyl)benzoyl-chlorid, das bei 140 °C schmilzt. Nach Umkristallisieren aus Benzol liegt der Schmelzpunkt bei 154-155 °C.

| Analyse für $C_{10}H_{10}Cl_2N_2O_3S$ | | | | | | | | | | |
|---|---|---|---|---|---|---|---|---|---|---|
| berechnet, %: | C | 38,84 | H | 3,26 | N | 9,06 | Cl | 22,93 | S | 10,37 |
| gefunden, %: | C | 38,28 | H | 3,07 | N | 8,94 | Cl | 23,14 | S | 10,58. |

<u>Beispiel 15</u>

Zu 25 g 2-Amino-4-methoxycarbonyl-N-[(4′-chlor-3′-N-dimethylamino-methyliden-sulfamoyl)-benzoyl]-phenylhydrazin werden 80 ml Acetanhydrid gegeben. Das Reaktionsgemisch wird über Nacht stehengelassen und anderntags mit 250 ml Wasser verrührt. Die ausgefallenen Kristalle werden abfiltriert, mit Wasser gewaschen und dann getrocknet. Man erhält 26 g 2-Acetamino-4-methoxycarbonyl-N-[(4′-chlor-3′-N-dimethylamino-methyliden-sulfamoyl)-benzoyl]-phenylhydrazin, das sich bei 218-220 °C zersetzt.

Die erhaltene Verbindung (26 g) wird 5 Stunden lang in Eisessig gekocht. Die klare gelbe Lösung wird im Vakuum eingedampft und der Rückstand mit Wasser verrieben. Die ausgefallenen Kristalle werden abgesaugt, mit Wasser gewaschen und dann getrocknet. In Form eines sandfarbenen Pulvers werden 24,5 g (auf die Ausgangsaminoverbindung bezogen 98 %) 1-[(4′-Chlor-3′-N-dimethylamino-methyliden-sulfamoy l-benzoyl)-amino]-2-methyl-5-methoxycarbonyl-benzimidazol erhalten. Zersetzungspunkt: 245-255 °C, nach Umkristallisieren aus Nitromethan 260-263 °C. Das Produkt ist in jeder Hinsicht mit dem Produkt gemäß Beispiel 14 identisch.

Herstellung der Ausgangsstoffe

A) Zu der Suspension von 70,76 g 2-Nitro-4-methoxycarbonyl-phenylhydrazin in 670 ml Dioxan wird die Lösung von 17,75 g kristallinem Natriumcarbonat in 330 ml Wasser gegeben. Das Reaktionsgemisch wird unter Rühren tropfenweise mit der Lösung von 103,6 g 4-Chlor-3-(N-dimethylamino-methyliden - sulfamoyl)-benzoylchlorid in 280 ml Dioxan versetzt. Während des Zutropfens bildet sich eine dunkle, klare Lösung. Der pH-Wert des Reaktionsgemisches wird von Zeit zu Zeit kontrolliert und, falls notwendig, durch Zusatz von n Natriumhydrogencarbonatlösung bei etwa 7 gehalten. Nach Beendigung der Zugabe wird das Gemisch noch eine Stunde lang gerührt und anschließend mit einem Liter Wasser verdünnt. Der ausfallende Niederschlag wird abgesaugt, mit Wasser gewaschen und bei 80 °C getrocknet. Man erhält 127,8 g (78,8 %) 2-Nitro-4-methoxycarbonyl-N-[4′-chlor-3′-(N-dimethylamino-methyliden)-sulfamoyl -benzoyl] -phenylhydrazin, das bei 243-247 °C schmilzt. Nach dem Umkristallisieren aus Nitromethan liegt der Schmelzpunkt bei 255-256 °C.

| Analyse für $C_{18}H_{18}ClN_5O_7S$ | | | | | | | | | | | | |
|---|---|---|---|---|---|---|---|---|---|---|---|---|
| berechnet, %: | C | 44,68 | H | 3,75 | N | 14,47 | Cl | 7,33 | S | 6,63 |
| gefunden, %: | C | 44,70 | H | 3,69 | N | 14,70 | Cl | 7,53 | S | 6,79 |

B) Zu der Suspension von 61 g der auf die beschriebene Weise hergestellten Nitroverbindung in 40 ml 96 %igem Alkohol werden 5 g 10 %ige Palladiumaktivkohle gegeben. Das Gemisch wird auf 60-70 °C erwärmt und bei dieser Temperatur tropfenweise mit 250 ml 30 %iger wäßriger Natriumhypophosphitlösung versetzt, wobei die Dosiergeschwindigkeit so gewählt wird, daß das Gemisch nicht zu sehr schäumt. Nach Beendigung der Zugabe wird das Gemisch bei 60-70 °C noch 30 Minuten lang gerührt und nach dem Abkühlen filtriert. Die Festsubstanz wird mit 250 ml Dioxan ausgewaschen. Das Filtrat wird im Vakuum eingedampft. Der Rückstand wird mit 200 ml 50 %igem wässrigem Alkohol verrieben, die entstandenen Kristalle werden abgesaugt, mit 50 %igem Alkohol gewaschen und bei 80 °C getrocknet. In Form butterfarbener Kristalle erhält man 40 g (68 %) 2-Amino-4-methoxycarbonyl-N-[4′-chlor-3′-(N-dimethylamino-methyliden) -sulfamoyl -benzoyl]-phenylhydrazin, das sich bei 219-220 °C zersetzt.

| Analyse für $C_{18}H_{20}ClN_5O_5S$ | | | | | | | | |
|---|---|---|---|---|---|---|---|---|
| berechnet, %: | C | 47,63 | H | 4,44 | N | 15,43 | Cl | 7,81 |
| gefunden, %: | C | 47,80 | H | 4,25 | N | 15,44 | Cl | 7,41. |

Beispiel 16

23,9 g 1-[(4′-Chlor-3′- N-dimethylamino-methyliden-sulfamoyl -benzoyl)amino] -2-methyl-5-methoxycarbonyl-benzimidazol werden in 150 ml 2n Natronlauge bei 50 °C bis zum Aufhören der Ammoniakentwicklung gerührt. Es bildet sich eine gelbe Lösung. Diese wird nach dem Abkühlen mit 2n Salzsäure auf pH 1 angesäuert. Der ausgefallene Niederschlag wird abgesaugt, mit Wasser gewaschen und dann getrocknet. In Form eines weißen Pulvers erhält man 19,8 g (97 %) 1-[(4′-Chlor-3′-sulfamoyl-benzoyl)-amino]-2-methyl-5-carboxy-benzimidazol, das nach Umkristallisieren aus 50 %igem wässrigem Alkohol einen Zersetzungspunkt von 293-296 °C aufweist.

| Analyse für $C_{16}H_{13}ClN_4O_5S$ | | | | | | | | | | | | |
|---|---|---|---|---|---|---|---|---|---|---|---|---|
| berechnet, %: | C | 47,00 | H | 3,20 | N | 13,71 | Cl | 8,67 | S | 7,85 |
| gefunden, %: | C | 45,80 | H | 3,71 | N | 13,93 | Cl | 8,80 | S | 7,74. |

Beispiel 17

Man verfährt wie in Beispiel 14, verwendet jedoch als Ausgangssubstanz 10,63 g 1-Amino-5-methoxycarbonyl-benzimidazol. Man erhält 21,1 g (82 %) 1-[(4′-Chlor-3′-N-dimethylamino-methyliden-sulfamoyl-benzoyl)-amino]-5-methoxycarbonyl-benzimidazol in Form eines blaßgelben Pulvers, das sich bei 249-252 °C zersetzt.

| Analyse für $C_{19}H_{18}ClN_5S$ | | | | | | | | |
|---|---|---|---|---|---|---|---|---|
| berechnet, %: | C | 49,19 | H | 3,91 | N | 15,10 | S | 6,91 |
| gefunden, %: | C | 49,62 | H | 4,36 | N | 15,30 | S | 6,68. |

Herstellung der Ausgangsstoffe

A) Zu der Lösung von 25,3 g 2-Nitro-4-methoxycarbonyl-N-acetyl-phenylhydrazin in 380 ml Methylcellosolv gibt man 2,5 g 10 %ige Palladiumaktivkohle und hydiert das Gemisch in einem Schüttelapparat bis zur Beendigung der Wasserstoffaufnahme. Nach dem Abfiltrieren des Katalysators wird das Filtrat im Vakuum eingedampft und der Rückstand mit 100 ml Wasser verrührt. Die sich abscheidenden Kristalle werden abgesaugt, mit Wasser gewaschen und bei 80 °C getrocknet. In Form sandfarbener Kristalle erhält man 19,1 g (85,6 %) 2-Amino-4-methoxycarbonyl-N-acetyl-phenylhydrazin, das bei 175-178 °C schmilzt. Nach Umkristallisieren aus Methanol liegt der Schmelzpunkt bei 180-182 °C.

| Analyse für $C_{10}H_{13}N_3O_3$ | | | | | | |
|---|---|---|---|---|---|---|
| berechnet, %: | C | 53,80 | H | 5,87 | N | 18,82 |
| gefunden, %: | C | 53,17 | H | 5,94 | N | 18,93 |

B) 22,3 g des auf die beschriebene Weise hergestellten Phenylhydrazinderivates werden in 150 ml wasserfreier Ameisensäure 5 Stunden lang gekocht. Die durchsichtige, schwach lila gefärbte Lösung wird im Vakuum eingedampft. Auf das zurückbleibende Harz werden 250 ml Wasser gegossen. Es entsteht eine Lösung, aus der sich bald weiße Kristalle ausscheiden. Man läßt einige Stunden abstehen und saugt dann die Kristalle ab. Sie werden mit Wasser gewaschen und bei 80 °C getrocknet. Man erhält in Form weißer Kristalle 17,2 g (76 %) 1-Acetamino-5-methoxycarbonyl-benzimidazol. Zersetzungspunkt: 210-212 °C.

| Analyse für $C_{11}H_{11}N_3O_3$ | | | | | | |
|---|---|---|---|---|---|---|
| berechnet, %: | C | 56,65 | H | 4,75 | N | 18,02 |
| gefunden, %: | C | 56,55 | H | 4,67 | N | 18,28 |

C) 32,5 g des auf die beschriebene Weise hergestellten Benzimidazolderivates werden in 140 ml 2n Salzsäure 5 Stunden lang gekocht. Die erhaltene Lösung wird heiß mit Aktivkohle geklärt und dann filtriert. Die sich beim Abkühlen aus dem Filtrat abscheidenden weißen Kristalle werden abgesaugt, mit wenig Wasser gewaschen und bei 80 °C getrocknet. In Form schneeweißer Kristalle erhält man 23,2 g (78 %) 1-Amino-5-carboxy-benzimidazol-hydrochlorid, das bei 325-330 °C schmilzt.

| Analyse für $C_8H_8ClN_3O_2$ | | | | | | | | |
|---|---|---|---|---|---|---|---|---|
| berechnet, %:<br>gefunden, %: | C<br>C | 44,98<br>44,49 | H<br>H | 3,77<br>3,80 | N<br>N | 19,67<br>19,68 | Cl<br>Cl | 16,60<br>16,52 |

D) 26 g des auf die beschriebene Weise hergestellten Benzimidazolderivates werden auf die im Beispiel 5/F beschriebene Weise mit 270 ml salzsaurem Methanol umgesetzt. Das entstandene Hydrochlorid wird mit 220 ml n Natriumhydrogencarbonatlösung zersetzt. Man erhält 19,3 g (83 %) 1-Amino-5-methoxycarbonyl-benzimidazol, das bei 194-195 °C schmilzt.

| Analyse für $C_9H_9N_3O_2$ | | | | | | |
|---|---|---|---|---|---|---|
| berechnet, %:<br>gefunden, %: | C<br>C | 56,54<br>56,95 | H<br>H | 4,75<br>4,63 | N<br>N | 21,98<br>21,62 |

Beispiel 18

28,7 g 2-Amino-4-methoxycarbonyl-N-[4'-chlor-3'-(N-dimethylamino-methyliden)-sulfamoyl -benzoyl]-phenylhydrazin werden in 200 ml wasserfreier Ameisensäure 6 Stunden lang gekocht. Die Lösung wird im Vakuum eingedampft und der Rückstand mit Wasser verrieben. Die ausgefallenen Kristalle werden abgesaugt, mit Wasser gewaschen und dann getrocknet. In Form eines sandfarbenen Pulvers erhält man 19 g (64 %) 1-[(4'-Chlor-3'-N-dimethylamino-methyliden-sulfamoyl-benzoyl)-amino]-5-methoxycarbonyl-benzimidazol, das sich bei 240-253 °C zersetzt.Das Produkt ist in jeder Hinsicht mit dem Produkt gemäß Beispiel 17 identisch.

Beispiel 19

Man arbeitet gemäß Beispiel 16, geht jedoch von 6,5 g 1-[(4'-Chlor-3'-(N-dimethylamino-methyliden-sulfamoyl-benzoyl)-amino]-5-methoxycarbonyl-benzimidazol und 40 ml 2n Natronlauge aus. Man erhält 5,1 g (92,8 %) 1-[(4'-Chlor-3'-sulfamoyl-benzoyl)-amino] -5-carboxy-benzimidazol, das nach Umkristallisieren aus 50 %igem wässrigem Alkohol einen Zersetzungspunkt von 290-291 °C aufweist.

| Analyse für $C_{15}H_{11}ClN_4O_5S$ | | | | | | | | | | |
|---|---|---|---|---|---|---|---|---|---|---|
| berechnet, %:<br>gefunden, %: | C<br>C | 45,63<br>45,27 | H<br>H | 2,81<br>2,79 | N<br>N | 14,19<br>13,85 | Cl<br>Cl | 8,98<br>8,93 | S<br>S | 8,12<br>7,71 |

Beispiel 20

Ausgehend von 2,32 g 2-Amino-4-cyano-N-(4'-Chlor-3'-sulfamoyl-benzoyl)-phenylhydrazin, 10 ml Methanol, 2,52 ml Schwefelkohlenstoff, 1,07 g Kaliumhydroxyd und 4,5 ml abs. Alkohol arbeitet man auf die im Beispiel 1 beschriebene Weise. Zum Ansäuern des Reaktionsgemisches werden 24 ml Essigsäure vewendet. Die erhaltenen 2,32 g (97 %) Rohprodukt werden an einer Silikagelsäure mit einem im Verhältnis 1:1 bereiteten Benzol-Aceton-Gemisch als Elutionsmittel chromatographiert. Man erhält in Form weißer Kristalle das 1-[(4'-Chlor-3'-sulfamoyl-benzoyl)-amino]-5-cyano-benzimidazol-2-thion, das bei 315 °C schmilzt.

| Analyse für $C_{15}H_{10}ClN_5O_3S_2$ | | | | | | | | | | |
|---|---|---|---|---|---|---|---|---|---|---|
| berechnet, %:<br>gefunden, %: | C<br>C | 44,16<br>44,40 | H<br>H | 2,47<br>2,67 | N<br>N | 17,17<br>16,94 | Cl<br>Cl | 8,69<br>8,96 | S<br>S | 15,72<br>15,33 |

Herstellung der Ausgangsstoffe

A) Ausgehend von 8,1 g 2-Nitro-4-cyano-phenylhydrazin (Herstellung: Beilst. 9, II, 298), 114 ml Dioxan, der Lösung von 2,4 g kristallinem Natriumcarbonat in 46 ml Wasser und der mit 53 ml Dioxan bereiteten Lösung von 11,6 g 4-Chlor-3-sulfamoyl-benzoylchlorid arbeitet man auf die im Beispiel 1/B beschriebene Weise. Nach dem Eindampfen des Reaktionsgemisches wird der Rückstand mit Wasser behandelt. Der gelbe Niederschlag wird abgesaugt, mit Wasser gewaschen und bei 80 °C getrocknet. Man erhält 3,75 g (65 %) 2-Nitro-4-cyano-N-(4′-chlor-3′-sulfamoyl -benzoyl)-phenylhydrazin, dessen Zersetzungspunkt nach Umkristallisieren aus Essigsäure bei 292-295 °C liegt.

| Analyse für $C_{14}H_{10}ClN_5O_5S$ . 1/2 $CH_3COOH$ | | | | | | | | | | |
|---|---|---|---|---|---|---|---|---|---|---|
| berechnet, %: | C | 40,63 | H | 2,92 | N | 16.92 | Cl | 8,57 | S | 7,78 |
| gefunden, %: | C | 40,06 | H | 2,52 | N | 16,85 | Cl | 8,23 | S | 7,65 |

B) 8,54 g der auf die beschriebene Weise hergestellten Nitroverbindung werden in 500 ml Methylcellosolv gelöst und in Gegenwart von 1 g Raney-Nickel bei Normaldruck und Raumtemperatur bis zur Beendigung der Wasserstoffaufnahme hydriert. Der Katalysator wird abfiltriert und das Filtrat im Vakuum eingedampft. Der Rückstand wird mit Wasser verrieben. Die ausgeschiedenen weißen Kristalle werden abgesaugt, mit Wasser gewaschen und bei 80 °C getrocknet. Man erhält 6,52 g (80 %) 2-Amino-4-cyano-N-(4′-chlor-3′-sulfamoyl-benzoyl)-phenylhydrazin, das sich bei 205 °C zersetzt.

| Analyse für $C_{14}H_{12}ClN_5O_3S$ | | | | | | | | | | |
|---|---|---|---|---|---|---|---|---|---|---|
| berechnet, %: | C | 45,96 | H | 3,31 | N | 19,15 | Cl | 9,69 | S | 8,77 |
| gefunden, %: | C | 46,06 | H | 3,28 | N | 19,21 | Cl | 9,81 | S | 8,80 |

Beispiel 21

Ausgehend von 2,9 g 2-Amino-4-benzoyl-N-(4′-chlor-3′-sulfamoyl-benzoyl)-phenylhydrazin, 10 ml Methanol, 2,8 ml Schwefelkohlenstoff und der mit 4,5 ml abs. Alkohol bereiteten Lösung von 1,2 g Kaliumhydroxyd arbeitet man auf die im Beispiel 1 beschriebene Weise. Das Reaktionsgemisch wird mit 24 ml Essigsäure angesäuert. Das Rohprodukt wird im nutschfeuchten Zustand aus 96 %igem Alkohol umkristallisiert. Man erhält 1,7 g (57 %) 1-[(4′-Chlor-3′-sulfamoyl-benzoyl)-amino]-5-benzoyl-benzimidazol-2-thion, das sich bei 233 °C zersetzt.

| Analyse für $C_{21}H_{15}ClN_4O_4S_2$ | | | | | | | | | | |
|---|---|---|---|---|---|---|---|---|---|---|
| berechnet, %: | C | 51,79 | H | 3,11 | N | 11,51 | Cl | 7,28 | S | 13,17 |
| gefunden, %: | C | 51,30 | H | 3,47 | N | 11,39 | Cl | 7,40 | S | 13,50 |

Herstellung der Ausgangsstoffe

A) Zu der Suspension von 52,33 g 3-Nitro-4-chlor-benzophenon in 200 ml abs. Alkohol werden unter Rühren 20 ml Hydrazinhydrat gegeben. Das Reaktionsgemisch wird bei Raumtemperatur 75 Minuten lang gerührt und dann langsam bis zum Sieden erhitzt. Nach Abklingen der heftigen Reaktion wird das Gemisch noch eine Stunde lang gekocht. Nach dem Abkühlen werden die Kristalle abgesaugt, mit Wasser gründlich gewaschen und bei 80 °C getrocknet. In Form orangeroter Kristalle erhält man 38,62 g (75 %) 2-Nitro-4-benzoyl-phenylhydrazin, das bei 163-164 °C schmilzt.

| Analyse für $C_{13}H_{11}N_3O_3$ | | | | | | |
|---|---|---|---|---|---|---|
| berechnet, %: | C | 60,69 | H | 4,31 | N | 16,33 |
| gefunden, %: | C | 60,60 | H | 4,15 | N | 16,40 |

B) Man arbeitet auf die im Beispiel 1/B beschriebene Weise, geht jedoch von 28 g 2-Nitro-4-benzoyl-phenylhydrazin, 272 ml Dioxan, der Lösung von 5,8 g wasserfreiem Natriumcarbonat in 110 ml Wasser und der mit 217 ml Dioxan bereiteten Lösung von 27,6 g 4-Chlor-3-sulfamoyl-benzoylchlorid aus. Man erhält 29,1 g (61 %) 2-Nitro-4-benzoyl-N-(4′-chlor-3′-sulfamoyl-benzoyl)-phenylhydrazin, dessen Zersetzungspunkt nach Umkristallisieren aus Methanol bei 148 °C liegt.

| Analyse für $C_{20}H_{15}ClN_4O_6S$ | | | | | | | | | | |
|---|---|---|---|---|---|---|---|---|---|---|
| berechnet, %: | C | 50,58 | H | 3,18 | N | 11,80 | Cl | 7,47 | S | 6,75 |
| gefunden, %: | C | 49,59 | H | 3,15 | N | 11,46 | Cl | 7,71 | S | 6,46 |

C) Ausgehend von 23,7 g der auf die beschriebene Weise hergestellten Nitroverbindung und 500 ml Methanol arbeitet man auf die im Beispiel 20/B beschriebene Weise und erhält 19,7 g (88,5 %) 2-Amino-4-benzoyl-N-(4′-chlor-3′-sulfamoyl-benzoyl)-phenylhydrazin, das sich bei 162 °C zersetzt.

| Analyse für $C_{20}H_{17}ClN_4O_4S$ | | | | | | | | | | |
|---|---|---|---|---|---|---|---|---|---|---|
| berechnet, %: | C | 53,99 | H | 3,85 | N | 12,59 | Cl | 7,97 | S | 7,21 |
| gefunden, %: | C | 53,80 | H | 3,94 | N | 12,87 | Cl | 7,71 | S | 7,43 |

Beispiel 22

Ausgehend von 6,2 g 1-[(4′-Chlor-3′-N-dimethylamino-methyliden-sulfamoyl-benzoyl)-amino]-benzimidazol und 40 ml 2n Natronlauge arbeitet man gemäß Beispiel 16 und erhält 6 g (87,6 %) 1-[(4′-Chlor-3′-sulfamoyl-benzoyl)-amino]-benzimidazol, dessen Zersetzungspunkt nach Umkristallisieren aus 96 %igem Alkohol bei 162 °C liegt.

| Analyse für $C_{14}H_{11}ClN_4O_3S$ | | | | | | | | | | |
|---|---|---|---|---|---|---|---|---|---|---|
| berechnet, %: | C | 47,93 | H | 3,16 | N | 15,97 | Cl | 10,11 | S | 9,14 |
| gefunden, %: | C | 47,50 | H | 3,08 | N | 15,70 | Cl | 9,95 | S | 8,96 |

Herstellung des Ausgangsstoffes

Man geht von 4 g 1-Amino-benzimidazol (Herstellung: J. Org. Chem. 28, 736 /1963/), 7 ml Pyridin und 9,3 g 4-Chlor-3-(N-dimethylamino-methyliden -sulfamoyl)-benzoylchlorid aus und arbeitet auf die im Beispiel 14 beschriebene Weise. Man erhält 11,1 g (91 %) 1-[(4′-Chlor-3′-N-dimethylamino-methyliden-sulfamoyl-benzoyl)-amino]-benzimidazol, das nach Umkristallisieren aus Nitromethan bei 258-261 °C schmilzt.

| Analyse für $C_{17}H_{16}ClN_5O_3S$ | | | | | | | | | | |
|---|---|---|---|---|---|---|---|---|---|---|
| berechnet, %: | C | 50,30 | H | 3,97 | N | 17,26 | Cl | 8,74 | S | 7,90 |
| gefunden, %: | C | 50,13 | H | 4,01 | N | 17,27 | Cl | 8,90 | S | 8,03 |

Beispiel 23

Ausgehend von 5,1 g 1-[(4′-Chlor-3′-N-dimethylamino-methyliden-sulfamoyl-benzoyl)-amino]-2-methyl-benzimidazol und 30 ml 2n Natronlauge arbeitet man auf die im Beispiel 16 beschriebene Weise und erhält 3,7 g (87 %) 1-[(4′-Chlor-3′-sulfamoyl-benzoyl)-amino]-2-methyl-benzimidazol. Das Produkt wird aus einem 1:1-Gemisch von Dimethylformamid und Wasser umkristallisiert und weist dann einen Zersetzungspunkt von 300 °C auf.

| Analyse für $C_{15}H_{13}ClN_4O_3S$ | | | | | | | | | |
|---|---|---|---|---|---|---|---|---|---|
| berechnet, %: | C | 49,38 | H | 3,59 | N | 15,36 | Cl | 9,72 | S | 8,79 |
| gefunden, %: | C | 49,18 | H | 3,56 | N | 15,20 | Cl | 9,72 | S | 8,98 |

Herstellung des Ausgangsstoffes

Man geht von 4,1 g 1-Amino-2-methyl-benzimidazol (Herstellung: J. Org. Chem. 28, 736 /1963/), 15 ml Pyridin und 9,3 g 4-Chlor-3-[(N-dimethylamino-methyliden)-sulfamoyl]-benzoylchlorid aus und arbeitet auf die im Beispiel 14 beschreibene Weise. Man erhält 10,2 g (77,6 %) 1-[(4′-Chlor-3′-N-dimethylamino-methyliden-sulfamoyl-benzoyl)-amino]-2-methyl-benzimidazolhydrat, dessen Zersetzungspunkt nach Umkristallisieren aus 96 %igem Alkohol bei 166 °C liegt. Nach Umkristallisieren aus Acetonitril schmilzt das Produkt bei 232-235 °C.

| Analyse für $C_{18}H_{18}ClN_5O_3S$ | | | | | | | | | | |
|---|---|---|---|---|---|---|---|---|---|---|
| berechnet, %: | C | 49,36 | H | 4,60 | N | 15,99 | Cl | 8,10 | S | 7,01 |
| gefunden, %: | C | 49,25 | H | 4,61 | N | 15,72 | Cl | 8,10 | S | 7,10 |

Beispiel 24

Ausgehend von 2,4 g 1-[(4′-Chlor-3′-N-dimethylamino-methyliden-sulfamoyl-benzoyl)-amino]-5-sulfamoyl-benzimidazol-2-thion und 12 ml 2n Natronlauge arbeitet man gemäß Beispiel 16 und erhält 1,64 g (76 %) 1-[(4′-Chlor-3′-sulfamoyl-benzoyl)-amino]-5-sulfamoyl-benzimidazol-2-thion. Das Produkt wird in Alkohol gekocht und weist danach einen Zersetzungspunkt von 326 °C auf.

| Analyse für $C_{14}H_{12}ClN_5O_5S_2$ | | | | | | | | | | |
|---|---|---|---|---|---|---|---|---|---|---|
| berechnet, %: | C | 36,40 | H | 2,62 | N | 15,16 | Cl | 7,68 | S | 20,82 |
| gefunden, %: | C | 36,31 | H | 2,45 | N | 15,66 | Cl | 7,36 | S | 20,80 |

Herstellung der Ausgangsstoffe

A) Zu der Suspension von 47,33 g 3-Nitro-4-chlor-benzolsulfonsäureamid (Herstellung: J. Am. Chem. Soc. 73, 2558 /1951/) in 200 ml abs. Alkohol werden 20 ml Hydrazinhydrat gegeben. Das Reaktionsgemisch wird unter Rühren 30 Minuten lang gekocht. Nach dem Abkühlen werden die Kristalle abgesaugt und gründlich mit Wasser gewaschen. Man erhält 43,38 g (93,4 %) 2-Nitro-4-sulfamoyl-phenylhydrazin, das sich bei 217-218 °C zersetzt.

| Analyse für $C_6H_8N_4O_4S$ | | | | | | | | |
|---|---|---|---|---|---|---|---|---|
| berechnet, %: | C | 31,03 | H | 3,47 | N | 24,13 | S | 13,81 |
| gefunden, %: | C | 31,50 | H | 3,48 | N | 24,37 | S | 13,57 |

B) Man geht von 65,7 g dar wie beschrieben hergestellten Nitroverbindung, 700 ml Dioxan, der Lösung von 15 g wasserfreiem Natriumcarbonat in 280 ml Wasser und der mit 560 ml Dioxan bereiteten Lösung von 87,5 g 4-Chlor-3-[(N-dimethylamino-methyliden)-sulfamoyl]-benzoylchlorid aus und arbeitet auf die im Beispiel 1/B beschreibene Weise. Das Reaktionsgemisch wird im Vakuum eingedampft, der Rückstand mit Wasser behandelt, die Festsubstanz abgesaugt und im nutschfeuchten Zustand in 600 ml Methanol gekocht. Nach Filtrieren und Trocknen erhält man 121,5 g (85 %) 2-Nitro-4-sulfamoyl-N-(4′-chlor-3′-dimethylamino-methyliden-sulfamoyl-benzoyl)-phenylhydrazin, das sich bei 248 °C zersetzt und nach Umkristallisieren aus Eissessig bei 252 °C unter Zersetzung schmilzt.

| Analyse für $C_{16}H_{17}ClN_6O_7S_2$ | | | | | | | | | | |
|---|---|---|---|---|---|---|---|---|---|---|
| berechnet, %: | C | 38,06 | H | 3,39 | N | 16,64 | Cl | 7,02 | S | 12,70 |
| gefunden, %: | C | 38,10 | H | 3,42 | N | 16,59 | Cl | 7,00 | S | 12,68 |

C) Man geht von 5 g der wie beschrieben hergestellten Nitroverbindung, 60 ml abs. Alkohol, 0,5 g 10 %iger Palladiumaktivkohle und 30 ml 30 %iger wässriger Natriumhypophosphitlösung aus und arbeitet auf die im Beispiel 15/B beschriebene Weise. Man erhält 4,2 g (88 %) 2-Amino-4-sulfamoyl-N-(4′-chlor-3′-dimethylamino-methyliden-sulfamoyl-benzoyl)-phenylhydrazin, das sich bei 152 °C zersetzt.

D) Ausgehend von 4,75 g der auf die beschriebene Weise hergestellten Phenylhydrazinverbindung, 20 ml Pyridin und 1,6 g Kaliumäthylxanthan arbeitet man auf die im Beispiel 3 beschriebene Weise. Man erhält 3,2 g (62 %) 1-[(4′-Chlor-3′-N-dimethylamino-methyliden-sulfamoyl-benzoyl)-amino]-5-sulfamoyl-benzimidazol-2-thion. Zersetzungspunkt: 124 °C.

Beispiel 25

Man geht von 6,3 g 2-Amino-4-methylsulfonyl-N-(4′-chlor-3′-sulfamoyl-benzol)-phenylhydrazin, 35 ml Pyridin und 2,4 g Kaliumäthylxanthat aus, arbeitet auf die im Beispiel 3 beschriebene Weise und erhält 5 g (73 %) 1-[(4′-Chlor-3′-sulfamoyl-benzoyl)-amino]-5-methylsulfonyl-benzimidazol-2-thion, das nach Umkristallisieren aus 50 %igem wässrigem Alkohol bei 298-300 °C schmilzt.

| Analyse für $C_{15}H_{13}ClN_4O_5S_3$ | | | | | | | | | | |
|---|---|---|---|---|---|---|---|---|---|---|
| berechnet, %: | C | 39,08 | H | 2,82 | N | 12,16 | Cl | 7,69 | S | 20,87 |
| gefunden, %: | C | 39,90 | H | 2,70 | N | 12,12 | Cl | 7,98 | S | 20,60 |

Herstellung der Ausgangsstoffe

A) Man geht von 12,15 g 2-Nitro-4-methylsulfonyl-chlorbenzol (Herstellung: J. Am. Chem. Soc. 75, 642 /1953/), 50 ml abs. Alkohol und 5,3 ml Hydrazinhydrat aus, arbeitet auf die im Beispiel 24/A beschriebene weise und erhält 11 g (92 %) 2-Nitro-4-methylsulfonyl-phenylhydrazin, das bei 188-190 °C schmilzt.

| Analyse für $C_7H_9N_3O_4S$ | | | | | | | | |
|---|---|---|---|---|---|---|---|---|
| berechnet, %: | C | 36,36 | H | 3,92 | N | 18,17 | S | 13,87 |
| gefunden, %: | C | 36,23 | H | 3,36 | N | 18,77 | S | 13,97 |

B) Man geht von 11 g der auf die beschriebene Weise hergestellten Phenylhydrazinverbindung, 120 ml Dioxan, der Lösung von 2,5 g wasserfreiem Natriumcarbonat in 47 ml Wasser und der mit 95 ml Dioxan bereiteten Lösung von 12 g 4-Chlor-3-sulfamoyl-benzoylchlorid aus, arbeitet auf die im Beispiel 1/B beschriebene Weise und erhält 19,5 g (94 %) 2-Nitro-4-methylsulfonyl-N-(4′-chlor-3′-sulfamoyl-benzoyl)-phenylhydrazin, dessen Zersetzungspunkt nach Umkristallisieren aus Essigsäure bei 279 °C liegt.

| Analyse für $C_{13}H_{13}ClN_4O_7S_2$ | | | | | | | | | | |
|---|---|---|---|---|---|---|---|---|---|---|
| berechnet, %: | C | 35,73 | H | 3,00 | N | 12,82 | Cl | 8,11 | S | 14,67 |
| gefunden, %: | C | 35,75 | H | 2,90 | N | 12,49 | Cl | 7,91 | S | 14,24 |

C) Man geht von 14,8 g der auf die beschriebene Weise hergestellten Nitroverbindung, 250 ml Methylcellosolv und 2 g Raney-Nickel aus,arbeitet auf die im Beispiel1/C beschriebene Wesie und erhält 14,1 g (99 %) 2-Amino-4-methylsulfonyl-N-(4′-chlor-3′-sulfamoyl-benzoyl)-phenylhydrazin, dessen Zersetzunspunkt nach Umkristallisieren aus Alkohol bei 200 °C liegt.

| Analyse für $C_{14}H_{15}ClN_4O_5S_2$ | | | | | | | | | | |
|---|---|---|---|---|---|---|---|---|---|---|
| berechnet, %: | C | 40,14 | H | 3,61 | N | 13,38 | Cl | 8,46 | S | 15,31 |
| gefunden, %: | C | 40,51 | H | 3,77 | N | 13,65 | Cl | 8,83 | S | 15,43 |

Beispiel 26

Man geht von 20,27 g 1-[(4′-Chlor-3′-N-dimethylamino-methyliden-sulfamoyl-benzoyl)-amino]-5-trifluormethyl-benzimidazol und 80 ml 2n Natronlauge aus und arbeitet auf die im Beispiel 16 beschriebene Weise. Das Rohprodukt wird aus einem 1:1-Gemisch von Benzol und Aceton umkristallisiert. Man erhält 14 g (77,7 %) 1-[(4′-Chlor-3′-sulfamoyl-benzoyl)-amino]-5-trifluoromethyl-benzimidazol, das bei 164 °C schmilzt.

| Analyse für $C_{15}H_{10}ClF_3N_4O_3S$ | | | | | | | | |
|---|---|---|---|---|---|---|---|---|
| berechnet, %: | C | 43,01 | H | 2,41 | N | 13,38 | Cl | 8,47 |
| gefunden, %: | C | 43,82 | H | 2,78 | N | 13,56 | Cl | 8,11. |

Herstellung der Ausgangsstoffe

A) 64 g 2-Nitro-4-trifluormethyl-phenylhydrazin (Herstellung: J. Org. Chem. 42, 542 /1977/) werden zusammen mit 200 ml 85 %iger Ameisensäure auf dem Wasserbad 90 Minuten lang erwärmt. Nach dem Abkühlen wird die gelbe Lösung in 250 ml Wasser gegossen. Die sich abscheidenden Kristalle werden abgesaugt, mit Wasser gewaschen und bei 80 °C getrocknet. Man erhält in Form gelber Kristalle 72 g (98 %) 2-Nitro-4-trifluormethyl-N-formyl-phenylhydrazin, das bei 152-153 °C schmilzt.

| Analyse für $C_8H_6F_3N_3O_3$ | | | | | | |
|---|---|---|---|---|---|---|
| berechnet, %: | C | 38,56 | H | 2,43 | N | 16,86 |
| gefunden, %: | C | 38,38 | H | 2,48 | N | 16,98 |

B) 65,45 g der auf die beschriebene Weise hergestellten Nitroverbindung werden in 780 ml abs. Alkohol gelöst, die Lösung wird mit 6 g 10 %iger Palladiumaktivkohle versetzt und bei Raumtemperatur unter Normaldruck in einem Schüttelapparat bis zur Beendigung der Wasserstoffaufnahme hydriert. Der Katalysator wird abfiltriert und das Filtrat eingedampft. Man erhält 55,8 g 2-Amino-4-trifluormethyl-N-formylphenylhydrazin, das bei 121-122 °C schmilzt.

| Analyse für $C_8H_8F_3N_3O$ | | | | | | |
|---|---|---|---|---|---|---|
| berechnet, %: | C | 43,84 | H | 3,68 | N | 19,18 |
| gefunden, %: | C | 43,25 | H | 3,94 | N | 19,19 |

C) 44 g des auf die beschriebene Weise hergestellten Phenylhydrazinderivates werden in 200 ml 85 %iger Ameisensäure 5 Stunden lang gekocht. Die entstandene rote Lösung wird im Vakuum eingedampft. Der Rückstand wird mit 200 ml Wasser verrieben. Die sich ausscheidenden Kristalle werden abgesaugt, mit Wasser gewaschen und bei 80 °C getrocknet. Man erhält 40,8 g (88,7 %) 1-Formamido-5-trifluormethyl-benzimidazol, das nach Umkristallisieren aus einem im Verhältnis von 1:2 bereiteten Gemisch aus Aceton und Wasser bei 209-210 °C schmilzt.

| Analyse für $C_9H_6F_3N_3O$ | | | | | | |
|---|---|---|---|---|---|---|
| berechnet, %: | C | 47,17 | H | 2.64 | N | 18,34 |
| gefunden, %: | C | 47,69 | H | 2.59 | N | 18,51 |

D) 15,11 g des auf die beschriebene Weise hergestellten Benzimidazolderivates werden in 68 ml 2n Salzsäure 5 Stunden lang gekocht. Die Lösung wird mit 2n Natronlauge alkalisch gemacht. Die ausgeschiedenen Kristalle werden abgesaugt, mit Wasser gewaschen und bei 80 °C getrocknet. Man erhält 11,7 g (88 %) 1-Amino-5-trifluormethyl-benzimidazol, das bei 140-142 °C schmilzt.

| Analyse für $C_8H_6F_3N_3$ | | | | | | |
|---|---|---|---|---|---|---|
| berechnet, %: | C | 47,76 | H | 3,00 | N | 20,89 |
| gefunden, %: | C | 47,82 | H | 2.94 | N | 20,64 |

E) Man geht von 8,69 g des auf die beschriebene Weise hergestellten Benzimidazolderivates, 10 ml Pyridin und 13,35 g 4-Chlor-3-(N-dimethylamino-methyliden-sulfamoyl)-benzoylchlorid aus, arbeitet auf die im Beispiel 14 beschriebene Weise und erhält 20,27 g (100 %) 1-[(4′-Chlor-3′-N-dimethylamino-methyliden-sulfamoyl-benzoyl)-amino]-5-trifluormethyl-benzimidazol, das nach Umkristallisieren aus Methanol bei 266-270 °C schmilzt.

| Analyse für $C_{18}H_{15}ClF_3N_5O_3S$ | | | | | | | | |
|---|---|---|---|---|---|---|---|---|
| berechnet, %: | C | 45,62 | H | 3,19 | N | 14,78 | S | 6,77 |
| gefunden, %: | C | 45,70 | H | 3,94 | N | 14,35 | S | 6,98 |

## Beispiel 27

Man geht von 7,4 g 1-[(4′-Chlor-3′-N-dimethylamino-methyliden-sulfamoyl-benzoyl)-amino]-2-methyl-5-trifluormethyl-benzimidazol und 20 ml 2n Natronlauge aus, arbeitet auf die im Beispiel 16 beschriebene Weise und erhält 5,3 g (80,7 %) 1-[(4′-Chlor-3′-sulfamoyl-benzoyl)-amino]-2-methyl-5-trifluormethyl-benzimidazol, das nach Umkristallisieren aus einem 1:1-Gemisch von Benzol und Aceton bei 193-195 °C schmilzt.

| Analyse für $C_{16}H_{12}ClF_3N_4O_3S$ | | | | | | | | |
|---|---|---|---|---|---|---|---|---|
| berechnet, %: | C | 44,40 | H | 2,80 | N | 12,95 | Cl | 8,19 |
| gefunden, %: | C | 44,92 | H | 2,64 | N | 13,13 | Cl | 8,36 |

## Herstellung der Ausgangsstoffe

A) 23,3 g 2-Amino-4-trifluormethyl-N-acetyl-phenylhydrazin (Herstellung: J. Org. Chem. 42, 542 /1977/) werden in einem Gemisch aus 120 ml Eisessig und 20 ml Acetanhydrid 6 Stunden lang gekocht. Die orangerote Lösung wird im Vakuum eingedampft und der Rückstand mit Wasser verrieben. Die sich abscheidenden Kristalle werden abgesaugt, mit Wasser gewaschen und bei 80 °C getrocknet. Man erhält 23,2 g (90 %) 1-Acetamino-2-methyl-5-trifluormethyl-benzimidazol, das nach Umkristallisieren aus Aceton bei 214-216 °C schmilzt.

| Analyse für $C_{11}H_{10}F_3N_3O$ | | | | | | |
|---|---|---|---|---|---|---|
| berechnet, %: | C | 51,36 | H | 3,92 | N | 16,34 |
| gefunden, %: | C | 51,43 | H | 3,98 | N | 16,38. |

B) 71,14 g des auf die beschriebene Weise hergestellten Benzimidazolderivates werden in 300 ml 2n Salzsäure 5 Stunden lang gekocht. Die gelbe Lösung wird heiß mit Aktivkohle geklärt, filtriert und das Filtrat mit 10n Natronlauge auf pH 6 gestellt. Die ausfallenden schneeweißen Kristalle werden abgesaugt, mit Wasser gründlich gewaschen und bei 80 °C getrocknet. Man erhält 53,2 g (89,5 %) 1-Amino-2-methyl-5-trifluormethyl-benzimidazol, das bei 190-192 °C schmilzt.

| Analyse für $C_9H_8F_3N_3$ | | | | | | |
|---|---|---|---|---|---|---|
| berechnet, %: | C | 50,23 | H | 3,75 | N | 19,23 |
| gefunden, %: | C | 50,35 | H | 3,97 | N | 19,63 |

C) Man geht von 18,02 g des auf die beschriebene Weise hergestellten Benzimidazolderivates, 25 ml Pyridin und 25,9 g 4-Chlor-3-(N-dimethylamino-methyliden-sulfamoyl)-benzoylchlorid aus, arbeitet auf die im Beispiel 14 beschriebene Weise und erhält 34,4 g (84 %) 1-[(4′-Chlor-3′-(N-dimethylamino-methyliden-sulfamoyl-benzoyl)-amino]-2-methyl-5-trifluormethyl-benzimidazol, das bei 300 °C schmilzt.

Beispiel 28

Man geht von 11,33 g 1-[(4′-Chlor-3′-N-dimethylamino-methyliden-sulfamoyl-benzoyl)-amino]-5-trifluormethyl-benzimidazol-2-thion und 50 ml 2n Natronlauge aus, arbeitet auf die im Beispiel 16 beschriebene Weise und erhält 7,92 g (77,7 %) 1-[(4′-Chlor-3′-sulfamoyl-benzoyl)-amino]-5-trifluormethyl-benzimidazol-2-thion, dessen Zersetzungspunkt nach Umkristallisieren aus 50 %igem wässrigem Alkohol bei 282-283 °C liegt.

| Analyse für $C_{15}H_{10}F_3ClN_4O_3S_2$ | | | | | | | | |
|---|---|---|---|---|---|---|---|---|
| berechnet, %: | C | 39,96 | H | 2,24 | N | 12,43 | Cl | 7,86 |
| gefunden, %: | C | 39,06 | H | 2,12 | N | 12,20 | Cl | 7,11 |

Herstellung der Ausgangsstoffe

A) Man geht von 8,84 g 2-Nitro-4-trifluormethyl-phenylhydrazin, 50 ml Pyridin und 12,36 g 4-Chlor-3-(N-dimethylamino-methyliden-sulfamoyl)-benzoylchlorid aus, arbeitet auf die im Beispiel 14 beschriebene Weise und erhält 17 g (86 %) 2-Nitro-4-trifluormethyl-N-(4′-chlor-3′-dimethylamino-methyliden-sulfamoyl-benzoyl)-phenylhydrazin, dessen Zersetzungspunkt nach Umkristallisieren aus Essigsäure bei 261-262 °C liegt.

| Analyse für $C_{17}H_{15}ClF_3N_5O_5S$ | | | | | | |
|---|---|---|---|---|---|---|
| berechnet, %: | C | 41,34 | H | 3.06 | N | 14,19 |
| gefunden, %: | C | 41,23 | H | 3,36 | N | 14,09 |

B) Man geht von 17 g der auf die beschriebene Weise hergestellten Nitroverbindung, 1,5 g 10 %iger Palladiumaktivkohle, 100 ml 96 %igem Alkohol und 60 ml 30 %iger wässriger Natriumhypophosphitlösung aus, arbeitet auf die im Beispiel 2/B beschriebene Weise und erhält 11,45 g (72 %) 2-Amino-4-trifluormethyl-N-[4′-chlor-3′-(N-dimethylaminomethyliden-sulfamoyl)-benzoyl]-phenylhydrazin, dessen Zersetzungspunkt nach Umkristallisieren aus 96 %igem Alkohol bei 199-200 °C liegt.

| Analyse für $C_{17}H_{17}ClF_3N_5O_3S$ | | | | | | | | |
|---|---|---|---|---|---|---|---|---|
| berechnet, %: | C | 44,01 | H | 3,69 | N | 15,10 | Cl | 7,64 |
| gefunden, %: | C | 43,98 | H | 3,62 | N | 14,98 | Cl | 7,27 |

C) Man geht von 10,4 g des auf die beschriebene Weise hergestellten Phenylhydrazinderivates, 45 ml Pyridin und 3,6 g Kaliumäthylxanthat aus, arbeitet auf die im Beispiel 3 beschriebene Weise und erhält 11,33 g (100 %) 1-[(4′-Chlor-3′-N-dimethylamino-methyliden-sulfamoyl-benzoyl)-amino]-5-trifluormethyl-benzimidazol-2-thion, das ohne weitere Reinigung weiterverwendet wird.

Beispiel 29

Man geht von 5,55 g 1-Amino-2-phenyl-5-trifluormethyl-benzimidazol, 15 ml Pyridin und 6,2 g 4-Chlor-3-(N-dimethylamino-methyliden-sulfamoyl)-benzoylchlorid aus, arbeitet auf die im Beispiel 14 beschriebene Weise und erhält 9,7 g 1-[(4′-Chlor-3′-N-dimethylamino-methyliden-sulfamoyl-benzoyl)-amino]-2-phenyl-5-trifluormethyl-benzimidazol, das auf die im Beispiel 16 beschriebene Weise mit 50 ml 2n Natronlauge hydrolysiert wird. Man erhält 7 g (98 %) 1-[(4′-Chlor-3′-sulfamoyl-benzoyl)-amino[-2-phenyl-5-trifluormethyl-benzimidazol, das nach Umkristallisieren aus Butanol 1 Mol Butanol enthält und sich bei 282-284 °C zersetzt.

| Analyse für $C_{21}H_{14}ClF_3N_4O_3S.C_4H_9OH$ | | | | | | |
|---|---|---|---|---|---|---|
| berechnet, %: | C | 52,76 | H | 4,25 | N | 9,84 |
| gefunden, %: | C | 52,35 | H | 4,37 | N | 9,66 |

Herstellung der Ausgangsstoffe

A) Man geht von 66,5 g 2-Amino-4-trifluormethyl-N-acetyl-phenylhydrazin, 70 ml Pyridin und 32,9 ml Benzoylchlorid aus, arbeitet auf die im Beispiel 14 beschriebene Weise und erhält 90 g (93,6 %) 2-Benzoylamino-4-trifluormethyl-N-acetyl-phenylhydrazin, das nach Umkristallisieren aus 96 %igem Alkohol bei 212-214 °C schmilzt.

| Analyse für $C_{16}H_{14}F_3N_3O_2$ | | | | | | |
|---|---|---|---|---|---|---|
| berechnet, %: | C | 56,97 | H | 4,18 | N | 12,46 |
| gefunden, %: | C | 57,15 | H | 4,08 | N | 12,48 |

B) 52,2 g des auf die beschriebene Weise hergestellten Phenylhydrazinderivates werden in 270 ml Eisessig 6 Stunden lang gekocht. Die Lösung wird im Vakuum eingedampft und der Rückstand mit Wasser verrieben. Die Kristalle werden abgesaugt, mit Wasser gewaschen und bei 80 °C getrocknet. Man erhält 46,3 g (88,5 %) 1-Acetamino-2-phenyl-5-trifluormethyl-benzimidazol, das nach Umkristallisieren aus 50 %igem wässrigem Alkohol bei 150-154 °C schmilzt.

| Analyse für $C_{16}H_{12}F_3N_3O$ | | | | | | |
|---|---|---|---|---|---|---|
| berechnet, %: | C | 60,19 | H | 3,79 | N | 13,16 |
| gefunden, %: | C | 59,98 | H | 3,74 | N | 13,04 |

C) 45 g des auf die beschriebene Weise hergestellten Benzimidazolderivates werden in einem Gemisch aus 100 ml 96 %igem Alkohol und 300 ml 2n Salzsäure 8 Stunden lang gekocht. Die Lösung wird heiß mit Aktivkohle geklärt und dann filtriert. Das Filtrat wird mit Natronlauge auf pH 10 alkalisch gemacht. Der Niederschlag wird abgesaugt, mit Wasser gewaschen und bei 80 °C getrocknet. Man erhält 34,1 g (87 %) 1-Amino-2-phenyl-5-trifluormethyl-benzimidazol, das nach Umkristallisieren aus 96 %igem Alkohol bei 200-203 °C schmilzt.

| Analyse für $C_{14}H_{10}F_3N_3$ | | | | | | |
|---|---|---|---|---|---|---|
| berechnet, %: | C | 60,65 | H | 3,64 | N | 15,16 |
| gefunden, %: | C | 60,25 | H | 3,24 | N | 15,58 |

Beispiel 30

Aus 9,88 g 1-Amino-2-methylthio-5-trifluormethyl-benzimidazol, 20 ml Pyridin und 12,2 g 4-Chlor-3-(N-dimethylamino-methyliden-sulfamoyl)-benzoylchlorid erhält man auf die im Beispiel 14 beschriebene Weise 19,4 g (98 %) 1-[(4′-Chlor-3′-N-dimethylamino-methyliden-sulfamoyl-benzoyl)-amino]-2-methylthio-benzimidazol. Dieses wird auf die im Beispiel 16 beschriebene Weise mit 100 ml 2n Natronlauge hydrolysiert. Man erhält 15,45 g (83 %) 1-[(4′-Chlor-3′-sulfamoyl-benzoyl)-amino]-2-methylthio-5-trifluormethyl-benzimidazol, dessen Zersetzungspunkt nach Umkristallisieren aus Äthylacetat bei 126-128 °C liegt.

| Analyse für $C_{16}H_{12}ClF_3N_4O_3S_2$ | | | | | | |
|---|---|---|---|---|---|---|
| berechnet, %: | C | 41,34 | H | 2,73 | N | 10,34 |
| gefunden, %: | C | 41,38 | H | 2,78 | N | 10,61 |

Herstellung der Ausgangsstoffe

A) Zu der Lösung von 19,07 g Kaliumhydroxyd in 300 ml abs. Alkohol werden 69,96 g 2-Amino-4-trifluormethyl-N-acetyl-phenylhydrazin gegeben. Das Reaktionsgemisch wird unter Rühren tropfenweise mit 20,5 ml Schwefelkohlenstoff versetzt, die erhaltene rote Lösung 5 Stunden lang gekocht, heiß mit Aktivkohle geklärt und dann filtriert. Zu dem Filtrat werden zuerst 370 ml Wasser, dann 70 ml eines im Verhältnis 1:2 bereiteten Gemisches aus Essigsäure und Wasser gegeben. Die sich abscheidenden Kristalle werden abgesaugt, mit Wasser gewaschen und bei 80 °C getrocknet. Man erhält 55,5 g (67 %) 1-Acetamino-5-trifluormethyl-benzimidazol-2-thion, das nach Umkristallisieren aus 50 %igem wässrigem Alkohol bei 294-295 °C schmilzt.

| Analyse für $C_{10}H_8F_3N_3OS$ | | | | | | |
|---|---|---|---|---|---|---|
| berechnet, %: | C | 43,63 | H | 2,93 | N | 15,27 |
| gefunden, %: | C | 43,52 | H | 2,95 | N | 15,40. |

B) Zu der Suspension von 27,5 g des auf die beschriebene Weise hergestellten Benzimidazolderivates in 200 ml n NaOH werden 10 ml Dimethylsulfat gegeben. Die anfangs schlecht rührbare Suspension wandelt sich innerhalb einiger Minuten um. Das Gemisch wird auf dem kochenden Wasserbad 30 Minuten lang gerührt. Nach dem Abkühlen wird der Niederschlag abgesaugt, mit Wasser gewaschen und bei 80 °C getrocknet. Man erhält 27,2 g (94 %) 1-Acetamino-2-methylthio-5-trifluormethyl-benzimidazol, das nach Umkristallisieren aus Benzol bei 175-178 °C schmilzt.

| Analyse für $C_{11}H_{10}F_3N_3OS$ | | | | | | |
|---|---|---|---|---|---|---|
| berechnet, %: | C | 45,67 | H | 3,48 | N | 14,52 |
| gefunden, %: | C | 45,36 | H | 3,76 | N | 14,27 |

C) 22,2 g des auf die beschriebene Weise hergestellten Benzimidazolderivates werden in 80 ml 2n Salzsäure 6,5 Stunden lang gekocht. Das Reaktionsgemisch wird im Vakuum zur Trockne eingedampft. Der Rückstand wird in 80 ml Natriumhydrogencarbonatlösung eingestreut und das Gemisch bis zur Beendigung der Gasentwicklung auf dem Wasserbad erwärmt. Nach dem Abkühlen werden die ausgefallenen Kristalle abgesaugt, mit Wasser gewaschen und bei 80 °C getrocknet. Man erhält 16,85 g (88,5 %) 1-Amino-2-methylthio-5-trifluormethyl-benzimidazol, das nach Umkristallisieren aus 50 %igem wässrigem Alkohol bei 170-173 °C schmilzt.

| Analyse für $C_9H_8F_3N_3S$ | | | | | | |
|---|---|---|---|---|---|---|
| berechnet, %: | C | 43,72 | H | 3,26 | N | 16,99 |
| gefunden, %: | C | 43,65 | H | 3,17 | N | 16,64. |

Beispiel 31

Man geht von 11,25 g 1-[(4'-Chlor-3'-sulfamoyl-benzoyl)-amino]-5-trifluormethyl-benzimidazol-2-thion aus, arbeitet auf die im Beispiel 8 beschriebene Weise und erhält 10,8 g (93 %) 1-[(4'-Chlor-3'-sulfamoyl-benzoyl)-amino]-2-methyl-thio-5-trifluormethyl-benzimidazol, das in jeder Hinsicht mit dem Produkt gemäß Beispiel 29 identisch ist.

Beispiel 32

Zu der Suspension von 8,2 g 1-([(4'-Chlor-3'-sulfamoyl-benzoyl)-amino]-2-methyl-5-carboxy-benzimidazol in 50 ml Methanol werden unter Rühren tropfenweise 1,46 ml Thionylchlorid gegeben. Das Reaktionsgemisch wird 5 Stunden lang gekocht, die erhaltene klare Lösung im Vakuum zur Trockne eingedampft und der feste Rückstand mit n Natriumhydrogencarbonatlösung verrieben. Die Lösung wird von der festen Phase abgegossen und der Rückstand aus einem 1:1-Gemisch von Dioxan und Wasser umkristallisiert. Man erhält 6,35 g (75 %) 1-[(4'-Chlor-3'-sulfamoyl-benzoyl)-amino]-2-methyl-5-methoxycarbonyl-benzimidazol, das sich bei 251-252 °C zersetzt.

| Analyse für $C_{17}H_{15}ClN_4O_5S$ | | | | | | | | | | | | |
|---|---|---|---|---|---|---|---|---|---|---|---|---|
| berechnet, %: | C | 48,28 | H | 3,58 | N | 13,25 | Cl | 8,39 | S | 7,58 |
| gefunden, %: | C | 47,98 | H | 3,58 | N | 12,95 | Cl | 8,12 | S | 7,52 |

Die Untersuchung der saldiuretischen Verbindungen an Ratten

Für die screen-Untersuchungen wurden männliche Ratten des Stammes LATI CFY verwendet, die ein durchschnittliches Gewicht von 240 g aufwiesen. Die Tiere wurden mit Standardrattenfutter gefüttert. 16 Stunden vor dem Versuch wurde die Fütterung eingestellt, die Flüssigkeitsaufnahme war jedoch nicht begrenzt. Die diuretische Wirkung wurde mit der von Kagawa und Kahn modifizierten Methode von Lipschitz (Arch. Int. Pharmacodyn. 137, 241-249 /1962/) nachgewiesen. Die Ergebnisse sind in der Tabelle 1 zusammengestellt.

Saldiuretische Untersuchung an Hunden

Die gemäß Beispiel 1 hergestellte Substanz wurde auch an Hunden getestet. Für die Untersuchung wurden perineotomisierte weibliche Hunde verwendet. Die Tiere wurden 2 Wochen lang ohne Behandlung beobachtet und dann operiert. Beginnend in der vierten Woche nach der Operation wurde den Tieren zum Zweck der Gewöhnung wöchentlich im Durchschnitt zweimal die Blase mit einem Katheter entleert beziehungsweise sie bekamen mit einer Magensonde Salzlösung. Während des Versuches erhielten die Tiere Standardfutter, die Wasseraufnahme war nicht beschränkt. An den Versuchstagen bekamen die Tiere kein Futter, und Wasser in einer Menge von 20 ml/kg. Mit dem Futter wurden 7,7 mMol/kg Natrium und 6,02 mMol Kalium eingebracht.

Am Versuchstag wurde die Blase der Tiere mit dem Katheter entleert und aus der Beinvene eine Blutprobe genommen. Dann wurden mit der Magensonde 10 ml/kg Diuretikumlösung beziehungsweise im Falle der Kontrolltiere Lösungsmittel eingeführt. Die Tiere wurden einzeln in Stoffwechselkäfigen untergebracht. Nach 7 beziehungsweise 24 Stunden wurde die Blase katheterisiert, der Urin wurde mittels Durchwaschen des Stoffwechseilkäfigs gesammelt. Nach 24 Stunden wurde, anschließend an die Katheterisierung, erneut eines Blutprobe genommen.

Gemessen wurden: entleertes Urinvolumen, Gehalt des Urins an $Na^+$ und $K^+$, ferner Glucose und Cholesterin im Serum.

Der an Hunden gemessene Zusammenhang zwischen der Diuretikumdosis und der Wasser-, Na- und K-entleerenden Wirkung ist in den Fig. 1 und 2 gezeigt. Fig. 1 zeigt die in der Periode der 0-7. Stunde nach der Verabreichung gemessene Wirkung der Verbindung gemäß Beispiel 1 und die des Furosemids (weil die des Hypothiazids in diesem Zeitabschnitt noch niedrig ist), während in Fig. 2 die im Meßzeitraum von 0-24 Stunden gewonnenen Werte für Urinvolumen, Na- und K-Gehalt gezeigt sind (für die Verbindung gemäß Beispiel 1 und Hypothiazid, weil in der 7-24. Stunde die Wirkung des Furosemids bereits schwach ist).

Antihypertensive Wirkung, untersucht an Ratten

Die blutdrucksenkende Wirkung wurde an spontan hypertensiven männlichen Ratten des Stammes Okamoto untersucht. Die Tiere wurden vor Beginn des Versuches wenigstens eine Woche lang einzeln in Käfigen gehalten, sie erhielten Wasser und halbsynthetisches Rattenfutter ad libitum. Jeden Tag wurde zu dem gleichen Zeitpunkt an jedem Tier der Blutdruck gemessen, beziehungsweise es wurde eine Magensondierung vorgenommen, wobei die Tiere immobilisiert wurden. Die Tiere mit einem Blutdruck von weniger als 0,226 bar (170 Hgmm) wurden selektiert. Auf Grund der täglichen Blutdruckmessungen wurden die Tiere in Versuchsgruppen eingeteilt, wobei in jeder Gruppe der Durchschnitt der Blutdruckwerte und die Streuung annähernd gleich waren.

Zu Beginn der Untersuchung wurde den Tieren mit der Magensonde die Lösung der zu untersuchenden Verbindung in einem Volumen von 0,2 ml/100 g beziehungsweise die Furosemidlösung in einem Volumen von 1 ml/100 g appliziert. An jedem Versuch nahm auch eine mit physiologischer Kochsalzlösung beziehungsweise mit Lösungsmittel behandelte Kontrollgruppe teil.

Vor der Blutdruckmessung wurden die Tiere für 30 Minuten in eine schall- und lichtisolierte Kammer von 38 °C gesetzt. Dann wurden die Tiere immobilisiert. An der Schwanzwurzel, immer an der gleichen Stelle, wurde eine mit einem Manometer verbundene Gummimanschette angelegt, deren Material und Durchmesser standardisiert waren. Gemessen wurde nach dem Riva Rocci Prinzip. Zur Feststellung des systolischen Blutdruckes wurde die Palpationsmethode herangezogen. Zur Wahrnehmung des Pulses diente ein auf dem Gummiring angeordneter piezoelektrischer Kristall. Der Blutdruck eines jeden Tieres wurde jeweils 2 Minuten nach der Immobilisierung und dann alle halbe Minute gemessen. Die nach 3-5 Minuten wiederholt wahrgenommenen Werte wurden berücksichtigt. Die Ergebnisse sind in der Tabelle 2 enthalten.

Tabelle 1

| Wirkung der Verbindungen in einer Dosis von 5 mg/kg auf die Wasser-, Na- und K-Entleerung von Ratten innerhalb von 24 Stunden, ausgedrückt in Prozent der Werte der unbehandelten Kontrolle, sowie das Na/K-Verhältnis | | | | |
|---|---|---|---|---|
| Beispiel Nr. | Wasser | Na | K | Na/K |
| Kontrolle | 100 | 100 | 100 | 3,0 |
| 1 | 148 | 152 | 122 | 4,6 |
| 7 | 129 | 133 | 110 | 3,7 |
| 9 | 99 | 105 | 95 | 3,4 |
| 16 | 111 | 117 | 109 | 3,3 |
| 19 | 117 | 121 | 120 | 3,1 |
| 22 | 107 | 99 | 103 | 3,7 |
| 23 | 98 | 105 | 102 | 4,0 |
| 24 | 108 | 104 | 104 | 3,9 |
| 26 | 107 | 108 | 106 | 4,0 |
| 27 | 95 | 94 | 85 | 4,3 |
| 29 | 97 | 101 | 105 | 2,9 |
| 30 | 114 | 106 | 108 | 3,0 |
| 32 | 117 | 121 | 115 | 3,2 |
| Hypothiazid | 124 | 129 | 114 | 3,5 |
| Furosemid | 107 | 106 | 105 | 2,7 |

Tabelle 2

Wirkung der Verbindung gemäß Beispiel 1 und von Furosemid auf den systolischen Blutdruck von SHR-Ratten nach einer einmaligen oralen Gabe

| Parameter | Lösungsmittel | Furosemid 100 mg/kg | Verbindung Beispiel 1 5 mg/kg |
|---|---|---|---|
| vor der Behandlung, Hgmm (0,00133 bar) | 195 | 201 | 216 |
| Blutdrucksenkung 6 Stunden nach der Behandlung | | | |
| Hgmm | | 51 | 31 |
| % | | 25,3 | 14,3 |
| 12 Stunden nach der Behandlung | | | |
| Hgmm | | 39 | 45 |
| % | | 19,4 | 20,1 |
| 24 Stunden nach der Behandlung | | | |
| Hgmm | | 3 | 30 |
| % | | 1,5 | 13,8 |

**Reaktionsschema A**

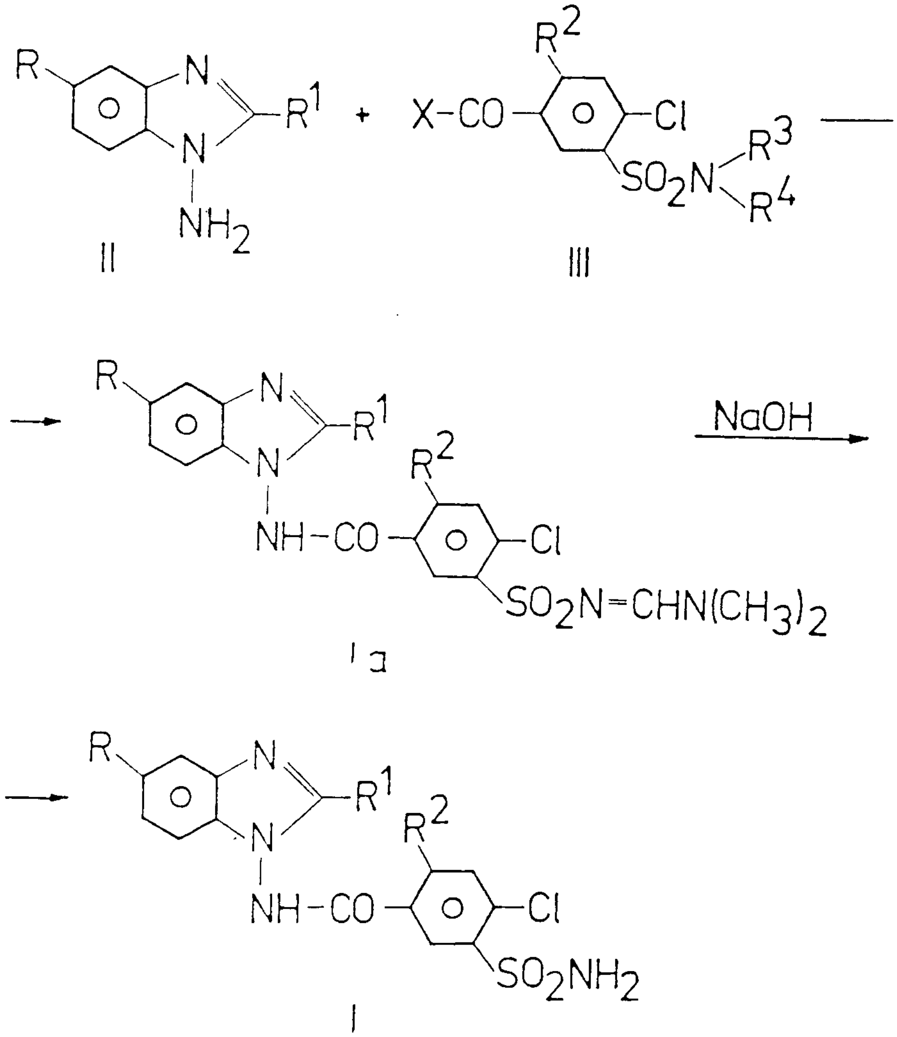


II

III

Ia

I

Reaktionsschema B

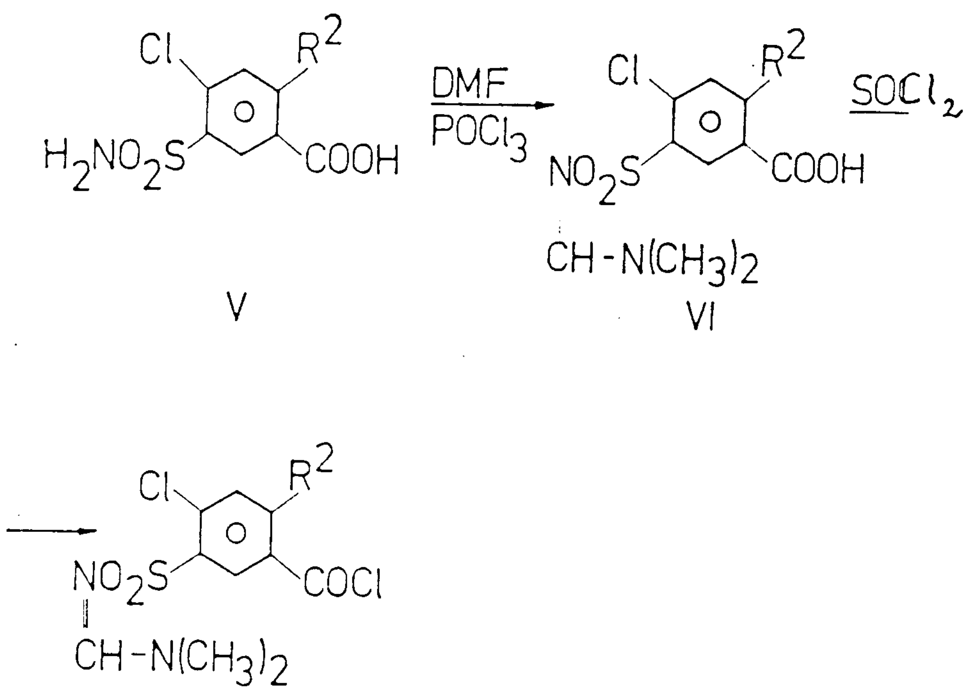


V

VI

IIIa

Reaktionsschema C

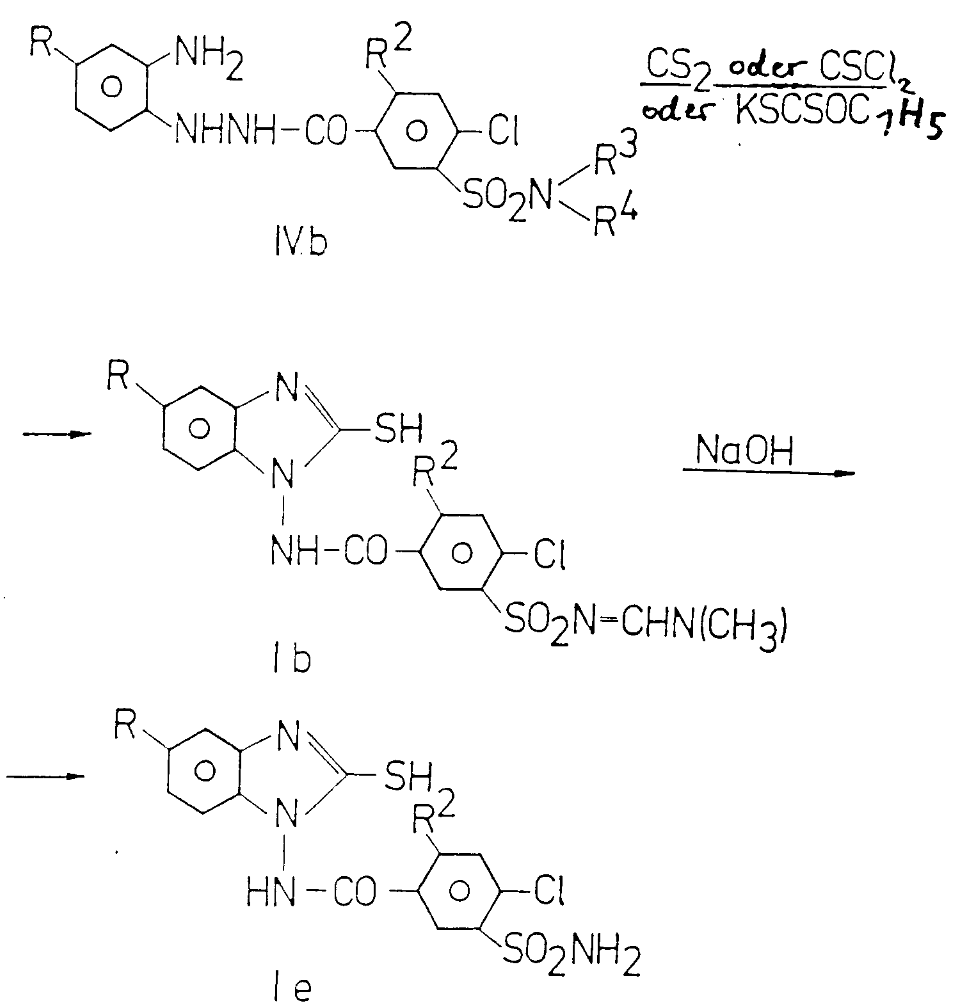

Reaktionsschema D

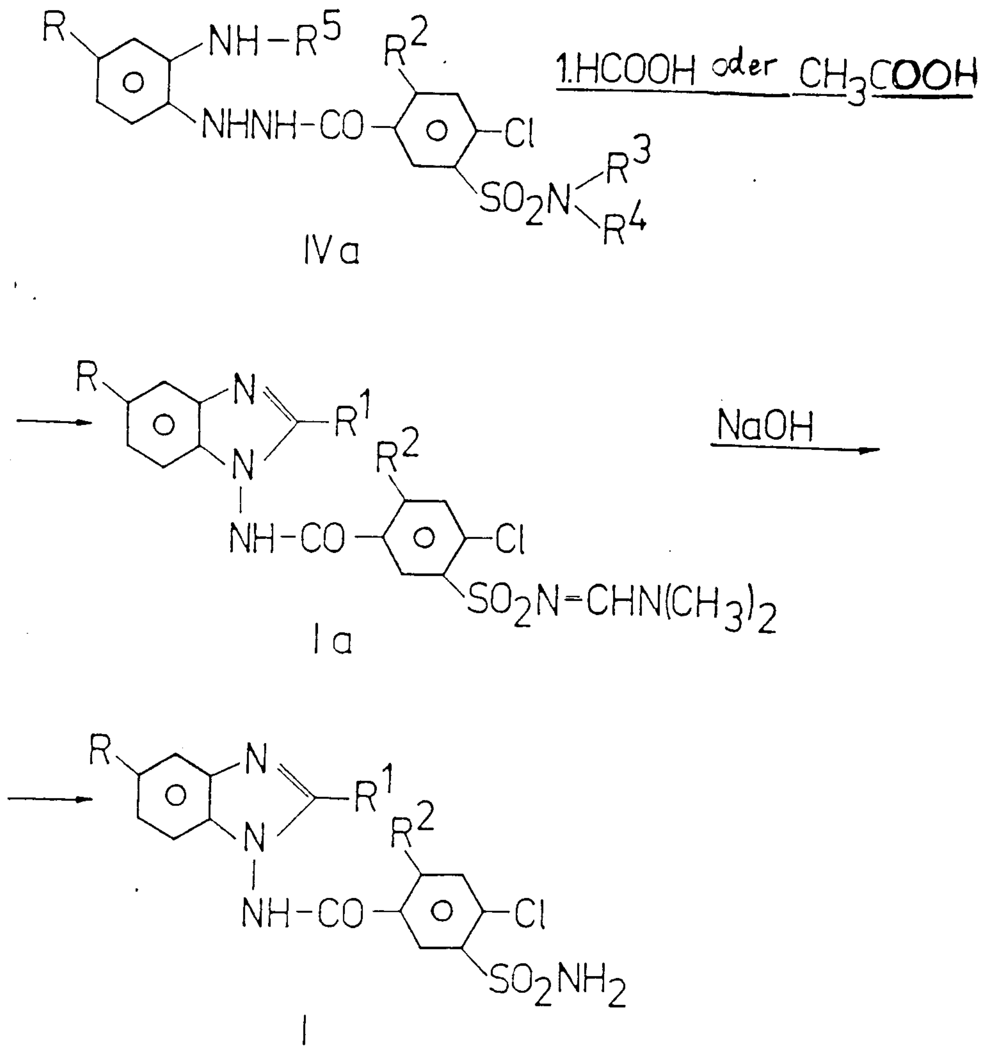


IVa

Ia

I

Reaktionsschema 5

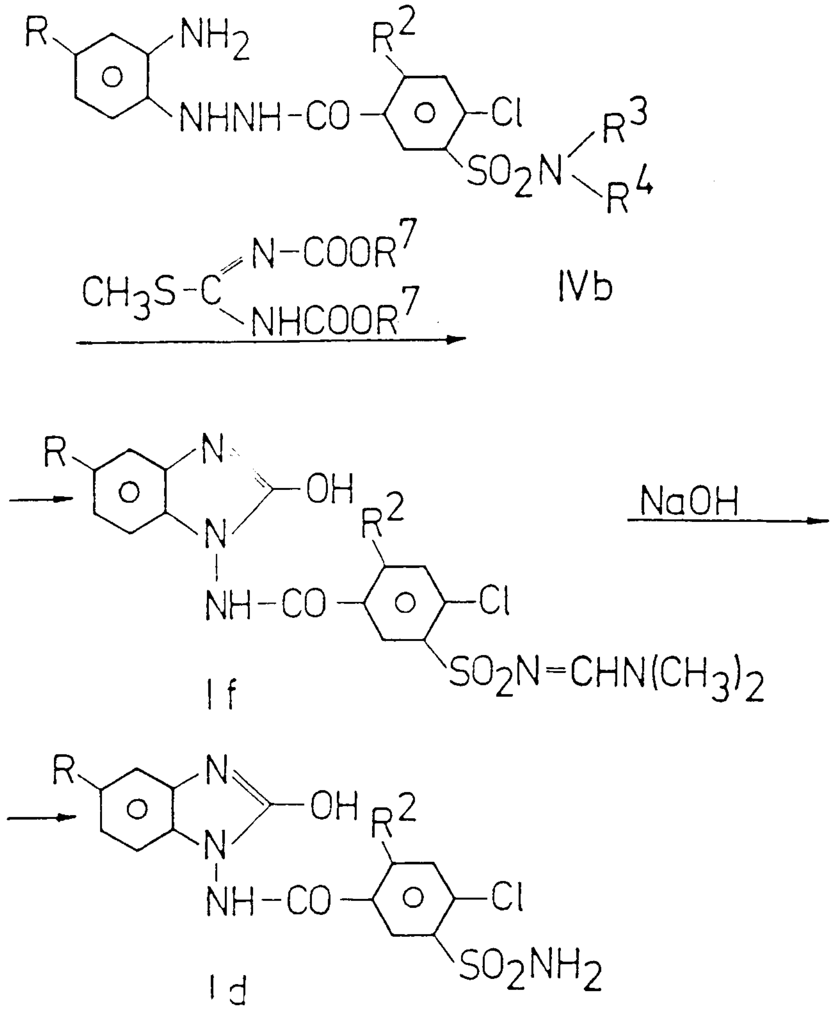

Reaktionsschema F

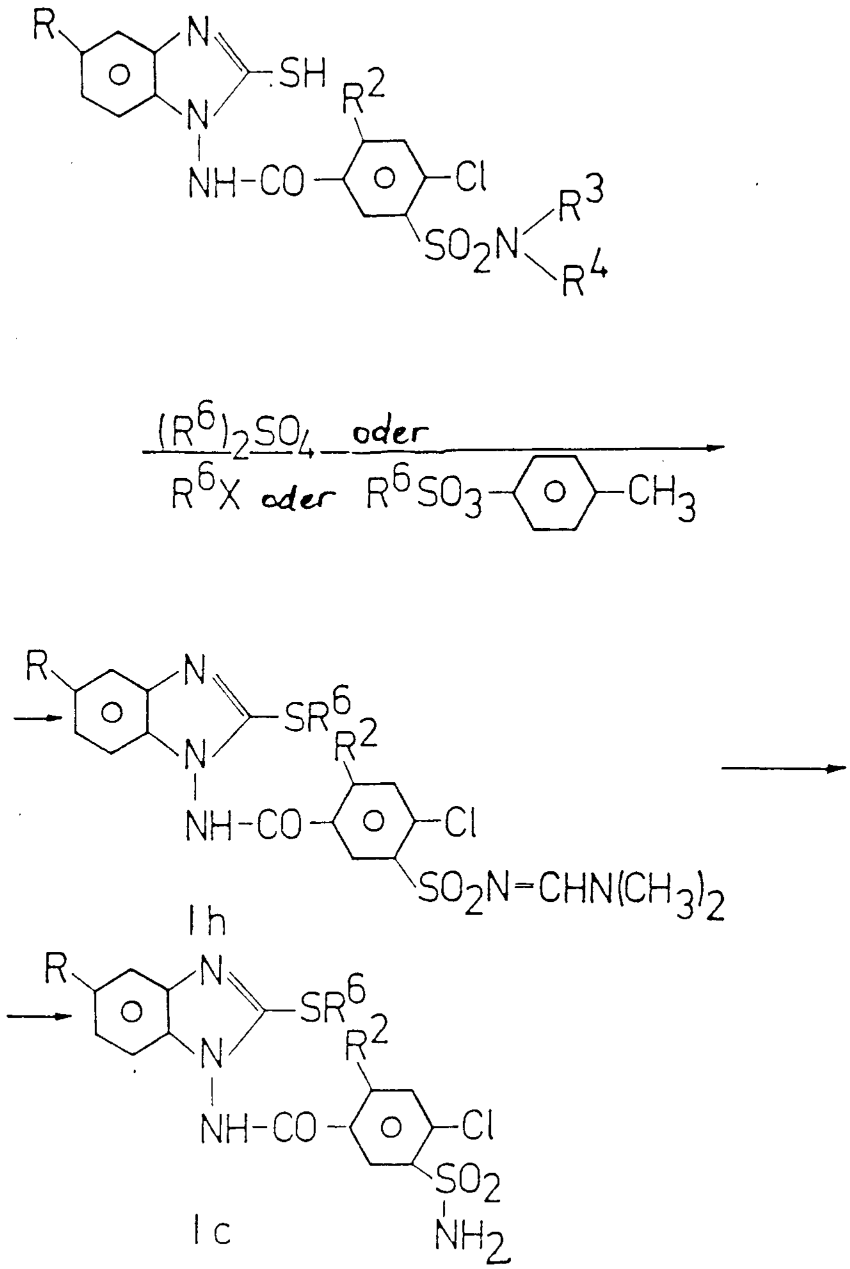


Ib

Ih

Ic

Reaktionsschema G

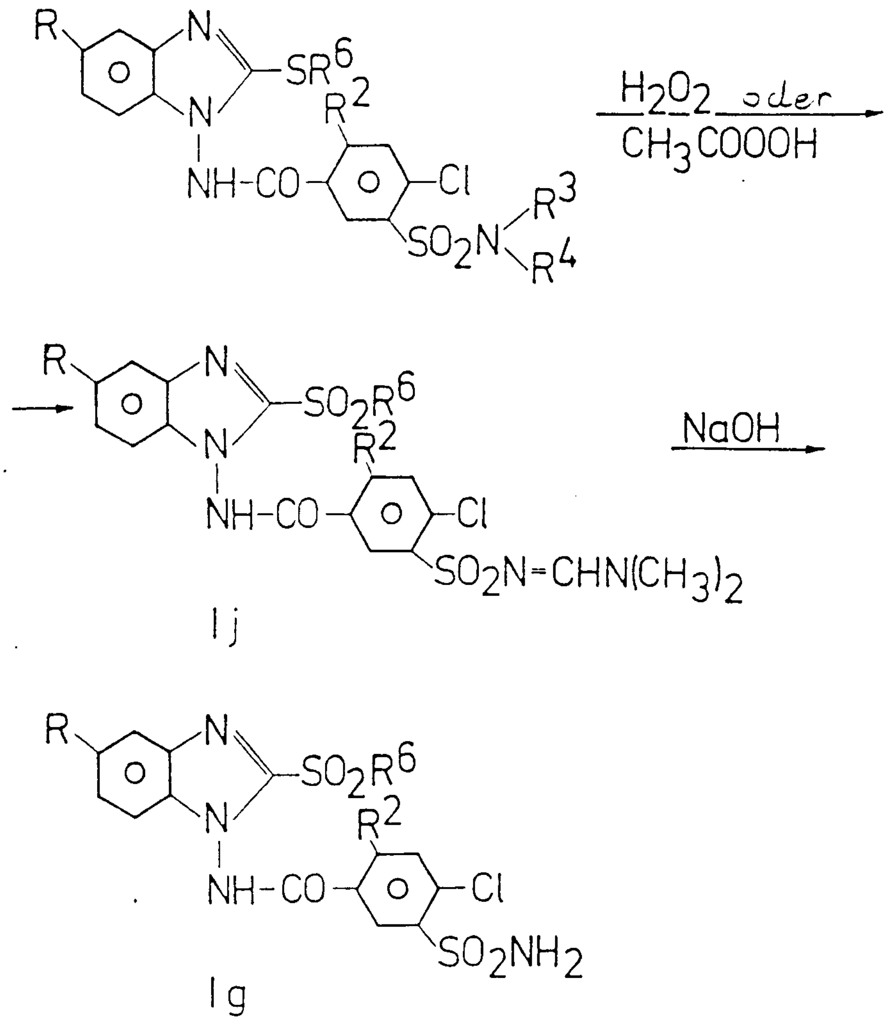


Ij

Ig

## Patentansprüche

1. Verfahren zur Herstellung von Benzimidazolderivaten der allgemeinen Formel I

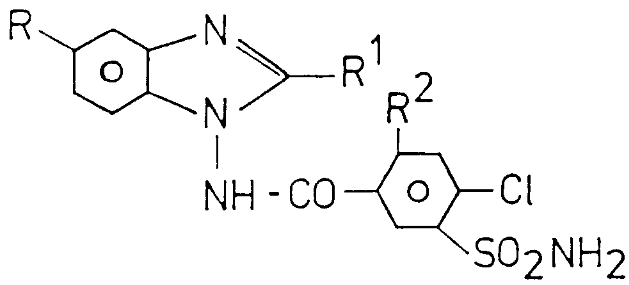

I

worin

R für Wasserstoff, Trifluormethyl-, Carboxylgruppe, Alkoxycarbonylgruppe mit 2-5 Kohlenstoffatomen, Cyano-, Benzoyl-, Sulfamoylgruppe oder Alkylsulfonylgruppe mit 1-4 Kohlenstoffatomen,

$R^1$ für Wasserstoff, gerade oder verzweigte Alkylgruppe mit 1-4 Kohlenstoffatomen, Alkylthio- oder Alkylsulfonylgruppe mit jeweils 1-4 Kohlenstoffatomen, Benzyl thio-, Benzylsulfonyl, Phenyl-, Hydroxyl- oder Thiolgruppe und

$R^2$ für Wasserstoff oder Chlor steht,

sowie der physiologisch verträglichen Salze dieser Verbindungen, dadurch gekennzeichnet, daß man

a) 1-Aminobenzimidazole der allgemeinen Formel II

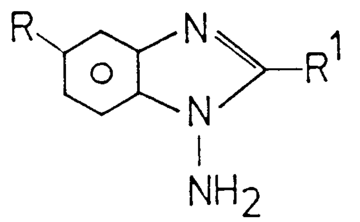

II

(worin die Bedeutung von R und $R^1$ die gleiche wie oben ist) mit Carbonsäurederivaten der allgemeinen Formel III

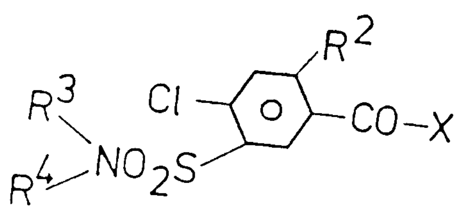

III

worin

X für Chlor, -OH, $OCH_2CN$, $-OCH_3$, $-OC_2H_5$, $-OCOOCH_3$ oder $-OCOOC_2H_5$ steht, die Bedeutung von

$R^2$ die gleiche wie oben ist und

$R^3$ und $R^4$ für Wasserstoffatom oder zusammen für die Gruppierung $=CHN(CH_3)_2$ stehen, umsetzt und die Schutzgruppe in alkalischem Medium abspaltet, oder

b) zur Herstellung der eine engere Gruppe der Verbindungen der allgemeinen Formel I bildenden Verbindungen der allgemeine Formel Ie

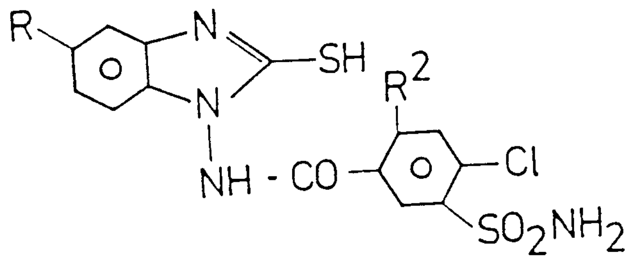

Ie

(worin die Bedeutung von R und $R^2$ die gleiche wie oben ist) 2-Aminophenylhydrazin-Derivate der allgemeinen Formel IVb

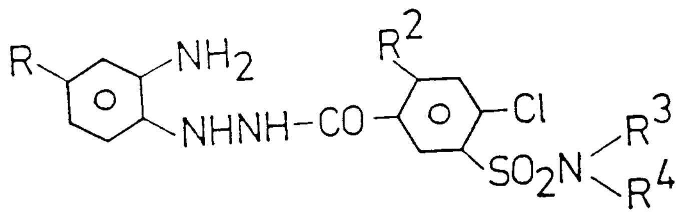

IVb

(worin die Bedeutung von R, $R^2$, $R^3$ und $R^4$ die gleiche wie oben ist) mit Schwefelkohlenstoff, Kaliumäthylxanthat oder Thiophosgen umsetzt und im Falle von Verbindungen der allgemeinen Formel Ib

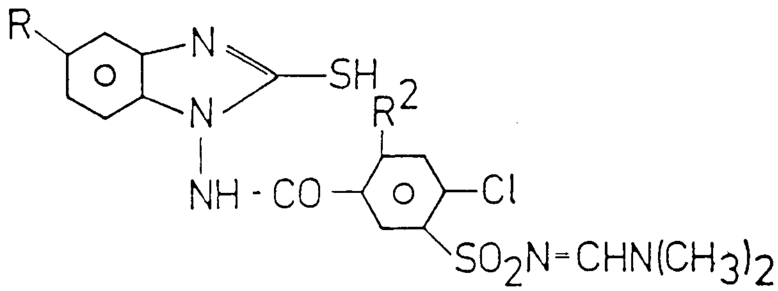

Ib

(worin die Bedeutung von R, $R^1$ und $R^2$ die gleiche wie oben ist) die Schutzgruppe in alkalischem Medium abspaltet, oder

c) zur Herstellung von Verbindungen der allgemeinen Formel I, in denen die Bedeutung von R und $R^2$ die gleiche wie oben ist und

$R^1$ für Wasserstoff, Methyl-, Äthyl-, Propyl-, Isopropyl-, Butyl-, Isobutyl-, tert.-Butyl- oder Phenylgruppe steht,

2-Aminophenylhydrazin-Derivate der allgemeinen Formel IVa

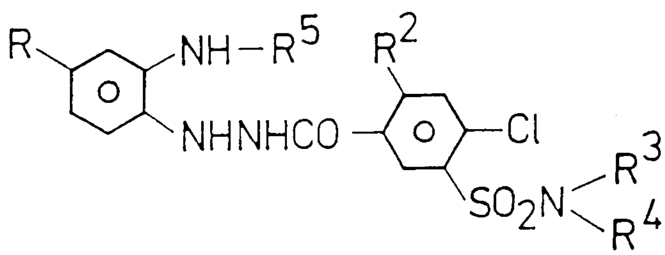

IVa

(worin die Bedeutung von R, $R^2$, $R^3$ und $R^4$ die gleiche wie oben ist und

$R^5$ für Wasserstoff, Acetyl-, Propionyl-, Butyryl-, Isobutyryl-, Pentanoyl-, 2-methylbutyryl-, Trimethylacetyl- oder Benzoylgruppe steht,

mit Ameisensäure oder Essigsäure umsetzt und im Falle von Verbindungen der allgemeinen Formel Ia

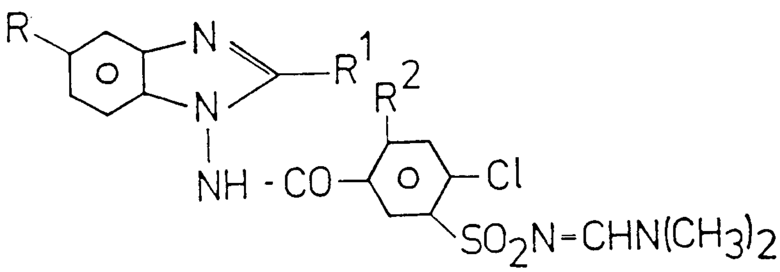

Ia

(worin die Bedeutung von R, $R^1$, $R^2$ die gleiche wie oben ist) die Schutzgruppe in alkalischem Medium abspaltet, oder

d) zur Herstellung der eine engere Gruppe der Verbindungen der allgemeinen Formel I bildenden Verbindungen der allgemeinen Formel Id

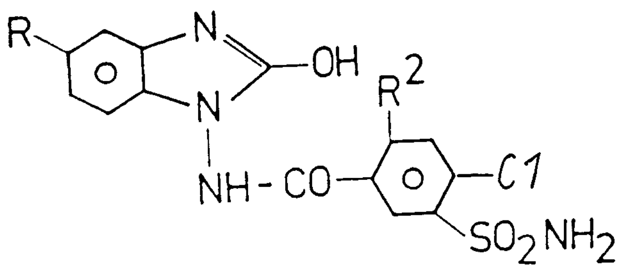

Id

(worin die Bedeutung von R und $R^2$ die gleiche wie oben ist) 2-Aminophenylhydrazin-Derivate der allgemeinen Formel IVb (worin die Bedeutung von R, $R^2$, $R^3$ und $R^4$ die gleiche wie oben ist) mit N,N$^1$-Dialkoxycarbonyl-S-methylisothioharnstoffen der allgemeinen Formel VII

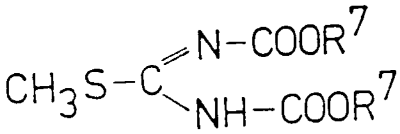

VII

(worin $R^7$ für Alkylgruppe mit 1-4 Kohlenstoffatomen steht) umsetzt und im Fall von Verbindungen der allgemeinen Formel If

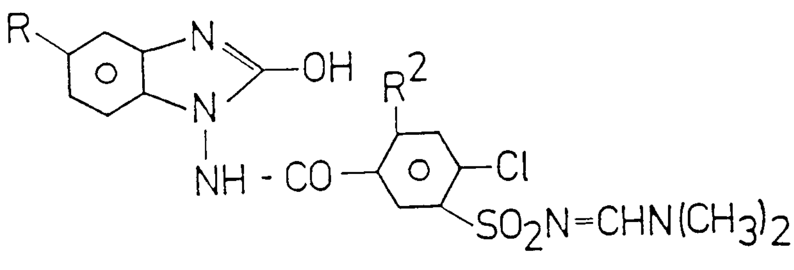

If

(worin die Bedeutung von R und $R^2$ die gleiche wie oben ist) die Schutzgruppe in alkalischem Medium abspaltet, oder

e) zur Herstellung der eine engere Gruppe der Verbindungen der allgemeinen Formel I bildenden Verbindungen der allgemeinen Formel Ic

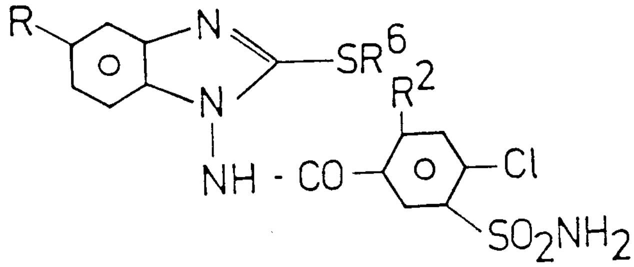

Ic

(worin die Bedeutung von R und $R^2$ die gleiche wie oben ist und $R^6$ für eine gerade oder verzweigte Alkylgruppe mit 1-4 Kohlenstoffatomen oder für Benzylgruppe steht) Verbindungen der allgemeinen Formeln Ib oder Ie (worin die Bedeutung von R, $R^2$, $R^3$ und $R^4$ die gleiche wie oben ist) mit einem Alkylierungsmittel umsetzt und im Falle von Verbindungen der allgemeinen Formel Ih

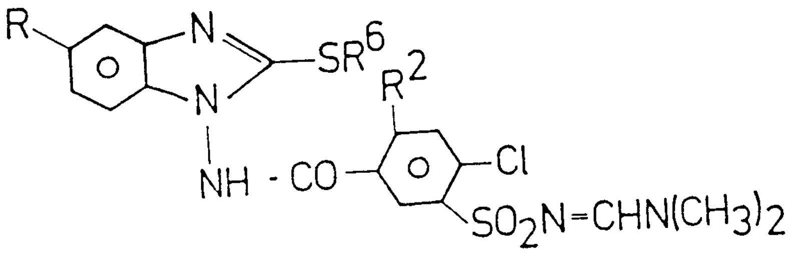

Ih

(worin die Bedeutung von R, $R^2$ und $R^6$ die gleiche wie oben ist) die Schutzgruppe in alkalischem Medium abspaltet, oder

f) zur Herstellung der eine engere Gruppe der Verbindungen der allgemeinen Formel I bildenden Verbindungen der allgemeinen Formel Ig

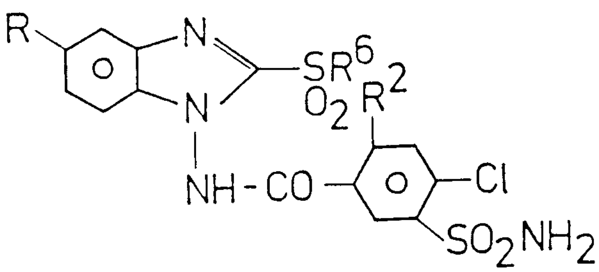

Ig

(worin die Bedeutung von R, $R^2$, $R^3$, $R^4$ und $R^6$ die gleiche wie oben ist) 2-Benzimidazolylthioäther der allgemeinen Formeln Ic oder Ih (worin die Bedeutung von R, $R^2$, $R^3$, $R^4$ und $R^6$ die gleiche wie oben ist) mit Oxydationsmitteln umsetzt und die Verbindung der allgemeinen Formel Ij

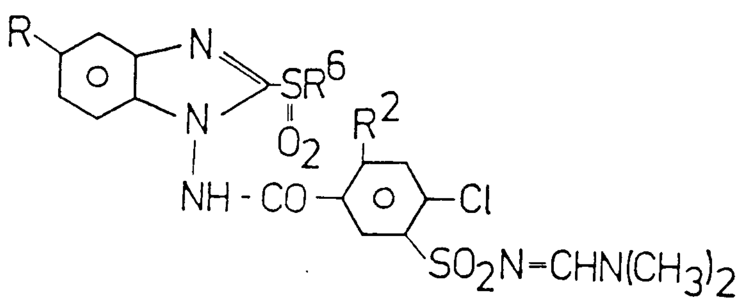

Ij

(worin die Bedeutung von R, $R^2$ und $R^6$ die gleiche wie oben ist) in alkalischem Medium von der Schutzgruppe befreit und die gemäß den Verfahren a) - f) erhaltenen Verbindungen gewünschtenfalls zu ihren physiologisch verträglichen Salzen umsetzt.

**2.** Benzimidazolderivate der allgemeinen Formel I

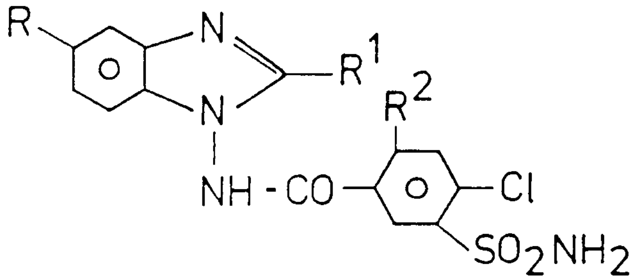

I

worin

R für Wasserstoff, Trifluormethyl-, Carboxylgruppe, Alkoxycarbonylgruppe mit 2-5 Kohlenstoffatomen, Cyano-, Benzoyl-, Sulfamoylgruppe oder Alkylsulfonylgruppe mit 1-4 Kohlenstoffatomen,

$R^1$ für Wasserstoff, gerade oder verzweigte Alkylgruppe mit 1-4 Kohlenstoffatomen, Alkylthio- oder Alkylsulfonylgruppe mit jeweils 1-4 Kohlenstoffatomen, Benzylthio-, Benzylsulfonyl-, Phenyl-, Hydroxyl- oder Thiolgruppe und

$R^2$ für Wasserstoff oder Chlor steht,

sowie die physiologisch verträglichen Salze dieser Verbindungen.

**3.** 1-[(4′-Chlor-3′-sulfamoyl-benzoyl)-amino]-5-carboxy-benzimidazol-2-thion und sein Monohydrat.

**4.** Arzneimittelpräparate, gekennzeichnet durch einen Gehalt an Verbindungen der allgemeinen Formel I gemäß Anspruch 1.

**5.** Verwendung von Verbindungen der allgemeinen Formel I gemäß Anspruch 1 zur Herstellung von diuretisch und saluretisch wirksamen Arzneimittelpräparaten.

## Claims

**1.** Process for preparing benzimidazole derivatives of general formula I

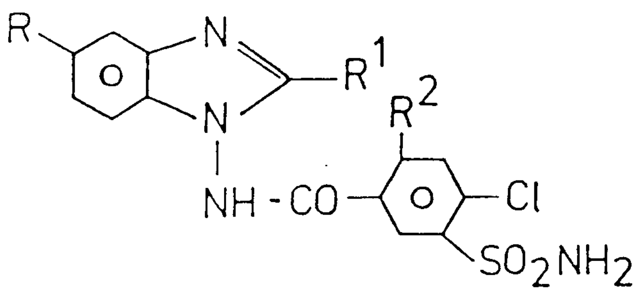

I

wherein

R represents hydrogen, trifluoromethyl, carboxyl, $C_{2-5}$-alkoxycarbonyl, cyano, benzoyl, sulphamoyl or $C_{1-4}$-alkylsulphonyl,

$R^1$ represents hydrogen, straight-chained or branched $C_{1-4}$-alkyl, $C_{1-4}$-alkylthio or $C_{1-4}$-alkylsulphonyl, benzylthio, benzylsulphonyl, phenyl, hydroxyl or thiol and

$R^2$ represents hydrogen or chlorine,

and the physiologically acceptable salts of these compounds, characterised in that

a) 1-aminobenzimidazoles of general formula II

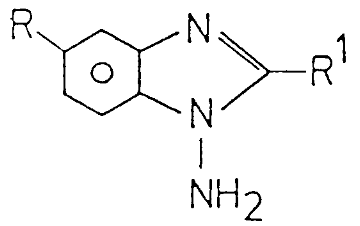

II

(wherein R and $R^1$ are as hereinbefore defined) are reacted with carboxylic acid derivatives of general formula III

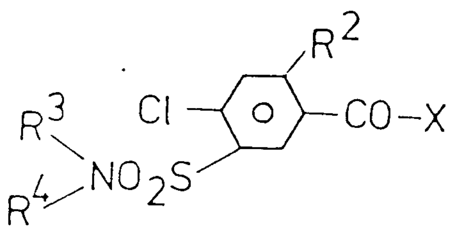

III

wherein

| | |
|---|---|
| X | represents chlorine, -OH, $-OCH_2CN$, $-OCH_3$, $-OC_2H_5$, $-OCOOCH_3$ or $-OCOOC_2H_5$, |
| $R^2$ | is as hereinbefore defined and |
| $R^3$ and $R^4$ | represent hydrogen atoms or together represent the group $=CHN(CH_3)_2$, and the protecting group is cleaved in an alkaline medium, or |

b) in order to prepare the compounds of general formula Ie which form a narrower group of compounds of formula I

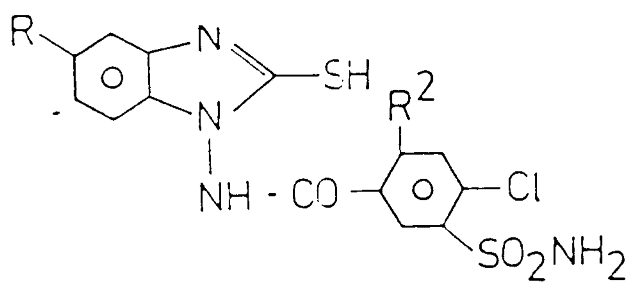

Ie

(wherein R and $R^2$ are as hereinbefore defined) 2-aminophenylhydrazine derivatives of general formula IVb

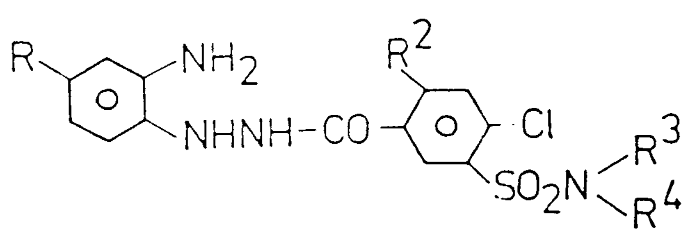

IVb

(wherein R, $R^2$, $R^3$ and $R^4$ are as hereinbefore defined) are reacted with carbon disulphide,

potassium ethylxanthate or thiophosgene and, in the case of compounds of general formula Ib

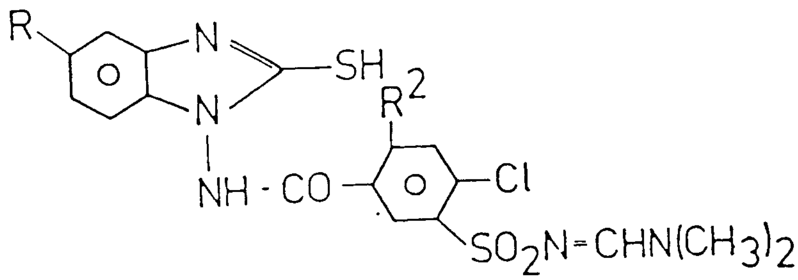

Ib

(wherein R, $R^1$ and $R^2$ are as hereinbefore defined) the protecting group is cleaved in an alkaline medium, or

c) in order to prepare compounds of general formula I wherein R and $R^2$ are as hereinbefore defined and

$R^1$ represents hydrogen, methyl, ethyl, propyl, isopropyl, butyl, isobutyl, tert.-butyl or phenyl,

2-aminophenylhydrazine derivatives of general formula IVa

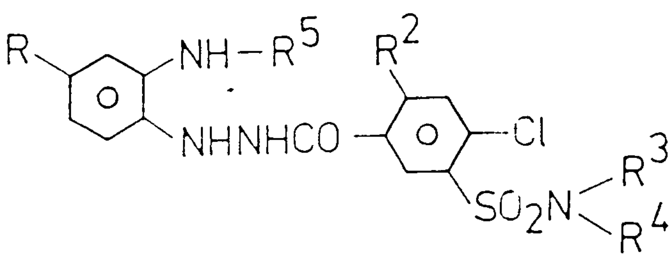

IVa

(wherein R, $R^2$, $R^2$ and $R^4$ are as hereinbefore defined and

$R^5$ represents hydrogen, acetyl, propionyl, butyryl, isobutyryl, pentanoyl, 2-methylbutyryl, trimethylacetyl or benzoyl,

are reacted with formic acid or acetic acid and, in the case of compounds of general formula Ia

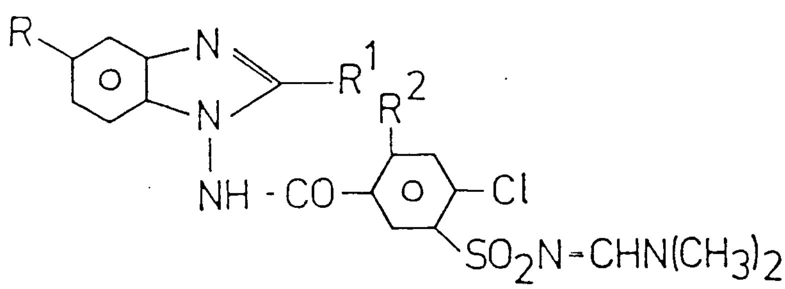

Ia

(wherein R, $R^1$ and $R^2$ are as hereinbefore defined) the protecting group is cleaved in an alkaline medium, or

d) in order to prepare the compounds of general formula Id which form a narrower group of the compounds of general formula I

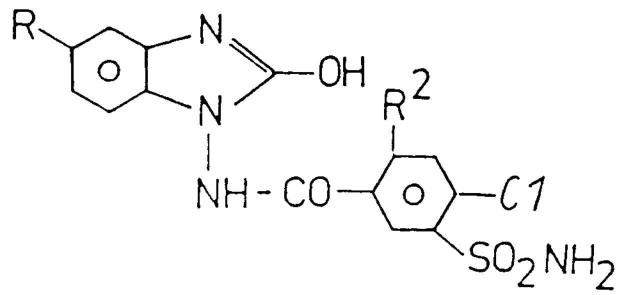

Id

(wherein R and $R^2$ are as hereinbefore defined) 2-aminophenylhydrazine derivatives of general formula IVb (wherein R, $R^2$, $R^3$ and $R^4$ are as hereinbefore defined) are reacted with N,N$^1$-dialkoxycarbonyl-S-methylisothioureas of general formula VII

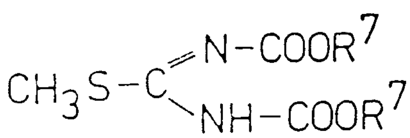

VII

(wherein $R^7$ represents $C_{1-4}$-alkyl) and in the case of compounds of general formula If

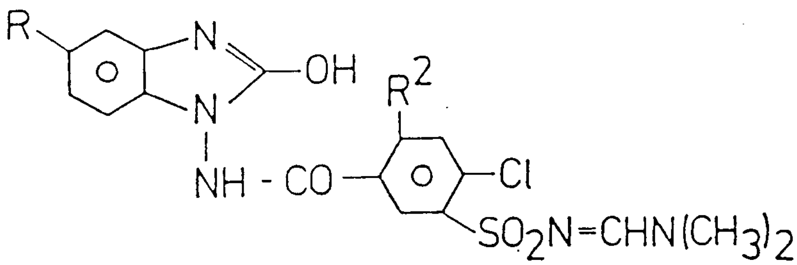

If

(wherein R and $R^2$ are as hereinbefore defined) the protecting group is cleaved in an alkaline medium, or

e) in order to prepare the compounds of general formula Ic which form a narrower group of compounds of general formula I

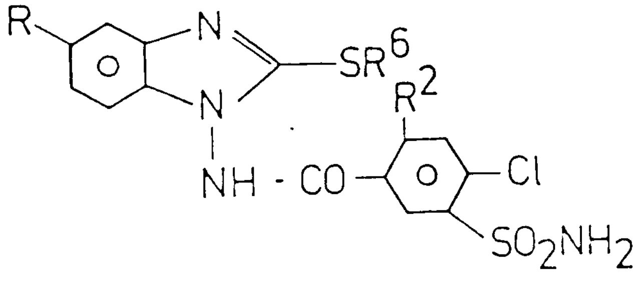

Ic

(wherein R and $R^2$ are as hereinbefore defined and $R^6$ represents a straight-chained or branched $C_{1-4}$-alkyl or a benzyl group) compounds of general formulae Ib or Ie (wherein R, $R^2$, $R^3$ and $R^4$ are

as hereinbefore defined) are reacted with an alkylating agent and, in the case of compounds of general formula Ih

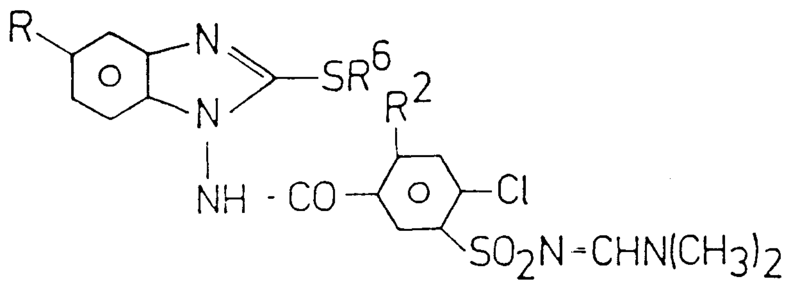

Ih

(wherein R, $R^2$ and $R^6$ are defined as hereinbefore) the protecting group is cleaved in an alkaline medium, or

f) in order to prepare the compounds of general formula Ig which form a narrower group of the compounds of general formula I

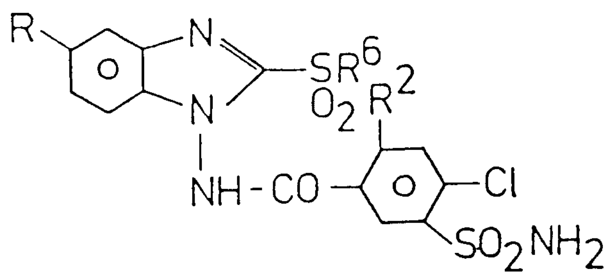

Ig

(wherein R, $R^2$, $R^3$, $R^4$ and $R^6$ are as hereinbefore defined) 2-benzimidazolylthioethers of general formulae Ic or Ih (wherein R, $R^2$, $R^3$, $R^4$ and $R^6$ are as hereinbefore defined) are reacted with oxidising agents and the compound of general formula Ij

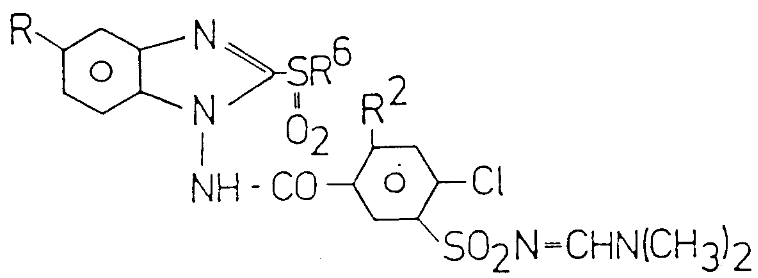

Ij

(wherein R, $R^2$ and $R^6$ are as hereinbefore defined) is freed from the protecting group in an alkaline medium and the compounds obtained according to processes a) to f) are reacted, if desired, to form the physiologically acceptable salts thereof.

**2.** Benzimidazole derivatives of general formula I

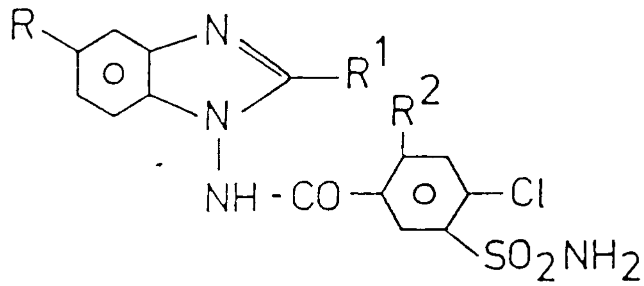

I

wherein

R represents hydrogen, trifluoromethyl, carboxyl, $C_{2-5}$-alkoxycarbonyl, cyano, benzoyl, sulphamoyl or $C_{1-4}$-alkylsulphonyl,

$R^1$ represents hydrogen, straight-chained or branched $C_{1-4}$-alkyl, $C_{1-4}$-alkylthio or $C_{1-4}$-alkylsulphonyl, benzylthio, benzylsulphonyl, phenyl, hydroxyl or thiol and

$R^2$ represents hydrogen or chlorine,

and the physiologically acceptable salts of these compounds.

**3.** 1-[(4'-Chloro-3'-sulphamoyl-benzoyl)-amino]-5-carboxybenzimidazole-2-thione and the monohydrate thereof.

**4.** Pharmaceutical preparations, characterised in that they contain compounds of general formula I according to claim 1.

**5.** Use of compounds of general formula I according to claim 1 for preparing pharmaceutical compositions having a diuretic and saluretic activity.

**Revendications**

**1.** Procédé pour la préparation des dérivés de benzimidazol de formule générale

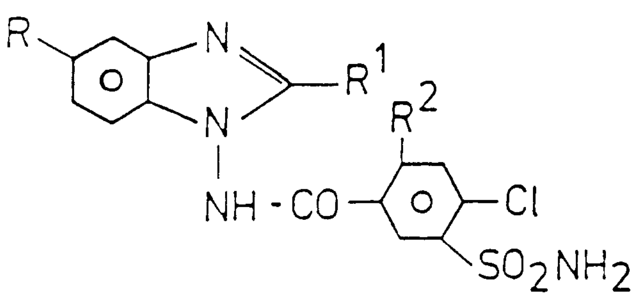

I

où

R représente l'hydrogène, les groupes trifluorométhyle, carboxyle, alcoxycarbonyle ayant 2-5 atomes de carbone, les groupes cyano, benzoyle, sulfamoyle ou alkylsulfonyle ayant 1-4 atomes de carbone,

$R^1$ représente l'hydrogène, un groupe alkyle linéaire ou ramifié ayant 1-4 atomes de carbone, le groupe alkylthio ou alkylsulfonyle ayant 1-4 atomes de carbone, les groupes benzylthio, benzylsulfonyle, phényle, hydroxyle ou thiol et

$R^2$ représente l'hydrogène ou le chlore,

ainsi que des sels physiologiquement compatibles de ces composés, caractérisé

a) en ce que l'on fait réagir les l-aminobenzimidazols de formule générale II

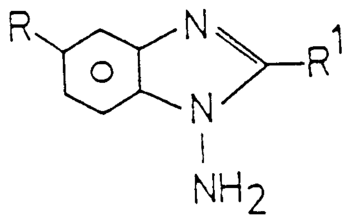

II

(R et $R^1$ ayant les mêmes significations que celles données ci-dessus) avec des dérivés d'acides carboxyliques de formule générale III

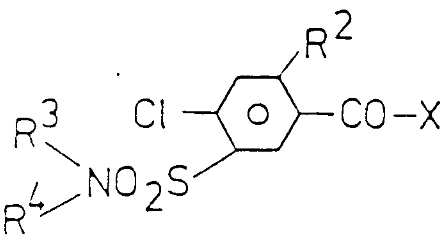

III

où

- X représente le chlore, -OH, $-OCH_2CN$, $-OCH_3$, $-OC_2H_5$, $-OCOOCH_3$ ou $-OCOOC_2H_5$,
- $R^2$ a la même signification que celle donnée ci-dessus,
- $R^3$ et $R^4$ représentent un atome d'hydrogène ou représentent ensemble le groupement $=CHN(CH_3)_2$, et en ce que l'on clive le groupe protecteur en milieu alcalin, ou

b) en ce que, pour la préparation des composés de formule générale Ie et formant un groupe plus restreint des composés de formule générale I

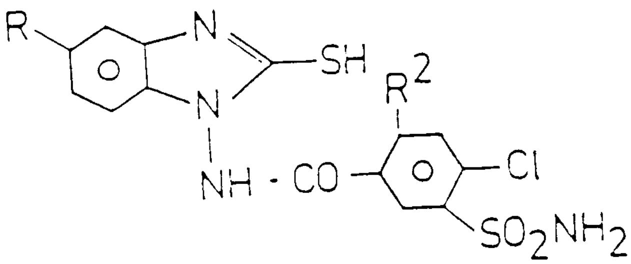

Ie

(R et $R^2$ ayant les mêmes significations que celles données ci-dessus), on fait réagir les dérivés de 2-aminophénylhydrazine de formule générale IVb

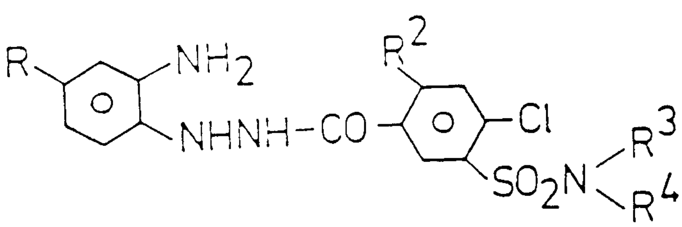

IVb

(où R, $R^2$, $R^3$ et $R^4$ ont les mêmes significations que celles données ci-dessus) avec le sulfure de carbone, le xanthate d'éthylpotassium ou le thiophosgène et en ce que, dans le cas des composés de formule générale Ib,

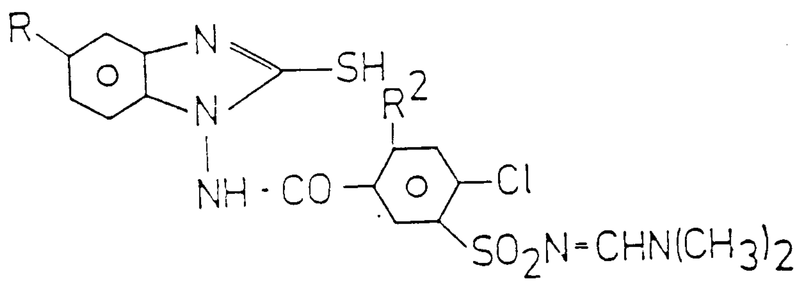

Ib

(où R, $R^1$ et $R^2$ ont les mêmes significations que celles données ci-dessus, on clive le groupe protecteur en milieu alcalin, ou

c) en ce que, pour la préparation des composés de formule générale I dans laquelle R et $R^2$ ont les mêmes significations que celles données ci-dessus, et

$R^1$ représente l'hydrogène, les groupes méthyle, éthyle, propyle, isopropyle, butyle, isobutyle, tert.-butyle ou phényle,

l'on fait réagir les dérivés de 2-aminophénylhydrazine de formule générale IVa

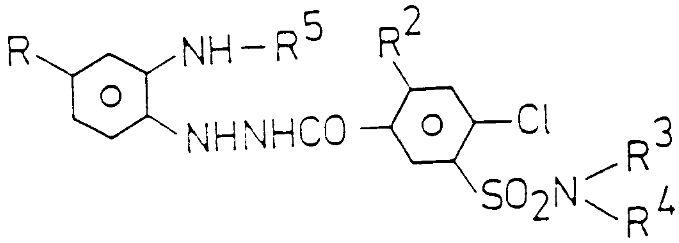

IVa

(où R, $R^2$, $R^3$ et $R^4$ ont les mêmes significations que celles données ci-dessus) et

$R^5$ représente l'hydrogène, les groupes acétyle, propionyle, butyryle, isobutyryle, pentanoyle, 2-méthylbutyryle, triméthylacétyle ou benzoyle,

avec l'acide formique ou acétique et, en ce que, dans le cas des composés de formule générale Ia

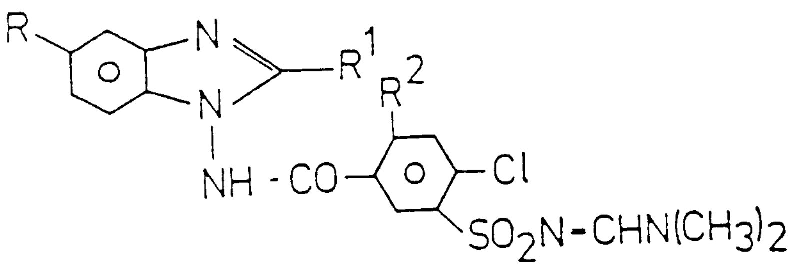

Ia

(où R, $R^1$, $R^2$ ont les mêmes significations que celles données ci-dessus), l'on clive le groupe protecteur en milieu alcalin, ou

d) en ce que, pour la préparation des composés de formule générale Id et formant un groupe plus restreint des composés de formule générale I

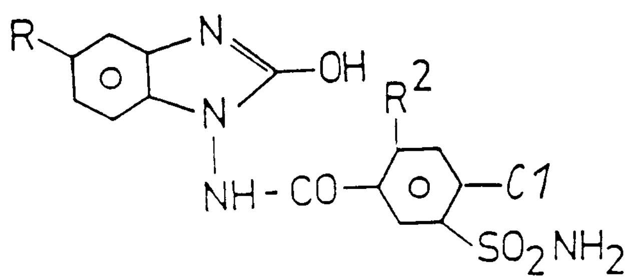

Id

(où R et $R^2$ ont les mêmes significations que celles données ci-dessus), l'on fait réagir les dérivés de 2-aminophénylhydrazinede formule générale IVb (où R, $R^2$, $R^3$ et $R^4$ ont les mêmes significations que celles données ci-dessus) avec les N,N[1]-dialcoxycarbonyl-S-méthylisothiourées de formule générale VI

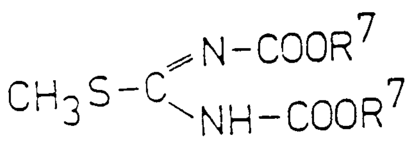

VII

(où $R^7$ représente un groupe alkyle ayant 1-4 atomes de carbone) et, dans le cas des composés de formule génerale If

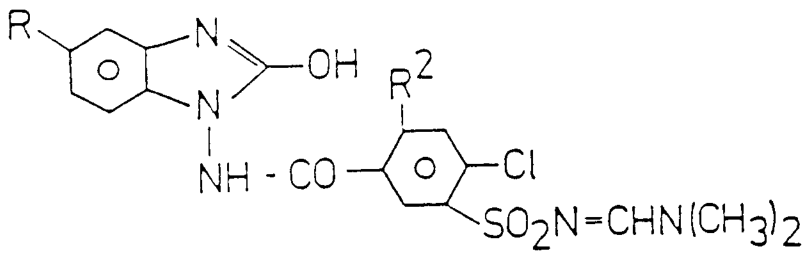

If

(où R et $R^2$ ont les mêmes significations que celles données ci-dessus), l'on clive le groupe protecteur en milieu alcalin, ou

e) en ce que, pour la préparation des composés de formule générale Ic et formant un groupe plus restreint des composés de formule générale I

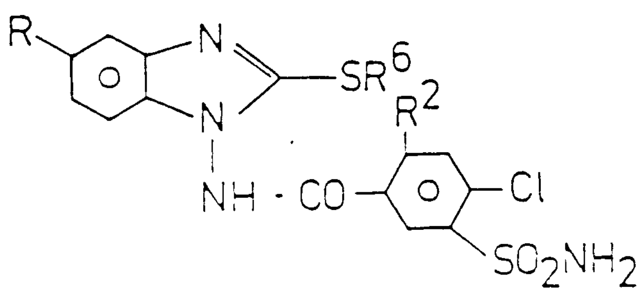

Ic

(où R et $R^2$ ont les mêmes significations que celles données ci-dessus et $R^6$ représente un groupe alkyle linéaire ou ramifie contenant 1-4 atomes de carbone ou le groupe benzyle), l'on fait réagir les

composés de formule générale Ib ou Ie (où R, $R^2$, $R^3$ et $R^4$ ont les mêmes significations que celles données ci-dessus) avec un agent d'alcoylation et en ce que, dans le cas des composés de formule générale Ih

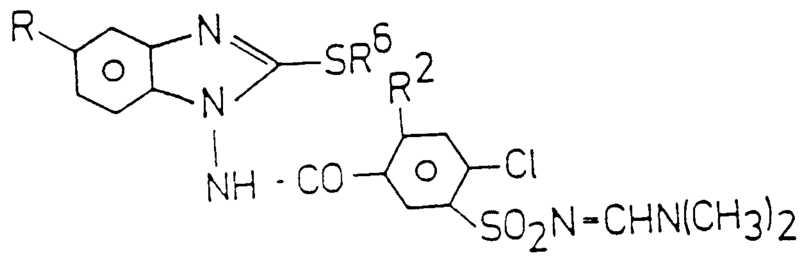

Ih

(où R, $R^2$ et $R^6$ ont les mêmes significations que celles données ci-dessus), l'on clive le groupe protecteur en milieu alcalin, ou

f) en ce que, pour la préparation des composés de formule générale Ig et formant un groupe plus restreint des composés de formule générale I

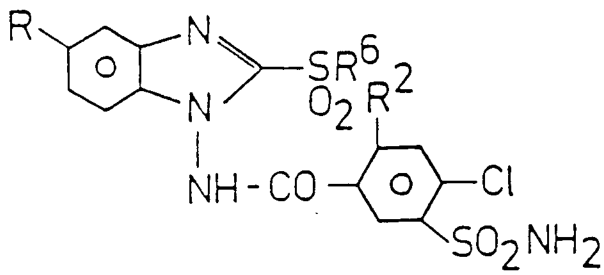

Ig

(où R, $R^2$, $R^3$, $R^4$ et $R^6$ ont les mêmes significations que celles données ci-dessus), l'on fait réagir les 2-benzimidazolylthioéthers de formule générale Ic ou Ih (où R, $R^2$, $R^3$, $R^4$ et $R^6$ ont les mêmes significations que celles données ci-dessus) avec des oxydants et en ce que l'on libère le composé de formule générale Ij

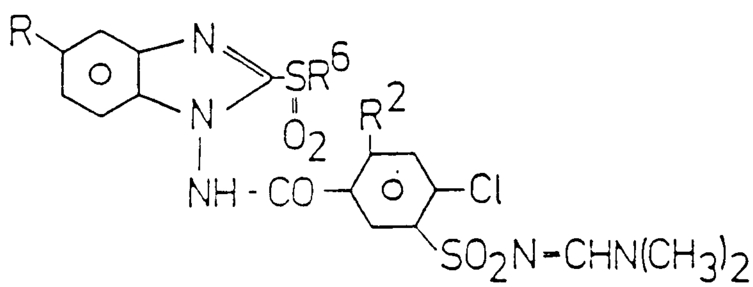

Ij

(où R, $R^2$ et $R^6$ ont les mêmes significations que celles données ci-dessus) du groupe protecteur en milieu alcalin et, si désiré, en ce que l'on transforme les composés obtenus selon les procédés a)-f) en leurs sels physiologiquement compatibles.

**2.** Dérivés de benzimidazol de formule générale I

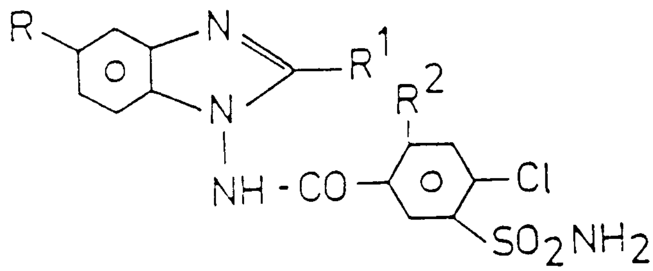

I

où

R représente l'hydrogène, les groupes trifluorométhyle, carboxyle, alcoxycarbonyle ayant 2-5 atomes de carbone, les groupes cyano, benzoyle, sulfamoyle ou alkylsulfonyle ayant 1-4 atomes de carbone,

$R^1$ représente l'hydrogène, un groupe alkyle linéaire ou ramifié ayant 1-4 atomes de carbone, le groupe alkylthio ou alkylsulfonyle ayant 1-4 atomes de carbone, les groupes benzylthio, benzylsulfonyle, phényle, hydroxyle ou thiol et

$R^2$ représente l'hydrogène ou le chlore,

ainsi que les sels physiologiquement compatibles de ces composés.

**3.** La 1-[(4'-chloro-3'-sulfamoyl-benzoyl)-amino]-5-carboxy-benzimidazol-2-thione et son monohydrate.

**4.** Médicament caractérisé en ce qu'il contient des composés de formule générale I selon la revendication 1.

**5.** Utilisation des composés de formule générale I selon la revendication 1 pour la préparation de médicaments ayant une action diurétique et salurétique.

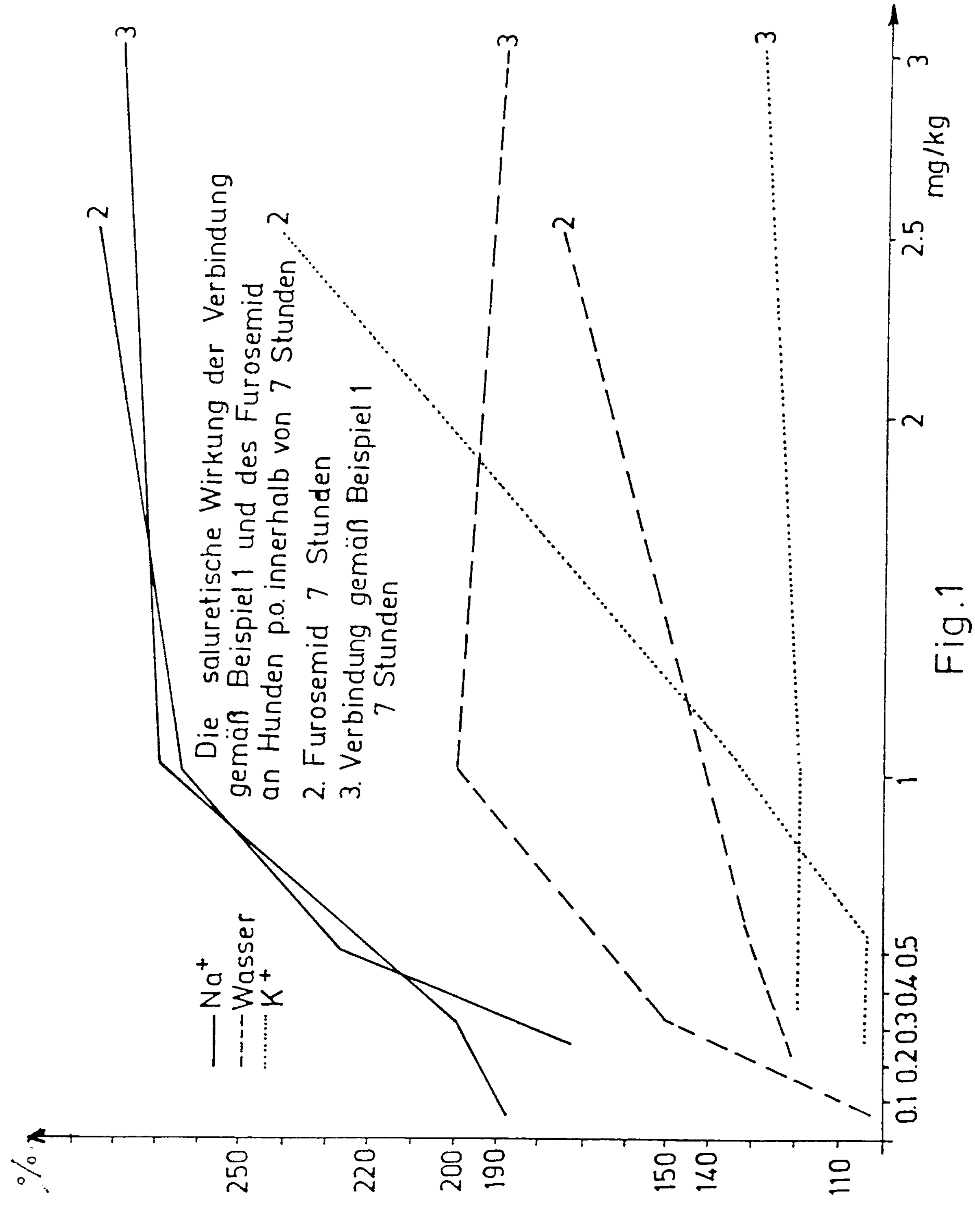
Die saluretische Wirkung der Verbindung
gemäß Beispiel 1 und des Furosemid
an Hunden p.o. innerhalb von 7 Stunden
2. Furosemid 7 Stunden
3. Verbindung gemäß Beispiel 1
7 Stunden
—Na+
---Wasser
.....K+
%
250
220
200
190
150
140
110
0.1 0.2 0.3 0.4 0.5
1
2
2.5
3
mg/kg
2
3

Fig.1

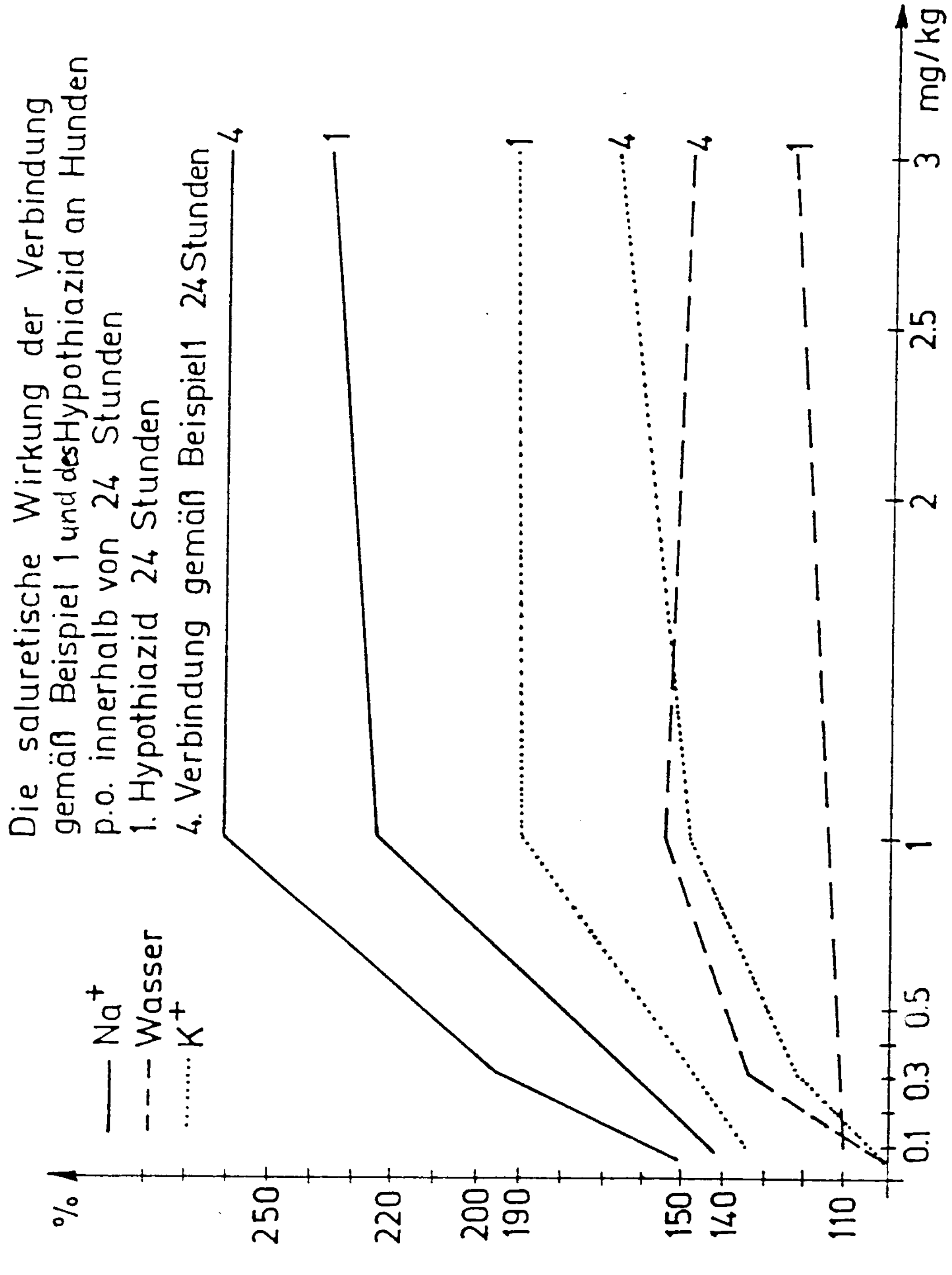

Die saluretische Wirkung der Verbindung gemäß Beispiel 1 und des Hypothiazid an Hunden p.o. innerhalb von 24 Stunden
1. Hypothiazid 24 Stunden
4. Verbindung gemäß Beispiel1 24 Stunden
Na+
Wasser
K+
%
250
220
200
190
150
140
110
0.1
0.3
0.5
1
2
2.5
3
mg/kg
4
1


Fig.2